(19) Europäisches Patentamt
European Patent Office
Office européen des brevets

(11) Veröffentlichungsnummer: **0 381 033 B1**

(12) **EUROPÄISCHE PATENTSCHRIFT**

(45) Veröffentlichungstag der Patentschrift: **23.03.94**

(51) Int. Cl.[5]: **C07C 311/19**, C07C 279/18, C07C 257/18, C07D 213/53, A61K 31/16

(21) Anmeldenummer: **90101404.3**

(22) Anmeldetag: **24.01.90**

# (54) Carbonsäure- und Sulfonsäureamide.

(30) Priorität: **31.01.89 CH 326/89**
**13.11.89 CH 4069/89**

(43) Veröffentlichungstag der Anmeldung:
**08.08.90 Patentblatt 90/32**

(45) Bekanntmachung des Hinweises auf die Patenterteilung:
**23.03.94 Patentblatt 94/12**

(84) Benannte Vertragsstaaten:
**AT BE CH DE DK ES FR GB GR IT LI LU NL SE**

(56) Entgegenhaltungen:
**EP-A- 0 214 429** **EP-A- 0 239 907**
**EP-A- 0 255 728** **EP-A- 0 325 245**
**AT-B- 22 882** **FR-M- 3 656**
**GB-A- 2 007 663**

(73) Patentinhaber: **F. HOFFMANN-LA ROCHE AG**
**Postfach 3255**
**CH-4002 Basel(CH)**

(72) Erfinder: **Alig, Leo, Dr.**
**Liebrütistrasse 32**
**CH-4303 Kaiseraugst(CH)**
Erfinder: **Edenhofer, Albrecht, Dr.**
**Rudolf Wackernagelstrasse 35**
**CH-4125 Riehen(CH)**
Erfinder: **Müller, Marcel, Dr.**
**Ouellenweg 10**
**CH-4402 Frenkendorf(CH)**
Erfinder: **Trzeciak, Arnold**
**Talstrasse 54**
**D-7860 Schopfheim(DE)**
Erfinder: **Weller, Thomas, Dr.**
**Riehenring 71**
**CH-4058 Basel(CH)**

(74) Vertreter: **Lederer, Franz, Dr. et al**
**Lederer, Keller & Riederer**
**Patentanwälte**
**Prinzregentenstrasse 16**
**D-80538 München (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 381 033 B1

## Beschreibung

Die vorliegende Erfindung betrifft neue Carbonsäure- und sulfonsäureamide der allgemeinen Formel

$R^1$-A-$(W)_a$-X-$(CH_2)_b$-$(Y)_c$-B-Z-COOR (I)

worin

A einen Rest

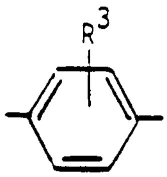 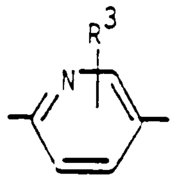 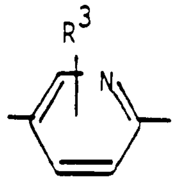 oder 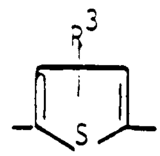 ;

B einen Rest

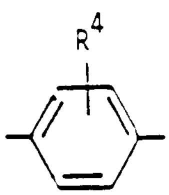 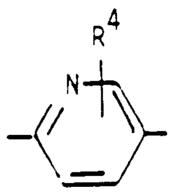 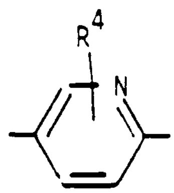 oder 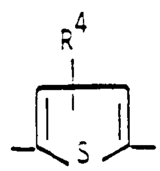 ;

W $-CH_2-$, $-CH_2CH_2-$, -CH = CH-, $-CH = CH-CH_2-$,

| | |
|---|---|
| W | $-CH_2-$, $-CH_2CH_2-$, -CH = CH, $-CH = CH-CH_2-$, $-(CH_2)_3-$, $-CH_2CH(CH_3)-$, $-COCH_2-$, -CH(OH)-$CH_2$- oder $-CH_2COCH_2-$; |
| X | $-CONR^2-$, $-NR^2CO-$, $-SO_2NR^2-$ oder $-NR^2SO_2-$; |
| Y | $-CH_2CH_2-$, $-CH_2CH_2O-$, $-OCH_2-$, $CH(CH_3)CH_2-$, -CH = CH-, $-CH_2-CH = CH-$, $-C(Q^1,Q^2)-CO-(CH_2)d-$,$-CH_2-$, $-CH_2CH_2CH_2-$, $-CH(CH_3)CH_2CH_2-$, $-CH_2COCH_2-$, $-C(Q^1,Q^2)-CH(OH)-$, $-C-(Q^1,Q^2)-CH(SSCH_3)-$, $-CH(CH_2OH)CH_2-$ oder $-CH(COOR)CH_2-$ bedeutet, wobei Carbonylgruppen auch als Oxim, Oximäther, Ketal oder Thioketal oder Enoläther und Hydroxygruppen als nieder-Alkyläther, Di(niederalkyl)amino-niederalkyläther oder als Ester von niederen Alkancarbonsäuren vorliegen können |
| Z | $-OCH_2-$, $-NR^6CH_2-$, $-CH_2CH_2-$, $-CH(CH_3)CH_2-$, $-CH_2-$, -CH = CH- oder $-C(CH_3) = CH-$; |
| R | Wasserstoff, nieder-Alkyl, Phenyl oder Phenyl-nieder-Alkyl; |
| $Q^1$ und $Q^2$ | Wasserstoff oder nieder-Alkyl sind oder zusammen mit dem C-Atom, an dem sie gebunden sind, einen 3- bis 6-gliedrigen gesättigten Ring bilden; |
| $R^1$ | Amidino oder Guanidino; |
| $R^2$ | Wasserstoff, nieder-Alkyl, Phenyl-nieder-alkyl, im Phenylteil durch Amino, Amidino oder -COOR substituiertes Phenyl-nieder-Alkyl, oder einen Rest $-CH_2COOR$ oder -Y-B-Z-COOR; |
| $R^3$ | Wasserstoff, nieder-Alkyl, nieder-Alkoxy, Halogen, nieder-Carbalkoxy, Amino, nieder-Alkylamino, di-nieder-Alkylamino oder Amidino; |
| $R^4$ | Wasserstoff, nieder-Alkyl, nieder-Alkoxy, Halogen, nieder-Carbalkoxy, Amino, nieder-Alkylamino, di-nieder-Alkyl-amino, oder einen Rest -Z-COOR oder $-CH = CH-(CH_2)_nCOOR$; |
| $R^6$ | Wasserstoff, nieder-Alkyl oder Benzyl; |
| n | eine ganze Zahl von 0-4; |
| a, c und d | unabhängig von einander 0 oder 1; und |
| b | eine ganze Zahl von 0-2 bedeuten, wobei jedoch a und b 0 sein sollen, wenn c 1 ist, und c 0 sein soll, wenn a oder b von 0 verschieden ist; |

und physiologisch verträgliche Salze davon.

Falls in den Verbindungen der obigen Formel I a = b = c = o ist und X für CONH steht, können zwei Phenylengruppen A und B über die besagte Gruppe CONH gebunden sein. Hingegen sind die zwei Phenylengruppen in den Benzoylester der EP-A-0 214 429 über eine Gruppe COO gebunden.

Die Erfindung betrifft weiterhin ein Verfahren zur Herstellung der Verbindungen der Formel I und ihrer Salze, ihre Verwendung zur Herstellung von Heilmitteln zur Behandlung von Thrombosen, Apoplexie, Herzinfarkt, Entzündungen, Arteriosklerose sowie von Tumoren; sowie Heilmittel, die diese Verbindungen enthalten.

Der Ausdruck "nieder" bezeichnet Gruppen mit 1-6 C-Atomen. Beispiele niederer Alkylgruppen für sich allein oder als Bestandteil von Alkoxy oder Alkylamino sind Methyl, Aethyl, Propyl, Isopropyl, n-Butyl, sek.-Butyl oder tert.-Butyl.

Beispiele von nieder-Alkancarbonsäuren, mit denen die in Y gegebenenfalls vorhandenen Hydroxygruppen verestert sein können, sind Essigsäure, Propionsäure und Buttersäure. Beispiele von Ketal- und Thioketalgruppen sind nieder-Alkyl- und nieder-Alkylenketale bzw. -thioketale wie Dimethoxy-, Aethylendioxy-, Dimethylthio- und Aethylendithiogruppen. Beispiele niederer Alkyläther sind Methyl- und Aethyläther. Beispiele von Enoläthergruppen Y sind die Gruppen $-CH=C(OCH)_3-$ und $-CH_2-C(OCH_3)=CH-$.

Die Verbindungen der Formel I können solvatisiert, insbesondere hydratisiert sein. Die Hydratisierung kann im Zuge des Herstellungsverfahrens erfolgen oder allmählich als Folge hygroskopischer Eigenschaften einer zunächst wasserfreien Verbindung der Formel I auftreten.

Beispiele von physiologisch verträglichen Salzen der Verbindungen der Formel I sind Salze mit physiologisch verträglichen Mineralsäuren, wie Salzsäure, Schwefelsäure oder Phosphorsäure, oder mit organischen Säuren, wie Methansulfonsäure, p-Toluolsulfonsäure, Essigsäure, Trifluoressigsäure, Zitronensäure, Fumarsäure, Bernsteinsäure oder Salicylsäure. Die Verbindungen der Formel I können auch Salze mit physiologisch verträglichen Basen bilden. Beispiele solcher Salze sind Alkalimetall-, Erdalkalimetall-, Ammonium- und Alkylammoniumsalze, wie Na-, K-, Ca- oder Trimethylammoniumsalz. Die Verbindungen der Formel I können auch in Form von Zwitterionen vorliegen.

Die Verbindungen der Formel I, die ein oder mehrere asymmetrische C-Atome enthalten, können als Enantiomere, als Diastereomere oder als Gemische davon, z.B. als Racemate, vorliegen.

Eine Untergruppe der Verbindungen der Formel I sind die Verbindungen der Formel

$R^1$-A-X-Y-B-Z-COOR (Ia)

Eine andere Untergruppe von Verbindungen der Formel sind die Verbindungen der Formel

$R^1$-A-W-X-$(CH_2)_b$-B-Z-COOR (Ib)

Bevorzugte Verbindungen der allgemeinen Formel I sind solche, in denen die Summe der in $(W)_a$, $(CH_2)_b$, $(Y)_c$, X und Z in gerader Kette vorliegenden C-, O-, N- und S-Atome 6 ist.

Beispiele solcher bevorzugter Verbindungen sind Verbindungen der Formel Ia, worin Y = $-CH_2CH_2-$, $-CH_2CO-$, $-CH(CH_3)CO-$, $-CH(CH_3)CH(SSCH_3)-$, $-CH(CH_3)CH_2-$, $-CH=CH-$, $-CH_2CHOH-$ oder $-CH(COOR)-CH_2-$; und Z = $-OCH_2-$, $-NR^6CH_2-$, $-CH_2CH_2-$, $-CH(CH_3)CH_2-$, $-CH=CH-$ oder $-C(CH_3)=CH-$; oder Verbindungen der Formel Ib, in denen W = $-CH_2-$, b = 1 und Z $-OCH_2-$, $-NR^6CH_2-$, $-CH_2CH_2-$, $-CH(CH_3)CH_2-$, $-CH=CH-$ oder $-C(CH_3)=CH-$ ist.

Bevorzugt sind ferner Verbindungen der allgemeinen Formel I, in denen $R^1$ Amidino ist, sowie solche, in denen Y $-CH_2CH_2$ oder $-CH_2CO-$; Z $-OCH_2-$ oder $-CH_2CH_2-$und X -NHCO- oder -CONH- ist.

R ist vorzugsweise Wasserstoff.

Die Verbindungen der allgemeinen Formel I und ihre Salze können erfindungsgemäss dadurch hergestellt werden, dass man

a) in einer Verbindung der allgemeinen Formel

NC-A-$(W)_a$-X-$(CH_2)_b$-$(Y)_c$-B-Z-$COOR^5$ (II)

worin $R^5$ nieder-Alkyl oder Benzyl ist und A, B, W, X, Y, Z, a, b und c oben angegebene Bedeutung haben und worin im Molekül gegebenenfalls vorhandene Carboxygruppen als Ester vorliegen,

die Nitrilgruppe in die Amidinogruppe überführt, oder

b) in einer Verbindung der allgemeinen Formel

$R^{11}$-A-$(W)_a$-X-$(CH_2)_b$-$(Y)_c$-B-Z-$COOR^5$ (III)

worin A, B, W, X, Y, Z, $R^5$, a, b und c die oben angegebene Bedeutung haben und $R^{11}$ eine geschützte Amidino- oder Guanidinogruppe darstellt,

die Schutzgruppen der Amidino- oder Guanidinogruppe abspaltet, oder

c) in einer Verbindung der allgemeinen Formel

$H_2N\text{-}A\text{-}(W)_a\text{-}X\text{-}(CH_2)_b\text{-}(Y)_c\text{-}B\text{-}Z\text{-}COOR^5$ (IV)

worin A, B, W, X, Y, Z, $R^5$, a, b und c die oben angegebene Bedeutung haben, und worin im Molekül gegebenenfalls vorhandene Carboxygruppen als Ester vorliegen,
die Aminogruppe in die Guanidinogruppe überführt, oder
d) eine Verbindung der allgemeinen Formel

$R^1\text{-}A\text{-}(W)_a\text{-}COE$ (V)

worin E Chlor oder Brom oder COE eine aktivierte Estergruppe oder Chlor oder Brom darstellt und $R^1$, A, W und a die oben angegebene Bedeutung haben,
mit einer Verbindung der allgemeinen Formel

$HNR^2\text{-}(CH_2)_b\text{-}(Y)_c\text{-}B\text{-}Z\text{-}COOR^5$ (VI)

worin Y, B, Z, $R^5$, $R^2$, b und c die oben angegebene Bedeutung haben,
umsetzt, eine gegebenenfalls anwesende Benzylgruppe $R^5$ abspaltet
und gewünschtenfalls in einer so erhaltenen Verbindung der allgemeinen Formel I anwesende, funktionelle Gruppen abwandelt.

Die Ueberführung der Nitrilgruppe in die Amidinogruppe gemäss Verfahrensvariante a) kann mittels an sich bekannter Methoden bewerkstelligt werden, beispielsweise dadurch, dass man das Nitril mit Schwefelwasserstoff und einer Base wie Triäthylamin in einem Lösungsmittel wie Pyridin zu einem Thioamid umsetzt, dieses z.B. mit Methyljodid in Aceton methyliert und die so erhaltene Methylthioformimidoyl-Verbindung (d.h. eine Verbindung der Formel I, in der anstelle von $R^1$ die Gruppe $-C(NH)SCH_3$ steht) mit Ammoniumacetat umsetzt.

In der Verfahrensvariante b) können Amidino- und Guanidinogruppen durch konventionelle Schutzgruppen wie Benzyloxycarbonyl oder tert.-Butoxycarbonyl geschützt sein. Beispiele von Gruppen $R^{11}$ sind -C(NH)NH-CO-OBenzyl, -C(N-Boc)NH-Boc, $-NHC(NH)NHNO_2$ und -NHC(N-Boc)NH-Boc, wobei Boc für tert.Butoxycarbonyl steht.

Diese Schutzgruppen können in an sich bekannter Weise abgespalten werden, z.B. durch katalytische Hydrierung im Fall einer Benzyloxycarbonylgruppe oder einer Nitrogruppe oder durch Behandlung mit einer Säure wie Trifluoressigsäure im Fall einer tert.Butoxycarbonylgruppe.

Die Ueberführung der Aminogruppe in die Guanidinogruppe nach Verfahrensvariante c) kann durch Umsetzung mit 2-S-Isothioharnstoff-äthansulfonat in Gegenwart einer Base wie $Na_2CO_3$ oder NaOH bei Temperaturen bis zu etwa 40 °C erfolgen.

Beispiele von aktivierten Estergruppen COE in den Verbindungen der Formel V sind der p-Nitrophenylester oder der 4,6-Dimethoxy-1,3,5-triazin-2-yl-ester. Die Umsetzung des aktivierten Esters V mit dem Amin VI gemäss Verfahrensvariante d) kann in an sich bekannter Weise in Gegenwart einer Base, wie Triäthylamin, N-Methylmorpholin oder Pyridin vorgenommen werden.

Eine im Verfahrensendprodukt enthaltene Benzylestergruppe $R^5$ kann in an sich bekannter Weise hydrogenolytisch, z.B. durch Hydrierung in Gegenwart eines Edelmetallkatalysators wie Pd oder $PtO_2$ abgespalten werden. Als Abwandlung funktioneller Gruppen in einer Verbindung der Formel I kommen in Betracht die Verseifung von Estergruppen $R^5$ und von Estergruppen, die in den Resten $R^2$, $R^3$, $R^4$ und Y anwesend sein können; die Spaltung von Aether- oder Enoläthergruppen, Ketal- oder Thioketalgruppen, die im Rest Y enthalten sein können; die Hydrierung von olefinischen Doppelbindungen im Rest Y oder Z; und die Ueberführung von Carbonsäuregruppen in Salze.

Diese Abwandlungen können in an sich bekannter Weise vorgenommen werden, z.B. durch Behandlung mit Basen wie wässrig-alkoholischer NaOH zur Verseifung von Estergruppen oder durch Behandlung mit Säuren, wie wässriger alkoholischer Salzsäure zur Spaltung von Aether- und Ketalgruppen, oder durch katalytische Hydrierung einer in Y oder Z enthaltenen -C = C-Bindung.

Die Ausgangsverbindungen der Formeln II-VI können wie nachstehend beschrieben hergestellt werden:

Ein Säurederivat der allgemeinen Formeln

$R^x\text{-}A\text{-}(W)_a\text{-}COE$ (VII)

oder

$R^x$-A-$(W)_a$-$SO_2$E (VIII)

worin $R^x$ -CN, eine Gruppe $R^{11}$ oder eine geschützte Aminogruppe und E eine aktivierte Estergruppe oder Chlor oder Brom darstellt,
kann mit einem Amin der allgemeinen Formel

HN($R^2$)-$(CH_2)_b$-$(Y)_c$-B-Z-COOR$^5$ (IX)

zu einer Verbindung der Formeln II, III oder IV umgesetzt werden, in denen X eine Gruppe -CONR$^2$ - oder -$SO_2$NR$^2$ darstellt.

Analog kann ein Säurederivat der allgemeinen Formeln

ECO-$(CH_2)_b$-$(Y)_c$-B-Z-COOR$^5$ (X)

oder

E$SO_2$-$(CH_2)_b$-$(Y)_c$-B-Z-COOR$^5$ (XI)

mit einem Amin der allgemeinen Formel

$R^x$-A-$(W)_a$-NHR$^2$ (XII)

zu einer Verbindung der Formeln II, III oder IV umgesetzt werden, in denen X eine Gruppe -NR$^2$CO- oder -NR$^2$$SO_2$-darstellt.

Die Kondensation kann in an sich bekannter Weise erfolgen, z.B. nach der Methode der aktivierten Ester oder der gemischten Anhydride oder über die Säurechloride. Beispielsweise wird zur Herstellung einer Verbindung VII oder VIII die entsprechende Säure mit 2-Chlor-4,6-dimethoxy-1,3,5-triazin in Gegenwart von N-Methylmorpholin zu einem aktivierten Ester umgesetzt, der in situ mit dem Amin IX umgesetzt werden kann. Als aktivierter Ester kann auch ein p-Nitrophenylester eingesetzt werden. Gemischte Anhydride können durch Umsetzung der Säure mit Chlorameisensäure-isobutylester erhalten werden. Alternativ kann ein Halogenid der Formel VII oder VIII in Gegenwart einer Base wie Triäthylamin mit einem Amin IX umgesetzt werden.

Sofern die Reaktionspartner in den Gruppen A und B primäre oder sekundäre Aminogruppen oder Carboxylgruppen enthalten, werden diese Gruppen zweckmässig geschützt. Eine geeignete Schutzgruppe für Aminogruppen ist, z.B. tert.-Butoxycarbonyl. Eine Carboxygruppe kann durch Veresterung, z.B. als Alkylester geschützt werden. Die Einführung und Entfernung solcher Gruppen kann in an sich bekannter Weise erfolgen.

Eine geschützte Guanidinogruppe, z.B. -NHC(N-Boc)-NH-Boc, kann aus einer Aminogruppe durch Umsetzung des Amins in t-Butanol, Wasser und Triäthylamin mit N,N′-Bis(t-butoxycarbonyl)-S-methylisothioharnstoff aufgebaut werden.

Ausgangsverbindungen VII oder VIII mit einer geschützten Amidinogruppe können dadurch hergestellt werden, dass man ein entsprechendes Nitril wie oben für die Verbindung II beschrieben, in das Amidin überführt und dieses mit einem die Schutzgruppe liefernden Reagens umsetzt. Beispielsweise kann durch Umsetzung eines Amidins mit Chlorameisensäurebenzylester in Gegenwart von Triäthylamin eine geschützte Amidinogruppe der Formel -C(NH)NH-CO-OBenzyl gebildet werden.

Die Verbindungen der Formel I, ihre Solvate und ihre Salze hemmen sowohl die Bindung von Fibrinogen, Fibronectin und des von Willebrand-Faktors an den Fibrinogenrezeptor der Blutplättchen (Glykoprotein IIb/IIIa) als auch die Bindung derselben und weiterer adhäsiver Proteine, wie Vitronectin, Kollagen und Laminin, an die entsprechenden Rezeptoren auf der Oberfläche verschiedener Zelltypen. Die erfindungsgemässen Verbindungen beeinflussen somit Zell-Zell- und Zell-Matrix-Wechselwirkungen. Sie verhindern insbesondere die Entstehung von Blutplättchenthromben und können bei der Bekämpfung bzw. Verhütung von Krankheiten wie Thrombose, Apoplexie, Herzinfarkt, Entzündungen und Arteriosklerose verwendet werden. Ferner haben diese Verbindungen einen Effekt auf Tumorzellen, indem sie deren Metastasierung hemmen. Somit können sie auch als Antitumor-Mittel eingesetzt werden.

Die Hemmung der Fibrinogenbindung an den Fibrinogenrezeptor, Glykoprotein IIb/IIIa, kann wie folgt nachgewiesen werden:

Das Glykoprotein IIb/IIIa wird aus Triton X-100 Extrakten von menschlichen Blutplättchen gewonnen und

durch Lectin-Aff initätschromatographie (Analytical Biochemistry 151, 1985, 169-177) und Chromatographie an einer Arg-Gly-Asp-Ser-Affinitätssäule (Science 231, 1986, 1559-62) gereinigt. Das so erhaltene Rezeptorprotein wird an Mikrotiterplatten gebunden. Die spezifische Bindung von Fibrinogen an den immobilisierten Rezeptor wird mit Hilfe eines ELISA-Systems ("enzyme-linked immunosorbent assay") bestimmt. Die nachstehenden $IC_{50}$-Werte entsprechen derjenigen Konzentration der Testsubstanz, welche benötigt wird, um die Bindung von Fibrinogen an den immobilisierten Rezeptor um 50% zu hemmen:

| Produkt von Beispiel: | 1 | 2 | 10 | 12 | 18 |
|---|---|---|---|---|---|
| $IC_{50}$ (µM) | 0,37 | 0,04 | 0,64 | 0,1 | 0,72 |

| Produkt von Beispiel: | 20 | 36 | 40 | 46 | 48 | 78 |
|---|---|---|---|---|---|---|
| $IC_{50}$ (µM) | 0,47 | 0,1 | 0,04 | 0,3 | 0,03 | 0,97 |

| Produkt von Beispiel: | 81 | 82 | 83 |
|---|---|---|---|
| $IC_{50}$ (µM) | 0,08 | 0,0081 | 0,00007 |

Arzneimittel, enthaltend eine Verbindung der Formel I, ein Solvat davon oder ein Salz davon können enteral, z.B. oral in Form von Tabletten, Lacktabletten, Dragées, Hart- und Weichgelatinekapseln, Lösungen, Emulsionen oder Suspensionen, oder rektal, z.B. in Form von Suppositorien, oder als Spray verabreicht werden. Die Verabreichung kann aber auch parenteral, z.B. in Form von Injektionslösungen, erfolgen.

Zur Herstellung von Tabletten, Lacktabletten, Dragées und Hartgelatinekapseln kann der Wirkstoff mit pharmazeutisch inerten, anorganischen oder organischen Excipientien vermischt werden. Als solche Excipientien kann man für Tabletten, Dragées und Hartgelatinekapseln z.B. Lactose, Maisstärke oder Derivate davon, Talk, Stearinsäure oder deren Salze, verwenden. Für Weichgelatinekapseln eignen sich als Excipientien z.B. vegetabile Oele, Wachse, Fette, halbfeste und flüssige Polyole; je nach Beschaffenheit des Wirkstoffes sind jedoch bei Weichgelatinekapseln überhaupt keine Excipientien erforderlich. Zur Herstellung von Lösungen und Sirupen eignen sich als Excipientien z.B. Wasser, Polyole, Saccharose, Invertzucker und Glukose, für Injektionslösungen eignen sich z.B. Wasser, Alkohole, Polyole, Glycerin und vegetabile Oele, und für Suppositorien eignen sich z.B. natürliche oder gehärtete Oele, Wachse, Fette und halbflüssige oder flüssige Polyole. Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten.

Für die Bekämpfung bzw. Verhütung der weiter oben genannten Krankheiten kann die Dosierung des Wirkstoffes innerhalb weiter Grenzen variieren und ist natürlich in jedem einzelnen Fall den individuellen Gegebenheiten anzupassen. Im allgemeinen dürfte bei oraler Verabreichung eine Dosis von etwa 0,1 bis 20 mg/kg, vorzugsweise von etwa 0,5 bis 4 mg/kg, pro Tag für den Erwachsenen angemessen sein.

Beispiel 1

Durch eine Suspension von 2,0 g 4-[2-(p-Cyanobenzamido)äthyl]phenoxyessigsäure-methylester in 24 ml Pyridin/Triäthylamin (7:1) wurde während 45 Minuten bei 0 ° C $H_2S$ geleitet. Nach 12 Stunden wurde die entstandene Lösung eingeengt, der Rückstand in Methanol aufgeschlämmt und filtriert. Der Filterrückstand wurde sogleich in 30 ml Aceton suspendiert und mit 4 ml Methyljodid versetzt. Nach 2,5 Stunden wurde abfiltriert, der Niederschlag mit wenig Aceton gewaschen und nach Trocknen in 20 ml Methanol gelöst. Nach Zugabe von 2,0 g Ammoniumacetat liess man 2 Stunden bei Raumtemperatur reagieren, wobei sich ein farbloser Niederschlag bildete, der abfiltriert und aus Methanol umgefällt wurde. Nach Trocknen im Hochvakuum wurde p-[2-(p-Amidinobenzamido)äthyl]phenoxyessigsäuremethylester als Acetatsalz, Schmelzpunkt über 200 ° C, erhalten.

Herstellung des Ausgangsmaterials:

Zu einer auf 0 ° C gekühlten Suspension von 1,47 g p-Cyanobenzoesäure und 1,79 g 2-Chlor-4,6-dimethoxy-1,3,5-triazin in 40 ml Dichlormethan wurden 1,12 ml N-Methyl-morpholin gegeben. Nach 3 Stunden bei Raumtemperatur wurde das Reaktionsgemisch auf 0 ° C gekühlt und mit einer Suspension von 2,45 g 4-(2-Aminoäthyl)-phenoxyessigsäure-methylester (hergestellt in Analogie zu der in der DE-OS 2809377 beschriebenen Herstellung des Aethylesters) und 1,1 ml N-Methylmorpholin in 40 ml Dichlormethan versetzt. Das Reaktionsgemisch wurde über Nacht bei Raumtemperatur gerührt und anschliessend eingeengt. Der Rückstand wurde in Essigester/Wasser aufgeschlämmt und filtriert, das Filtrat nacheinander mit 1N Salzsäure, Wasser, gesättigter Natriumbicarbonatlösung und Wasser gewaschen, über Natriumsulfat getrocknet und eingedampft. Nach Aufschlämmen des Rückstandes in Aether/Dichlormethan/Hexan wurde filtriert und der erhaltene 4-[2-(p-Cyanobenzamido)äthyl]phenoxyessigsäure-methylester getrocknet, Schmelzpunkt 149-151 ° C.

Beispiel 2

Zu einer Suspension von 800 mg des Acetatsalzes von p-[2-(p-Amidinobenzamido)äthyl]-phenoxyessigsäure-methylester in Methanol wurde eine Lösung von 99 mg Natriumhydroxid in 1,5 ml Wasser gegeben. Nach 2 Stunden bei 63 ° C wurde das Reaktionsgemisch eingeengt, der Rückstand in 20 ml Wasser suspendiert und mit 822 mg p-Toluolsulfonsäure-monohydrat neutralisiert. Der Niederschlag wurde abfiltriert, mit wenig Wasser gewaschen und im Hochvakuum getrocknet, wobei das p-Toluolsulfonsäuresalz von [p-[2-(p-Amidinobenzamido)-äthyl]phenoxy]essigsäure, Schmelzpunkt über 200 ° C erhalten wurde.

Beispiel 3

In Analogie zu Beispiel 1 wurde aus Methyl [4-[2-(p-cyanobenzamido)äthyl]-2-jodphenoxy]acetat das Essigsäuresalz von Methyl [4-[2-(p-amidinobenzamido)äthyl]-2-jodphenoxy]acetat, Schmelzpunkt 205-206 ° C, erhalten.

Das Ausgangsmaterial wurde wie folgt hergestellt:

Zu einer Suspension von 6,85 g Tyramin in 75 ml Tetrahydrofuran tropfte man eine Lösung von 8,25 g 4-Cyanobenzoesäurechlorid in 25 ml Tetrahydrofuran. Nach Zugabe von 4 ml Pyridin bildete sich ein dicker Brei, welcher nach 1 Stunde bei Raumtemperatur eingeengt wurde. Der Rückstand wurde zwischen Essigester/1N Salzsäure verteilt, zweimal mit Essigester extrahiert, der Extrakt mit Wasser gewaschen, über Natriumsulfat getrocknet und eingedampft. Nach Verrühren des Rückstandes mit Methylenchlorid erhielt man 5,8 g eines farblosen Pulvers, welches in 50 ml Essigsäure suspendiert und mit 6,5 g Jodmonochlorid in 30 ml Essigsäure versetzt wurde. Nach 3 Stunden bei Raumtemperatur wurde das Reaktionsgemisch eingeengt und erneut mit Methylenchlorid verrührt. Der gebildete Niederschlag wurde abgesaugt und getrocknet. Der Rückstand wurde in 50 ml Dimethylformamid gelöst, mit 4,5 g pulverisiertem Kaliumcarbonat und 3,7 g Bromessigsäuremethylester versetzt und 1 Stunde bei Raumtemperatur gehalten. Danach wurde das Reaktionsgemisch zwischen Essigester/0,5N Salzsäure verteilt, mit Essigester extrahiert und aus Aethanol umkristallisiert, wobei Methyl [4-[2-(p-cyanobenzamido)äthyl]-2-jodphenoxy]acetat, Schmelzpunkt 160 ° C, erhalten wurde.

Beispiel 4

Aus dem Essigsäuresalz von Methyl [4-[2-(p-amidinobenzamido)äthyl]-2-jodphenoxy]acetat wurde durch Behandlung mit wässrig-methanolischer Natronlauge bei Raumtemperatur die [4-[2-(p-Amidinobenzamido)-äthyl]-2-jodphenoxy]essigsäure, Schmelzpunkt über 250 °C, erhalten.

Beispiel 5

In Analogie zu Beispiel 1 wurde aus (E)-5-[5-[2-(p-Cyanobenzamido)äthyl]-2-(carbomethoxymethoxy)-phenyl]-4-pentensäurebenzylester durch aufeinanderfolgende Behandlung mit Schwefelwasserstoff, Methyljodid und Ammoniumacetat der (E)-5-[5-[2-(p-Amidinobenzamido)äthyl] -2-(carbomethoxymethoxy)phenyl]-4-pentensäurebenzylester (beiger Schaum nach Chromatographie an Kieselgel mit Dichlormethan/Methanol 19:1 bis 4:1 als Eluens) erhalten.

Das Ausgangsmaterial wurde wie folgt hergestellt:

Ein Gemisch von 464 mg 4-[2-(p-Cyanobenzamido)äthyl]-2-jodphenoxyessigsäuremethylester, 2 ml Allylessigsäurebenzylester, 5,2 mg Triphenylphosphin, 5,2 mg Palladium(II)-acetat und 0,2 ml Triäthylamin wurde unter Argon 6 Stunden auf 100 °C erhitzt, wobei man nach 5 Stunden nochmals dieselben Mengen an Triäthylamin, Triphenylphosphin und Palladium(II)-acetat zufügte. Danach liess man auf Raumtemperatur abkühlen und verdünnte mit Dichlormethan. Nach Chromatographie an Kieselgel (Eluens Aether/Petroläther 1:1) erhielt man 480 mg (E)-5-[5-[2-(p-Cyanobenzamido)äthyl]-2-(carbomethoxymethoxy)phenyl]-4-pentensäurebenzylester als beiges Harz.

Beispiel 6

Durch basische Hydrolyse in Analogie zu Beispiel 2 wurde aus (E)-5-[5-[2-(p-Amidinobenzamido)äthyl] -2-(carbomethoxymethoxy)phenyl]-4-pentensäurebenzylester die (E)-5-[5-[2-(p-Amidinobenzamido)äthyl] -2-(carboxymethoxy)phenyl]-4-pentensäure als Monohydrat vom Schmelzpunkt 247-248 °C (aus Acetonitril/Wasser) erhalten.

Beispiel 7

Aus p-[2-(p-Cyanobenzamido)äthoxyphenyl]essigsäuremethylester erhielt man in Analogie zu Beispiel 1 p-[2-(p-Amidinobenzamido)äthoxyphenyl]essigsäuremethylester als Essigsäuresalz vom Schmelzpunkt 222-223 °C (aus Aethanol).

Das Ausgangsmaterial wurde wie folgt hergestellt:

1,66 g 4-Hydroxyphenylessigsäuremethylester wurden in 30 ml N,N-Dimethylformamid in Gegenwart von 2,8 g Kaliumcarbonat mit 3:05 g N-Benzyloxycarbonyl-2-amino-äthyljodid umgesetzt. Nach 2 Stunden bei 100 °C dampfte man ein, verteilte den Rückstand zwischen Wasser/Aether und extrahierte mit Aether. Nach Chromatographie des Rohproduktes erhielt man 2,1 g Benzyl [2-[(α-acetoxy-p-tolyl)oxy]äthyl]-carbamat als farbloses, zähes Oel.

Eine Lösung von 1 g Benzyl [2-[(α-acetoxy-p-tolyl)oxy]äthyl]carbamat in Methanol wurde in Gegenwart von 10% Pd-Kohle bis zur Sättigung hydriert. Der nach Filtration und Eindampfen erhaltene Rückstand wurde in Chloroform gelöst, nach Zugabe von 0,75 ml Triäthylamin und 660 mg 4-Cyanobenzoylchlorid 30 Minuten gerührt, danach mit 0,5N Natronlauge, 0,5N Salzsäure und Wasser gewaschen, getrocknet und eingeengt. Der Rückstand wurde aus Essigester umkristallisiert, wobei 620 mg p-[2-(p-Cyanobenzamido)-äthoxyphenyl]essigsäuremethylester, Schmelzpunkt 143-144 °C erhalten wurden.

Beispiel 8

In Analogie zu Beispiel 2 wurde aus dem Essigsäuresalz von p-[2-(p-Amidinobenzamido)äthoxyphenyl]-essigsäuremethylester das p-Toluolsulfonat von p-[2-(p-Amidinobenzamido)äthoxy]phenylessigsäure, farblose Nadeln vom Schmelzpunkt 183-184 °C (Wasser/Acetonitril) erhalten.

Beispiel 9

In Analogie zu Beispiel 1 wurde aus p-[2-(p-Cyanophenylsulfonamido)äthoxy]phenylessigsäure-methylester das Essigsäuresalz von p-[2-(p-Amidinophenylsulfonamido)äthoxy]phenylessigsäure-methylester als farblose Kristalle vom Schmelzpunkt 221-222 °C (Aethanol) erhalten.

Herstellung des Ausgangsmaterials:

Das Ausgangsmaterial wurde aus Benzyl [2-[(α-acetoxy-p-tolyl)oxy]äthyl]carbamat durch Hydrogenolyse des Benzyloxycarbonylrests und anschliessende Umsetzung mit 4-Cyanobenzolsulfonylchlorid in Gegenwart von Triäthylamin erhalten.

Beispiel 10

In Analogie zu Beispiel 2 wurde aus dem Essigsäuresalz von p-[2-(p-Amidinophenylsulfonamido)-äthoxy]phenylessigsäure-methylester das p-Toluolsulfonat der p-[2-(p-Amidinophenylsulfonamido)äthoxy]-phenylessigsäure als farblose Kristalle vom Schmelzpunkt 210-211 °C (aus Wasser) erhalten.

Beispiel 11

In eine Lösung von 1,49 g p-[2-(p-Cyanobenzolsulfonamido)äthyl]phenoxyessigsäure -methylester in 15 ml Pyridin und 1,5 ml Triäthylamin wurde bei 0-5 °C während 60 Minuten Schwefelwasserstoff eingeleitet. Die Reaktionsgemischung wurde dann über Nacht bei Raumtemperatur gehalten und anschliessend im Vakuum zur Trockene verdampft. Man erhielt 1,65 g Thioamid, die in 15 ml Aceton mit 15 ml Methyljodid 3 Stunden unter Rückfluss erwärmt wurden. Das Reaktionsgemisch wurde wiederum im Vakuum eingedampft und der Rückstand in 120 ml Chloroform mit 1,30 g Ammoniumacetat und 1,0 ml Essigsäure 3 Tage bei Raumtemperatur gerührt. Der nach Abdampfen des Lösungsmittels erhaltene Rückstand wurde in Methanol/Wasser gelöst und durch 30 g Levafit M-5080 filtriert. Das Filtrat wurde eingedampft und mit Wasser/Methanol (10:1) über 35 g MCI Gel chromatographiert. Man erhielt 1,0 g reines, amorphes p-[2-(p-Amidinobenzolsulfonamido)äthyl]phenoxyessigsäure -methylester-hydrochlorid. IR-Banden (KBr) bei 3117, 3053, 1756, 1681, 1512, 1333, 1228, 1155, 849 $cm^{-1}$.

Das Ausgangsmaterial wurde wie folgt hergestellt:

In einer Lösung von 1,47 g 4-(2-Aminoäthyl)phenoxyessigsäure-methylester-hydrochlorid (Smp. 190 °) in 75 ml Methylenchlorid und 1,85 ml Triäthylamin gibt man bei 0-5 °C innert 20 Minuten unter Rühren eine Lösung von 1,21 g p-Cyanobenzolsulfochlorid in 20 ml Methylenchlorid. Nach 2 Stunden Rühren bei Raumtemperatur wird wie üblich aufgearbeitet und das Rohprodukt auf Silicagel mit Chloroform/n-Propanol/30% $NH_3$ (1000:10:1) chromatographiert. Nach Umkristallisieren der DC-reinen Fraktionen aus Aceton/Hexan erhält man 1,95 g reinen p-[2-(p-Cyanobenzolsulfonamido)äthyl]phenoxyessigsäure -methylester als farblose Nadeln, Schmelzpunkt 148-149 °C.

Beispiel 12

Eine Lösung von 2,0 g p-[2-(p-Amidinobenzolsulfonamido)äthyl]phenoxyessigsäure -methylester-hydrochlorid in 100 ml Aethanol und 50 ml 1N Natronlauge wird 90 Minuten unter Argon gerührt. Zur Aufarbeitung wird mit verdünnter Salzsäure auf pH 6-7 neutralisiert und im Vakuum auf 50 °C eingeengt. Die abgeschiedenen Kristalle werden abgenutscht und im Vakuum über KOH bei 50 °C getrocknet. Man isoliert 1,70 g reine p-[2-(p-Amidinobenzolsulfonamido)äthyl]phenoxyessigsäure, Schmelzpunkt 292-293 °C.

Beispiel 13

Gemäss Beispiel 11 wurden aus 1,16 g p-[(S)-2-(p-Cyanobenzolsulfonamido)propyl]phenoxyessigsäure -methylester 470 mg p-[(S)-2-(p-Amidinobenzolsulfonamido)propyl]phenoxyessigsäure -methylester-acetat vom Schmelzpunkt 208-210 °C (aus Methanol) erhalten.

Das Ausgangsmaterial wurde durch Reaktion von p-[(S)-2-Aminopropyl]phenoxyessigsäure -methylester-hydrochlorid (Smp. 150-152 °) und p-Cyanobenzolsulfochlorid in Pyridin erhalten. Schmelzpunkt 120-122 °C.

Beispiel 14

Gemäss Beispiel 12 und anschliessende Behandlung mit einem Aequivalent Salzsäure wurde aus p-[(S)-2-(p-Amidinobenzolsulfonamido)propyl]phenoxyessigsäure -methylester-acetat (Beispiel 13) reines, amorphes p-[(S)-2-(p-Amidinobenzolsulfonamido)propyl]phenoxyessigsäure -hydrochlorid erhalten. IR-Banden (KBr) bei 3342, 3102, 1680, 1609, 1510, 1324, 1206, 1161, 847 $cm^{-1}$.

Beispiel 15

Gemäss Beispiel 11 wurden aus 700 mg p-[2-(p-Cyano-N-methylphenylsulfonamido)äthyl]-phenoxyessigsäure-methylester 450 mg reines, amorphes p-[2-(p-Amidino -N-methylphenylsulfonamido)-äthyl]phenoxyessigsäure -methylester-hydrochlorid erhalten. IR-Banden (KBr) bei 3372, 3136, 3047, 1754, 1682, 1610, 1519, 1341, 1219, 1159, 828 $cm^{-1}$.

Das Ausgangsmaterial wurde durch Reaktion von p-[2-(p-Cyanobenzolsulfonamido)äthyl]-phenoxyessigsäure -methylester (siehe Beispiel 1) mit Butyllithium und Methyljodid in Dimethoxyäthan bei Raumtemperatur hergestellt. Schmelzpunkt 82-83 ° C.

Beispiel 16

Gemäss Beispiel 14 wurde aus p-[2-(p-Amidino -N-methylphenylsulfonamido)äthyl]phenoxyessigsäure -methylester-hydrochlorid (Beispiel 15) reines, amorphes p-[2-(p-Amidino-N-methylphenylsulfonamido)-äthyl]phenoxyessigsäure-hydrochlorid erhalten. IR-Banden (KBr) bei 3277, 2942, 1692, 1609, 1512, 1421, 1335, 1227, 1156, 823 $cm^{-1}$.

Beispiel 17

Gemäss Beispiel 11 wurden aus 1,0 g p-[2-(p-Cyano-N-benzylphenylsulfonamido)äthyl]-phenoxyessigsäure-methylester 400 mg reines, amorphes p-[2-(p-Amidino -N-benzylphenylsulfonamido)-äthyl]phenoxyessigsäure -methylester-hydrochlorid erhalten. IR-Banden (KBr) bei 3348, 3031, 1756, 1678, 1607, 1510, 1340, 1210, 1159, 852, 724, 697 $cm^{-1}$.

Das Ausgangsmaterial wurde durch Reaktion von p-[2-(p-Cyanobenzolsulfonamido)äthyl]-phenoxyessigsäure-methylester (siehe Beispiel 1) mit Butyllithium und Benzylbromid in Dimethoxyäthan bei Raumtemperatur erhalten. Schmelzpunkt 108-109 ° C (aus Aceton-Hexan).

Beispiel 18

Gemäss Beispiel 12 wurde aus 340 mg p-[2-(p-Amidino-N-benzylphenylsulfonamido)äthyl]-phenoxyessigsäure-methylester hydrochlorid (Beispiel 17) 240 mg reine p-[2-(p-Amidino-N-benzylphenylsulfonamido)äthyl]phenoxyessigsäure, Schmelzpunkt 275-276 ° C (Zers.) erhalten.

Beispiel 19

Gemäss Beispiel 11 wurden aus 660 mg p-[2-(p-Cyanobenzolsulfonamido)-1-hydroxyäthyl]-phenoxyessigsäuremethylester 300 mg reines p-[2-(p-Amidinobenzolsulfonamido)-1-hydroxyäthyl]-phenoxyessigsäure-methylester-hydrochlorid, Schmelzpunkt 227 ° C (Zers.) erhalten.

Das Ausgangsmaterial wurde durch Reaktion von 4-(2-Amino-1-hydroxyäthyl)phenoxessigsäure -methylester-hydrochlorid (Smp. 123-125 ° C) und p-Cyanobenzolsulfochlorid in Pyridin erhalten. Schmelzpunkt 126-129 ° C (aus Aceton/Aether).

Beispiel 20

Gemäss Beispiel 12 wurde aus 260 mg p-[2-(p-Amidinobenzolsulfonamido)-1-hydroxyäthyl]-phenoxyessigsäure-methylester-hydrochlorid (Beispiel 19) 150 mg reine, amorphe p-[2-(p-Amidinobenzolsulfonamido) -1-hydroxyäthyl]phenoxyessigsäure erhalten. IR-Banden (KBr) bei 3431, 3036, 1688, 1608, 1572, 1511, 1419, 1321, 1152, 831 $cm^{-1}$.

Beispiel 21

Gemäss Beispiel 11 wurden aus 650 mg p-[2-(p-Cyanobenzamido)-1-hydroxyäthyl]phenoxyessigsäure-methylester 450 mg reines p-[2-(p-Amidinobenzamido)-1-hydroxyäthyl]phenoxyessigsäure -methylester-hydrochlorid vom Schmelzpunkt 215-218 ° C erhalten.

Das Ausgangsmaterial wurde durch Reaktion von 4-(2-Amino-1-hydroxyäthyl)phenoxyessigsäure -methylester-hydrochlorid (Smp. 123-125 ° ) und p-Cyanobenzoesäurechlorid in Pyridin erhalten. Schmelzpunkt 140-142 ° C (aus Aceton/Hexan).

Beispiel 22

Gemäss Beispiel 12 wurde aus 450 mg p-[2-(p-Amidinobenzamido)-1-hydroxyäthyl]phenoxyessigsäure-methylester-hydrochlorid 340 mg reine p-[2-(p-Amidinobenzamido)-1-hydroxyäthyl]phenoxyessigsäure, Schmelzpunkt über 250°C, erhalten.

Beispiel 23

Gemäss Beispiel 11 wurden aus 460 mg p-[(p-Cyanophenylsulfonamido)acetyl]phenoxyessigsäure-methylester 250 mg reines p-[(p-Amidinophenylsulfonamido)acetyl]phenoxyessigsäure -methylester-hydrochlorid als weisses Pulver erhalten. IR-Banden (KBr) bei 3375, 3078, 1751, 1680, 1599, 1511, 1333, 1217, 1170, 832 $cm^{-1}$.

Das Ausgangsmaterial wurde durch Oxidation von p-[2-(p-Cyanobenzolsulfonamido)-1-hydroxyäthyl]-phenoxyessigsäure-methylester mit Mangandioxid in Chloroform erhalten. Schmelzpunkt 160-162°C (aus Aceton/Hexan).

Beispiel 24

Gemäss Beispiel 12 wurden aus 180 mg p-[(p-Amidinophenylsulfonamido)acetyl]phenoxyessigsäure-methylesterhydrochlorid (Beispiel 23) 80 mg reine, kristalline p-[(p-Amidinophenylsulfonamido)acetyl]-phenoxyessigsäure erhalten. NMR-Banden bei 2,49; 2.50; 3,30; 4,44(d); 4,35 ppm.

Beispiel 25

Gemäss Beispiel 11 wurden aus 300 mg p-[(p-Cyanobenzamido)acetyl]phenoxyessigsäure-methylester 120 mg reines, kristallines p-[(p-Amidinobenzamido)acetyl]phenoxyessigsäure -methylester-hydrochlorid, Schmelzpunkt 270-271° (Zers.) erhalten.

Das Ausgangsmaterial wurde durch Oxidation von p-[2-(p-Cyanobenzamido) -1-hydroxyäthyl]-phenoxyessigsäure-methylester mit Mangandioxid in Chloroform erhalten. Schmelzpunkt 200-203°C.

Beispiel 26

Eine Mischung von 80 mg p-[(p-Amidinobenzamido)acetyl]phenoxyessigsäure-methylester (Beispiel 25), 40 mg Kaliumcarbonat, 10 ml Aethanol und 4 ml Wasser wurde 4 Stunden bei Raumtemperatur unter Argon gerührt. Die Mischung wurde dann mit 10 ml Wasser versetzt und mit 1N HCl auf pH 6 neutralisiert. Man konzentrierte im Vakuum auf 10 ml und liess über Nacht im Kühlschrank stehen. Der abgeschiedene Niederschlag wurde abgenutscht und über KOH im Vakuum bei 50°C getrocknet. Man erhielt 52 mg amorphe p-[(p-Amidinobenzamido)acetyl]phenoxyessigsäure,IR-Banden (KBr) bei 3354, 3286, 3042, 1647, 1598, 1542, 1485, 1422, 1360, 1232, 1177, 994 und 710 $cm^{-1}$.

Beispiel 27

Gemäss Beispiel 11 wurden aus 300 mg p-[2-[p-Cyano-N-(p-carbomethoxybenzyl)phenylsulfonamido]-äthyl]phenoxyessigsäuremethylester 170 mg reines p-[2-[p-Amidino-N-(p -carbomethoxybenzyl)-phenylsulfonamido]äthyl]phenoxyessigsäure -methylester-hydrochlorid erhalten. IR-Banden (KBr) bei 3380, 2952, 1757, 1719, 1684, 1611, 1511, 1285, 1210, 1156, 586 $cm^{-1}$.

Das Ausgangsmaterial wurde durch Reaktion von p-[2-(p-Cyanobenzolsulfonamido)äthyl]-phenoxyessigsäure-methylester (siehe Beispiel 1) mit Butyllithium und p-Carbomethoxybenzylbromid in Dimethoxyäthan bei Raumtemperatur erhalten. Schmelzpunkt 118-119°C (aus Aceton/Hexan).

Beispiel 28

Gemäss Beispiel 12 wurden aus 150 mg p-[2-[p-Amidino-N-(p-carbomethoxybenzyl)-phenylsulfonamido]äthyl]phenoxyessigsäure-methylester-hydrochlorid (Beispiel 27) 84 mg reine, amorphe p-[2-[p-Amidino-N-(p -carboxybenzyl)phenylsulfonamido]äthyl]phenoxyessigsäure erhalten. IR-Banden (KBr) bei 3381, 2930, 1689, 1609, 1546, 1511, 1386, 1335, 1156, 823, 592 $cm^{-1}$.

Beispiel 29

Gemäss Beispiel 11 wurden aus 2,31 g [[4-[2-(p-Cyanophenylsulfonamido)äthyl] -o-phenylen]dioxy]-diessigsäure-dimethylester 1,3 g reines [[4-[2-(p-Amidinophenylsulfonamido)äthyl] -o-phenylen]dioxy]-diessigsäure-dimethylester-hydrochlorid als weisses, amorphes Pulver erhalten. IR-Banden (KBr) bei 3351, 3087, 2857, 1759, 1680, 1596, 1515, 1479, 1330, 1218, 1156, 850 $cm^{-1}$.

Das Ausgangsmaterial wurde durch Reaktion von 3,4-(2-Aminoäthyl)phenylen-dioxy-diessigsäure-dimethylester mit p-Cyanobenzolsulfochlorid in Pyridin erhalten, Schmelzpunkt 130-131 ° C (aus Aceton/Hexan).

Beispiel 30

Gemäss Beispiel 12 wurden aus 500 mg [[4-[2-(p-Amidinophenylsulfonamido)äthyl] -o-phenylen]dioxy]-diessigsäure-dimethylester-hydrochlorid 400 mg reine, kristalline [[4-[2-(p-Amidinophenylsulfonamido)äthyl] -o-phenylen]dioxy]diessigsäure vom Schmelzpunkt 203-205 ° C erhalten.

Beispiel 31

Gemäss Beispiel 11 wurden aus 960 mg [4-[2-(p-Cyanophenylsulfonamido)äthyl] -2-methoxyphenoxy]-essigsäuremethylester 830 mg reines, amorphes [4-[2-(p-Amidinophenylsulfonamido)äthyl] -2-methoxyphenoxy]essigsäure-methylester-hydrochlorid erhalten. IR-Banden (KBr) bei 3365, 3091, 1749, 1681, 1605, 1515, 1329, 1262, 1219, 1151, 850, 806 $cm^{-1}$.

Das Ausgangsmaterial wurde durch Reaktion von 4-(2-Aminoäthyl)-2-methoxyphenoxyessigsäure -methylester-hydrochlorid (Smp. 152-154 °) und p-Cyanobenzolsulfochlorid in Pyridin erhalten, Schmelzpunkt 132-133 ° C.

Beispiel 32

Gemäss Beispiel 12 wurden aus 770 mg [4-[2-(p-Amidinophenylsulfonamido)äthyl] -2-methoxyphenoxy]essigsäure-methylester-hydrochlorid (Beispiel 31) 570 mg reine, kristalline [4-[2-(p-Amidinophenylsulfonamido)äthyl] -2-methoxyphenoxy]essigsäure vom Schmelzpunkt 190-192 ° C (Zers.) erhalten.

Beispiel 33

Gemäss Beispiel 11 wurden aus 1,0 g (E)-p-[2-(p-Cyanophenylsulfonamido)äthyl] -$\beta$-methyl-zimtsäure-methylester 0,80 g reines, amorphes (E)-p-[2-(p-Amidinophenylsulfonamido)äthyl] -$\beta$-methyl-zimtsäure-methylester-hydrochlorid erhalten.

Das Ausgangsmaterial wurde durch Reaktion von 4-(2-Aminoäthyl)-$\beta$-methyl-zimtsäure-methylester-hydrochlorid (Smp. 197-199 °) und p-Cyanobenzolsulfochlorid in Pyridin erhalten, Smp. 118-120 ° C.

Beispiel 34

Gemäss Beispiel 12 wurden unter Verlängerung der Reaktionszeit auf 24 Stunden aus 300 mg (E)-p-[2-(p-Amidinophenylsulfonamido)äthyl] -$\beta$-methyl-zimtsäure-methylester-hydrochlorid 140 mg reine, amorphe (E)-p-[2-(p-Amidinophenylsulfonamido)äthyl] -$\beta$-methyl-zimtsäure erhalten. IR-Banden (KBr) bei 3393, 2926, 1698, 1627, 1556, 1479, 1385, 1329, 1246, 1156, 1093, 832, 597 $cm^{-1}$.

Beispiel 35

300 mg (E)-p-[2-(p-Amidinophenylsulfonamido)äthyl]-$\beta$-methyl-zimtsäure-methylester-hydrochlorid wurden in 40 ml Methanol und 40 ml Essigsäure gelöst und nach Zugabe von 300 mg 10% Pd/C-Katalysator bis zur Aufnahme von 1 Aequivalent Wasserstoff bei Normaldruck und Raumtemperatur hydriert. Nach Abfiltrieren des Katalysators und Eindampfen des Lösungsmittels erhielt man 273 mg amorphes p-[2-(p-Amidinophenylsulfonamido)äthyl] -$\beta$-methyl-hydrozimtsäure-methylester-hydrochlorid.

Beispiel 36

Gemäss Beispiel 12 wurden unter Verlängerung der Reaktionszeit auf 4 Stunden aus 200 mg p-[2-(p-Amidinophenylsulfonamido)äthyl] -$\beta$-methyl-hydrozimtsäure-methylester-hydrochlorid 120 mg reine, kristalline p-[2-(p-Amidinophenylsulfonamido)äthyl] -β-methyl-hydrozimtsäure, Schmelzpunkt oberhalb 200 °C, erhalten.

Beispiel 37

Gemäss Beispiel 11 wurden aus 3,70 g (E)-5-[(RS)-2-(p-Cyanobenzamido)propyl] -β-methyl-2-thiophen-acrylsäure-äthylester-hydrochlorid 2,3 g amorphes (E)-5-[(RS)-2-(p-Amidinobenzamido)propyl] -β-methyl-2-thiophen-acrylsäure-äthylester-hydrochlorid erhalten. IR-Banden (KBr) bei 3254, 3057, 1700, 1679, 1638, 1611, 1541, 1485, 1290, 1164, 861, 712 $cm^{-1}$.

Das Ausgangsmaterial wurde durch Reaktion von (E)-5-[(RS)-2-Aminopropyl]-β-methyl -2-thiophen-acrylsäure-äthylester mit p-Cyanobenzoylchlorid in Pyridin hergestellt, Schmelzpunkt 153-154 °C (aus Aceton/Hexan).

Beispiel 38

Gemäss Beispiel 34 wurden aus 435 mg (E)-5-[(RS)-2-(p-Amidinobenzamido)propyl]-β-methyl -2-thiophen-acrylsäure-äthylester-hydrochlorid (Beispiel 37) 220 mg reine (E)-5-[(RS)-2- (p-Amidinobenzamido)propyl]-β-methyl -2-thiophen-acrylsäure erhalten. Schmelzpunkt oberhalb 230 °C.

Beispiel 39

Gemäss Beispiel 35 wurden aus 700 mg (E)-5-[(RS)-2-(p-Amidinobenzamido)propyl]-β-methyl -2-thiophen-acrylsäure-äthylester-hydrochlorid 360 mg reines, kristallines (RS)-5-[(RS)-2-(p-Amidinobenzamido)propyl]-β-methyl -2-thiophen-propionsäure-äthylester-hydrochlorid erhalten. IR-Banden (KBr) bei 3397, 2929, 1733, 1680, 1636, 1548, 1485, 1278, 1021, 864 $cm^{-1}$.

Beispiel 40

Gemäss Beispiel 12 wurden aus 320 mg (RS)-5-[(RS)-2-(p-Amidinobenzamido)propyl]-β-methyl -2-thiophen-propionsäure-äthylester-hydrochlorid (Beispiel 39) 260 mg farblose, amorphe (RS)-5-[(RS)-2-(p-Amidinobenzamido)propyl)-β-methyl -2-thiophen-propionsäure erhalten. IR-Banden (KBr) bei 3262, 2928, 1633, 1557, 1499, 1401, 1155, 869, 698 $cm^{-1}$.

Beispiel 41

Gemäss Beispiel 11 wurden aus 770 mg p-[2-(p-Cyanobenzamido)äthyl]-hydrozimtsäure-methylester 530 mg reines p-[2-(p-Amidinobenzamido)äthyl]-hydrozimtsäure -methylester-hydrochlorid erhalten. IR-Banden (KBr) bei 3297, 3088, 1735, 1682, 1633, 1548, 1487, 1295, 1172, 864 $cm^{-1}$.

Das Ausgangsmaterial wurde durch Reaktion von 4-(2-Aminoäthyl)hydrozimtsäure-methylester-hydrochlorid mit p-Cyanobenzoylchlorid in Methylenchlorid in Gegenwart von Triäthylamin erhalten. Schmelzpunkt 142-144 °C.

Beispiel 42

Gemäss Beispiel 12 wurden aus 490 mg p-[2-(p-Amidinobenzamido)äthyl]-hydrozimtsäure -methylester-hydrochlorid 3430 mg reine kristalline p-[2-(p-Amidinobenzamido)äthyl]-hydrozimtsäure, Schmelzpunkt oberhalb 230 °C, erhalten.

Beispiel 43

Gemäss Beispiel 11 wurden aus 1,0 g p-[3-(p-Cyanobenzamido)propyl]phenylessigsäure -methylester-hydrochlorid 0,75 g reines p-[3-(p-Amidinobenzamido)propyl]phenylessigsäure -methylester-hydrochlorid erhalten. IR-Banden (KBr) bei 3261, 3059, 1734, 1680, 1638, 1545, 1484, 1436, 1155, 1014, 863 $cm^{-1}$.

Das Ausgangsmaterial wurde durch Reaktion von 4-(3-Aminopropyl phenylessigsäure-methylester-hydrochlorid (Smp. 168-171 °) mit p-Cyanobenzoylchlorid in Methylenchlorid und in Gegenwart von Triäthylamin erhalten. Schmelzpunkt 122-124 °C (aus Aceton/Hexan).

Beispiel 44

Gemäss Beispiel 12 und anschliessende Ueberführung mit HCl in das Hydrochlorid wurde aus 650 mg p-[3-(p-Amidinobenzamido)propyl]phenylessigsäure -methylester-hydrochlorid 420 mg p-[3-(p-Amidinobenzamido)propyl]phenylessigsäure-hydrochlorid als amorphes Pulver erhalten. IR-Banden (KBr) bei 3498, 2923, 1670, 1643, 1545, 1517, 1389, 1280, 1136, 839, 707 $cm^{-1}$.

Beispiel 45

Gemäss Beispiel 11 wurden aus 1,0 g p-[3-(p-Cyanophenylsulfonamido)propyl]phenylessigsäure-methylester 510 mg reines, amorphes p-[3-(p-Amidinophenylsulfonamido)propyl]phenylessigsäure-methylester-hydrochlorid erhalten. IR-Banden (KBr) bei 3420, 3257, 3105, 1718, 1659, 1775, 1520, 1477, 1339, 1227, 1152, 851, 806 $cm^{-1}$.

Das Ausgangsmaterial wurde durch Reaktion von 4-(3-Aminopropyl)phenylessigsäure-methylester-hydrochlorid (Smp. 168-171°) mit p-Cyanobenzolsulfochlorid in Methylenchlorid und in Gegenwart von Triäthylamin erhalten. Schmelzpunkt 149-151°C (Aceton/Hexan).

Beispiel 46

Gemäss Beispiel 12 wurden aus 510 mg p-[3-(p-Amidinophenylsulfonamido)propyl]phenylessigsäure-methylester-hydrochlorid (Beispiel 45) 340 mg reine, kristalline p-[3-(p-Amidinophenylsulfonamido)propyl]-phenylessigsäure, Schmelzpunkt oberhalb 250°C, erhalten.

Beispiel 47

Gemäss Beispiel 11 wurden aus 1,0 g p-[2-(4-Cyano-3-pyridylamido)äthyl]phenoxyessigsäure -methylester 890 mg reines, amorphes p-[2-(4-Amidino-3-pyridylamido)äthyl]phenoxyessigsäure -methylester-hydrochlorid erhalten. IR-Banden (KBr) bei 3430, 3242, 3063, 1762, 1695, 1643, 1515, 1437, 1215, 1081 $cm^{-1}$.

Das Ausgangsmaterial wurde durch Reaktion von 6-Cyano-nicotinsäure (Smp. 184-186°) mit Chlorameisensäureäthylester in Gegenwart von 4-Aethylmorpholin und anschliessende Umsetzung mit 4-(2-Aminoäthyl)-phenoxyessigsäure-methylester-hydrochloridin Tetrahydrofuran erhalten. Schmelzpunkt 147-149°C (aus Aceton/Hexan).

Beispiel 48

Gemäss Beispiel 12 wurden aus 800 mg p-[2-(4-Amidino-3-pyridylamido)äthyl]phenoxyessigsäure -methylester-hydrochlorid (Beispiel 47) 625 mg reines, kristallines p-[2-(4-Amidino-3-pyridylamido)äthyl]-phenoxyessigsäure erhalten. Schmelzpunkt >200°C.

Beispiel 49

Gemäss Beispiel 11 wurden aus 530 mg p,p′-[[(p-Cyanobenzoyl)imino]diäthylen]-dihydrozimtsäure-dimethylester 220 mg reines, amorphes p,p′-[[(p-Amidinobenzoyl)imino]diäthylen]-dihydrozimtsäure-dimethylester-hydrochlorid erhalten. IR-Banden (KBr) bei 3010, 2945, 1794, 1682, 1612, 1514, 1495, 1205, 1018, 851 $cm^{-1}$.

Das Ausgangsmaterial wurde durch Reaktion von p-Cyanobenzoylchlorid mit N,N-Bis-[4-(2-aminoäthyl)-dihydrozimtsäure-dimethylester erhalten. Letzteres wurde durch katalytische Hydrierung von 4-(2-Nitroäthylen)zimtsäuremethylester in Methanol/Pd/C erhalten.

Beispiel 50

Gemäss Beispiel 12 wurden aus 210 mg p,p′-[[(p-Amidinobenzoyl)imino]diäthylen]-dihydrozimtsäure -dimethylester-hydrochlorid 115 mg reine, kristalline p,p′-[[(p-Amidinobenzoyl)imino]diäthylen]-dihydrozimtsäure erhalten, Schmelzpunkt >220°C.

Beispiel 51

Gemäss Beispiel 11 wurden aus 940 mg p-[2-[(p-Cyanophenyl)carbamoyl]äthyl]phenoxyessigsäure -methylester 600 mg kristallines p-[-2-[(p-Amidinophenyl)carbamoyl]äthyl]phenoxyessigsäure -methylester-hydrochlorid erhalten. Smp. 230-232 °C.

Das Ausgangsmaterial wurde durch Reaktion von 3-(4-Hydroxyphenyl)propionsäure mit Chlorameisensäure-äthylester, 4-Aethylmorpholin und p-Aminobenzonitril zum p-[2-[(p-Cyanophenyl)carbamoyl]äthyl]-phenol (Smp. 169-172 °C) und anschliessende Verätherung mit Bromessigsäure-methylester/KOH in Aceton erhalten. Smp. 140-143 °C (aus Aceton-Hexan).

Beispiel 52

Gemäss Beispiel 12 wurden aus 570 mg p-[2-[(p-Amidinophenyl)carbamoyl]äthyl]phenoxyessigsäure -methylester-hydrochlorid 450 mg p-[2-[(p-Amidinophenyl)carbamoyl]äthyl]phenoxyessigsäure erhalten. Smp. >250 °C.

Beispiel 53

Gemäss Beispiel 11 wurden aus 1,02 g α-[(p-Cyanobenzyl)carbamoyl]-p-tolyloxyessigsäure -methylester 0,8 g reines, amorphes α-[(p-Amidinobenzyl)carbamoyl]-p-tolyloxyessigsäure -methylester-hydrochlorid erhalten. IR-Banden (KBr) bei 3376, 3263, 3060, 1751, 1654, 1612, 1511, 1180, 1080 $cm^{-1}$ .

Das Ausgangsmaterial wurde durch Reaktion von 4-Hydroxyphenylessigsäure mit Chlorameisensäureäthylester/4-Aethylmorpholinund nachfolgend p-Cyanobenzylamin zu p-[(p-Cyanobenzyl)carbamoyl]methyl]-phenol (Smp. 176-178 °C) und anschliessende Verätherung mit Bromessigsäuremethylester/KOH in Aceton hergestellt. Smp. 143-145 °C (aus Aceton-Hexan). IR-Banden (KBr) bei 3284, 3034, 2232, 1747, 1637, 1610, 1536, 1509, 1374, 1219, 1093, 810 $cm^{-1}$.

Beispiel 54

Gemäss Beispiel 12 wurden aus 391 mg α-[(p-Amidinobenzyl)carbamoyl)-p-tolyloxyessigsäure -methylester-hydrochlorid 305 mg amorphes α-[(p-Amidinobenzyl)carbamoyl]-p-tolyloxyessigsäure erhalten. IR-Banden (KBr) bei 3289, 3044, 1697, 1651, 1612, 1409, 1249, 1222, 1057, 850, 775 $cm^{-1}$.

Beispiel 55

Gemäss Beispiel 11 wurden aus 400 mg p-[(p-Cyanophenäthyl)carbamoyl]-phenoxyessigsäuremethylester 322 mg reines, amorphes p-[(p-Amidinophenäthyl)carbamoyl]-phenoxyessigsäuremethylester-hydrochlorid erhalten. IR-Banden (KBr) bei 3400, 3175, 1755, 1694, 1640, 1608, 1550, 1509, 1435, 1294, 1086, 764 $cm^{-1}$.

Das Ausgangsmaterial wurde durch Reaktion von 4-Hydroxybenzoesäure mit Chlorameisensäureäthylester/4-Aethylmorpholin und nachfolgend Zugabe von p-Cyanophenäthylamin zu p-[(p-Cyanophenäthyl)carbamoyl]phenol und anschliessende Verätherung mit Bromessigsäuremethylester/KOH in Aceton hergestellt.

Beispiel 56

Gemäss Beispiel 12 wurden aus 260 mg p-[(p-Amidinophenäthyl)carbamoyl]phenoxyessigsäure -methylester-hydrochlorid 190 mg farblose, amorphe p-[(p-Amidinophenäthyl)carbamoyl]phenoxyessigsäure erhalten. IR-Banden (KBr) bei 3373, 3057, 1694, 1638, 1608, 1575, 1542, 1500, 1409, 1313, 1227, 1179, 1055, 766 $cm^{-1}$.

Beispiel 57

Gemäss Beispiel 11 wurden aus 1,0 g p-[2-(p-Cyanobenzamido)-1-hydroxy-äthyl]hydrozimtsäure -methylester 0,9 g reines, amorphes p-[2-(p-Amidinobenzamido)-1-hydroxy-äthyl]hydrozimtsäure -methylester-hydrochlorid erhalten. IR-Banden (KBr) bei 3294, 3087, 1729, 1682, 1641, 1545, 1483, 1292, 863 $cm^{-1}$.

Das Ausgangsmaterial wurde durch Reaktion von 4-(2-Amino-1-hydroxyäthyl)hydrozimtsäure -methylester-hydrochlorid (Smp. 141-144 °C) und p-Cyanobenzoesäurechlorid in Methylenchlorid-Triäthylamin erhalten. Smp. 139-141 °C (aus Aceton/Hexan). IR-Banden (KBr) bei 3324, 2237, 1736, 1644, 1549, 1501, 1293, 1170, 841 $cm^{-1}$.

Beispiel 58

Gemäss Beispiel 12 wurden aus 920 mg p-[2-(p-Amidinobenzamido)-1-hydroxyäthyl]hydrozimtsäure -methylester-hydrochlorid 690 mg reine p-[2-(p-Amidinobenzamido)-1-hydroxyäthyl]hydrozimtsäure erhalten. Smp. >250 °C.

Beisiel 59

Gemäss Beispiel 11 wurden aus 2,1 g p-[(p-Cyanobenzamido)acetyl]hydrozimtsäure-methylester 1,41 g farbloses, amorphes p-[(p-Amidinobenzamido)acetyl]hydrozimtsäuremethylester-hydrochlorid erhalten. IR-Banden (KBr) bei 3371, 3045, 1737, 1680, 1650, 1605, 1538, 1484, 1229, 864 $cm^{-1}$.

Das Ausgangsmaterial wird durch Oxidation von p-[2-(p-Cyanobenzamido)-1-hydroxyäthyl]-hydrozimtsäure -methylester (siehe Beispiel 47) mit Mangandioxid in Chloroform erhalten. Smp. 160-161 °C (aus Aceton/Hexan).

Beispiel 60

100 mg p-[(p-Amidinobenzamido)acetyl]hydrozimtsäure -methylester-hydrochlorid werden mit 4 ml 2N Salzsäure 60 Minuten auf 100 °C erhitzt. Das Reaktionsgemisch wird abgekühlt, mit 4 ml Wasser versetzt und 2 Stunden bei 5 °C gerührt. Die ausgefallenen Kristalle werden abgenutscht und im Vakuum über KOH getrocknet. Man erhält 70 mg reines p-[(p-Amidinobenzamido)acetyl]hydrozimtsäure-hydrochlorid, Smp. 273-274 °C (Zers.).

Beispiel 61

Eine Mischung von 486 mg 4-(2-Aminoäthyl)hydrozimtsäure-methylester-hydrochlorid, 672 mg 4-Guanidino-benzoesäure-4-nitrophenylester-hydrochlorid, 404 mg Triäthylamin und 10 ml Dimethylformamid wurde 5 Stunden bei Raumtemperatur unter Argon gerührt. Das Reaktionsgemisch wurde dann im Vakuum zur Trockene eingedampft und der Rückstand auf 25 g MCI-Gel chromatographiert. Man erhielt 520 mg amorphes p-[2-(p-Guanidinobenzamido)äthyl]hydrozimtsäure -methylester-hydrochlorid. IR-Banden (KBr) bei 3339, 3187, 1727, 1678, 1644, 1579, 1500, 1289, 1107, 852 $cm^{-1}$.

Beispiel 62

Gemäss Beispiel 11 wurden aus 450 mg p-[2-(p-Guanidinobenzamido)äthyl]hydrozimtsäure -methylester-hydrochlorid 220 mg p-[2-(p-Guanidinobenzamido)äthyl]hydrozimtsäure als amorphes Pulver erhalten. IR-Banden (Nujol) bei 3507, 3454, 3308, 2925, 1704, 1638, 1554, 1505, 1481, 1376, 1312, 865 $cm^{-1}$.

Beispiel 63

Gemäss Beispiel 61 wurden aus 231 mg p-Aminomethyl-phenoxyessigsäure-methylester-hydrochlorid und 336 mg 4-Guanidino-benzoesäure-4-nitrophenylester-hydrochlorid 310 mg reines, amorphes [α-(p-Guanidinobenzamido)-p-tolyl]oxy-essigsäure -methylester-hydrochlorid isoliert. IR-Banden (KBr) bei 3304, 3169, 1749, 1675, 1641, 1602, 1568, 1507, 1435, 1207, 1080 $cm^{-1}$.

Beispiel 64

Gemäss Beispiel 14 wurden aus 255 mg [α-(p-Guanidinobenzamido)-p-tolyl]oxy-essigsäure -methylester-hydrochlorid 181 mg farbloses, amorphes [α-(p-Guanidinobenzamido)-p-tolyl]oxy -essigsäure-hydrochlorid erhalten. IR-Banden (KBr) bei 3285, 2538, 1674, 1622, 1509, 1419, 1252, 1176, 852, 699 $cm^{-1}$.

Beispiel 65

Gemäss Beispiel 61 wurden aus 193 mg 4-Aminomethyl-hydrozimtsäure-methylesterund 336 mg 4-Guanidino-benzoesäure-4-nitrophenylester 168 mg reines, amorphes p-[(p-Guanidinobenzamido)methyl]-

hydrozimtsäure -methylester-hydrochloriderhalten. IR-Banden (KBr) bei 3367, 2946, 1691, 1601, 1544, 1366, 1252, 1170, 1126, 961 $cm^{-1}$.

Beispiel 66

Gemäss Beispiel 14 wurden aus 137 mg p-[(p-Guanidinobenzamido)methyl]hydrozimtsäure -methylester-hydrochlorid 75 mg farbloses, amorphes p-[(p-Guanidinobenzamido)methyl]hydrozimtsäure-hydrochlorid erhalten. IR-Banden (KBr) bei 3304, 3129, 1703, 1668, 1626, 1567, 1503, 1285, 827 $cm^{-1}$.

Beispiel 67

Eine Lösung von 200 mg [[α-(p-Amidinohydrocinnamamido)-p-tolyl]oxy]essigsäure -methylester-hydrojodid (5:4) in 10 ml Methanol und 0,5 ml 1N Natronlauge wird 2 Stunden und nach Zugabe einer weiteren Portion von 0,5 ml 1N Natronlauge nochmals 2 Stunden bei Raumtemperatur gerührt. Nach Zugabe von 2 ml $H_2O$ wird das Lösungsmittel verdampft, die zurückbleibende wässrige Lösung mit 1M $KHSO_4$-Lösung auf pH 2 eingestellt und mit Essigester extrahiert. Durch Filtration vom Unlöslichen und Reinigung des Rückstands aus der organischen Phase durch Chromatographie (reverse phase) an Octadecyl derivatisiertem Kieselgel Merck RP18 mit $H_2O$ als Elutionsmittel erhält man 77 mg [[α-(p-Amidinohydrocinnamamido)-p-tolyl]oxy]essigsäure als weisses Pulver vom Smp. 180 °C (Zers.).

Der als Ausgangsmaterial eingesetzte Ester kann auf folgende Weise hergestellt werden:

A. Zu einer Suspension von 350 mg p-Cyanohydrozimtsäure und 463 mg p-Aminomethylphenoxyessigsäuremethylester HCl in 20 ml DMF/THF (1:1) fügt man unter Rühren und Kühlen auf 0 °C 404 mg N-Methylmorpholin und 758 mg O-Benzotriazolyl-N,N,N,N-tetramethyluroniumhexafluorophosphat zu. Nach 90 Minuten Reaktionszeit bei Raumtemperatur wird im Hochvakuum zur Trockene verdampft, der ölige Rückstand in Essigester gelöst und diese Lösung mit $NaHCO_3$ 5%, $H_2O$, $KHSO_4$ 2M und gesättigter NaCl-Lösung gewaschen. Das aus der organische Phase erhältliche Rohprodukt wird durch Chromatographieren an Kieselgel mit Essigester als Elutionsmittel gereinigt und aus Essigester/Hexan umkristallisiert: 630 mg Methyl [α-(p-cyanohydrocinnamamido)-p-tolylyoxy]acetat, Smp. 125 °C.

B. Eine Lösung von 585 mg des Produkts aus A. in 60 ml Pyridin und 4 ml Triäthylamin wird bei Raumtemperatur mit $H_2S$ gesättigt und über Nacht bei dieser Temperatur aufbewahrt. Nach dem Verdampfen im R.V. wird der Rückstand in $H_2O$ aufgenommen und mit Essigester extrahiert. Das rohe ölige Produkt wird durch Chromatographie an Kieselgel mit Essigester/MeOH (99:1 v/v) als Elutionsmittel gereinigt. Ausbeute: 560 mg Methyl [(α-[p-(thiocarbamoyl)hydrocinnamamido]-p-tolyl]oxy]acetat, Smp. 135 °C.

C. 560 mg Thioamid des Produkts aus B. werden in 50 ml Aceton gelöst, mit 2 ml Methyljodid versetzt und 45 Minuten unter Rückfluss erhitzt. Das nach dem Verdampfen des Lösungsmittels im R.V. erhältliche rohe Methyl [[α-(p-Methylthiocarboximidohydrocinnamamido) -p-tolyl]oxy]-acetat-hydrojodid wird ohne weitere Reinigung in der nächsten Stufe eingesetzt. Eine Lösung von 575 mg der Vorstufe in 40 ml MeOH wird mit 125 mg $AcONH_4$ versetzt und 6 Stunden unter Rückfluss erhitzt. Der nach dem Verdampfen des Lösungsmittels im R.V. zurückbleibende Rückstand wird mit Essigester/Hexan zur Kristallisation gebracht. Ausbeute: 401 mg Methyl [[α-(p-Amidinohydrocinnamamido) -p-tolyl]oxy]acetat-hydrojodid (5:4), Smp. 155 °C, MS (FAB): 370 $(M+H)^+$.

Beispiel 68

Analog zu Beispiel 67 erhält man bei Verseifung von Methyl [p-(p-Amidinohydrocinnamamido)phenoxy]-acetat-hydrojodid (1:1) die [p-(p-Amidinohydrocinnamamido)phenoxy]essigsäure, Smp. >300 °C (Wasser).

Der als Ausgangsmaterial eingesetzte Ester kann auf folgende Weise hergestellt werden:

A. Analog zu Beispiel 67, Abschnitt A. erhält man durch Kupplung von p-Cyanohydrozimtsäure und p-Aminophenoxyessigsäuremethylester-HCl das Methyl [p-(p-cyanohydrocinnamamido)phenoxy]acetat, Smp. 141 °C (Essigester/Hexan), und daraus in Analogie zu Beispiel 67 Abschnitt B. durch Thionierung das Methyl [p-[p-(thiocarbamoyl)hydrocinnamamido]phenoxy]acetat, Smp. 80 °C (Essigester), Methylierung mit Methyljodid liefert Methyl [p-[p-[1-(methylthio)formimidoyl]hydrocinnamamido]phenoxy] acetat-hydrojodid, Smp. 189 °C (Aceton), aus dem durch Ammonolyse das Methyl [p-(p-amidinohydrocinnamamido)phenoxy]acetat-hydrojodid, Smp. 203-205 °C (Essigester/Hexan) erhalten wird.

Beispiel 69

120 mg [p-[(E)-p-Amidinocinnamamido]phenoxy]essigsäuremethylester werden in 12 ml konz. HCl unter Begasung mit Argon unter Rückfluss erhitzt und noch heiss filtriert. Das erhaltene [p-[(E)-p-Amidinocinnamamido]phenoxy]essigsäure-hydrochloridwird mit wenig konz. HCl und Hexan gewaschen und bei 40 °C im Vakuum über KOH getrocknet. Gelbe Kristalle, Smp. 290 °C (Zers.).

Das Ausgangsmaterial kann auf folgende Weise hergestellt werden:

A. Analog Beispiel 67, Abschnitt A. erhält man durch Kupplung von p-Cyanozimtsäure und p-Aminophenoxyessigsäuremethylester-HCl Methyl [p-[(E)-p-cyanocinnamamido]phenoxy]acetat. Gelbe Kristalle, Smp. 197 °C (Essigester/Hexan).

B. 1,52 g Produkt aus A. werden in 106 ml Dioxan suspendiert, 2 ml Aether und 0,42 g Methanol zugesetzt und das Gemisch auf 5 °C abgekühlt. Unter starkem Rühren werden 1,40 g Chlorwasserstoffgas bei dieser Temperatur eingeleitet. Das Rühren und Kühlen wird noch 3 Stunden fortgesetzt. Uebers Wochenende wird bei Raumtemperatur weitergerührt, das Unlösliche anschliessend abfiltriert, mit Aether gewaschen und im Vakuum getrocknet.

209 mg dieses Produkts werden in 50 ml EtOH suspendiert und das Gemisch auf 5 °C abgekühlt. Hierzu wird so lange äthanolische $NH_3$-Lösung (9 g/100 ml) zugesetzt, bis ein deutlicher $NH_3$-Ueberschuss vorhanden ist. Man erwärmt über Nacht unter Rühren auf 70 °C und filtriert noch heiss vom Unlöslichen. Das Lösungsmittel wird i.R.V. bis auf etwa 5 ml verdampft und der [p-[(E)-p-Amidinocinnamamido]-phenoxy]essigsäuremethylester durch Zugabe von Hexan zur Kristallisation gebracht. Gelbe Kristalle, Smp. ab 235 °C, Ausbeute: 136 mg (68% d.Th.).

Beispiel 70

In Analogie zu Beispiel 1 wird aus N-(p-Cyanobenzoyl)-3-[p-(tert-butoxycarbonyl)methoxy)phenyl]-D-alanin-methylester der N-(p-Amidinobenzoyl)-3-[p-(tert-butoxycarbonyl)methoxy)phenyl]-D-alanin-methylester als hellgelber Schaum erhalten.

Das Ausgangsmaterial kann wie folgt hergestellt werden:

(R)-N-Benzyloxycarbonyl-tyrosin-methylester kann in analoger Weise wie unter Beispiel 3 beschrieben mit Bromessigsäure-t-butylester in Gegenwart von Kaliumcarbonat zu N-Benzyloxycarbonyl-3-[p-[(tert-butoxycarbonyl)methoxy]phenyl] -D-alanin-methylester umgesetzt werden. Das farblose Oel wird in Methanol nach Zugabe von 5-proz. Palladiumkohle in einer Schüttelapparatur bei Raumtemperatur unter Normaldruck hydriert, wobei nach Filtration und Entfernen des Lösungsmittels 3-[p-[(tert-Butoxycarbonyl)methoxy]-phenyl] -D-alanin-methylester in Form eines farblosen Oels gewonnen werden kann.

Aus 1.65 g 3-[p-[tert-Butoxycarbonyl)methoxy]phenyl]-D-alanin-methylester können nach Umsetzung mit 0,97 g 4-Cyanobenzoesäurechlorid und 1,08 g Triäthylamin in Chloroform nach üblicher Aufarbeitung und Chromatographie an Kieselgel mit Aether/Petroläther 1:1 1,8 g N-(p-Cyanobenzoyl)-3-[p-(tert-butoxycarbonyl)methoxy)phenyl] -D-alanin-methylester als farbloses Harz gewonnen werden.

Beispiel 71

462 mg N-(p-Amidinobenzoyl)-3-[p-[(tert-butoxycarbonyl)methoxy]phenyl]-D-alanin-methylester werden in einem Gemisch von 4,5 ml Methylenchlorid und 3,5 ml Trifluoressigsäure 2 Stunden bei Raumtemperatur stehen gelassen. Danach wird im Vakuum eingeengt und der Rückstand mit Diäthyläther verrührt. Das erhaltene orange Pulver wird an Kieselgel mit Wasser und anschliessend mit Wasser/Acetonitril 9:1 chromatographiert. Die nach dem Entfernen der Lösungsmittel gewonnenen gelben Kristalle, 160 mg, [p-[-(R)-2-(p-Amidinobenzamido) -2-(methoxycarbonyl)äthyl]phenoxy]essigsäure-trifluoracetat schmelzen bei 153-155 °C. Optische Drehung $[\alpha]_D^{20}$ = +48,6° (Methanol, c = 0,97).

Beispiel 72

In Analogie zu Beispiel 2 erhält man aus 232 mg des Trifluoracetatsalzes der [p-[(R)-2-(p-Amidinobenzamido)-2-(methoxycarbonyl)äthyl]phenoxy]essigsäure durch Hydrolyse mit methanolischer, wässriger Natronlauge und anschliessender Neutralisation mit p-Toluolsulfonsäure 98 mg N-(p-Amidinobenzoyl)-3-[p-(carboxymethoxy)phenyl]-D-alanin-monohydrat in Form farbloser Kristalle vom Schmelzpunkt 191-193 °C.

Beispiel 73

In Analogie zu Beispiel 1 erhält man aus p-[2-(p-Cyanobenzamido)äthyl]-β-methylzimtsäuremethylester das Acetatsalz von (E)-p-[2-(p-Amidinobenzamido)äthyl]-β-methylzimtsäuremethylester. Schmelzpunkt 196 ° C.

Das Ausgangsmaterial kann aus p-2-Aminoäthyl-β-methylzimtsäuremethylester-hydrochlorid (EP-A1-25331) und p-Cyanobenzoylchlorid hergestellt werden. Schmelzpunkt 162-164 ° C.

Beispiel 74

190 mg (E)-p-[2-(p-Amidinobenzamido)äthyl]-β-methylzimtsäuremethylester ergeben nach Hydrierung in Methanol in Gegenwart von 10% Pd/C unter Normaldruck bei Raumtemperatur nach 24 Stunden 113 mg kristallines Acetat von rac-p-[2-(p-Amidinobenzamido)äthyl]-β-methylhydrozimtsäuremethylester.

Beispiel 75

113 mg rac-p-[2-(p-Amidinobenzamido)äthyl]-β-methylhydrozimtsäuremethylester werden in Methanol/2N Natronlauge gelöst und über Nacht bei Raumtemperatur aufbewahrt. Nach Entfernen des Lösungsmittels wird der Rückstand in heissem Methanol gelöst und mit Aether versetzt. Die ausgefallenen Kristalle werden abgenutscht und im Hochvakuum getrocknet, wobei 52 mg des Natriumsalzes von rac-p-[2-(p-Amidinobenzamido)äthyl]-β-methylhydrozimtsäure erhalten werden. Schmelzpunkt >200 ° C.

Beispiel 76

Zu einer Lösung von 219 mg 4-(2-Aminoäthyl)phenoxyessigsäure-methylester-hydrochlorid in Dimethylformamid (10 ml) gibt man 0,4 ml Triäthylamin gefolgt von einer Lösung von 300 mg 4-Guanidinobenzoesäure-4-nitrophenylester-hydrochlorid. Nach 5 Stunden bei Raumtemperatur wird das Lösungsmittel entfernt und der Rückstand an 20 g Kieselgel mit Essigester/Aceton/Wasser/Essigsäure 9:5:1:1 chromatographiert. Man erhält 103 mg des Acetatsalzes von p-[2-(p-Guanidinobenzamido)äthyl]phenoxyessigsäuremethylester in Form eines blassgelben Harzes.

Beispiel 77

In Analogie zu Beispiel 1 erhält man aus [[α-[2-(p-Cyanophenyl)acetamido]-p-tolyl]oxy]-essigsäuremethylester das Acetatsalz von [[α-[2-(p-Amidinophenyl)acetamido]-p-tolyl]oxy]-essigsäuremethylester. Schmelzpunkt 97 ° C (aus Methanol).

Das Ausgangsmaterial kann wie folgt hergesellt werden (vgl. DE-OS 2320387):

1,61 g p-Aminomethylphenoxyessigsäuremethylester-hydrochlorid werden mit 2,32 g p-Cyanophenylessigsäure in Gegenwart von 1,79 g 2-Chlor-4,6-dimethoxy-1,3,5-triazin, wie in Beispiel 1 beschrieben, umgesetzt. Nach Aufarbeitung und Umkristallisation des Rohproduktes aus Dichlormethan/Aether werden 680 mg [[α-[2-(p-Cyanophenyl)acetamido] -p-tolyl]oxy]essigsäuremethylester mit Schmelzpunkt 147 ° C erhalten.

Beispiel 78

200 mg [[α-[2-(p-Amidinophenyl)acetamido]-p-tolyl]oxy]essigsäuremethylesteracetat werden über Nacht bei Raumtemperatur in 4 ml Methanol/2N Natronlauge 3:1 aufbewahrt. Die ausgefallenen Kristalle werden mit Wasser/Aether gewaschen und im Hochvakuum getrocknet. Man erhält 117 mg des Natriumsalzes von [[p-[2-(p-Amidinophenyl)acetamido]-p-tolyl]oxy]essigsäure. Schmelzpunkt >200 ° C.

Beispiel 79

In Analogie zu Beispiel 1 erhält man aus N-Benzyl-N-[p-[2-(p-cyanobenzamido)äthyl]phenyl]-glycinmethylester den N-Benzyl-N-[p-[2-(p-amidinobenzamido)äthyl]phenyl]glycinmethylester, Schmelzpunkt 193-194 ° C (aus Aethanol).

Herstellung des Ausgangsmaterials (vgl. DE-OS 3622865):

920 mg N-[p-[2-Aminoäthyl]phenyl]glycinmethylestersulfat werden analog zu Beispiel 3 mit 4-Cyanobenzoylchlorid zu N-[p-[2-(p-Cyanobenzamido)äthyl]phenyl]glycinmethylester, Schmelzpunkt 161-162 °C (aus Toluol) umgesetzt.

Zu einer Lösung von 675 mg N-[p-[2-(p-Cyanobenzamido)äthyl]phenyl]glycinmethylester in 15 ml Chloroform gibt man 1,2 ml Triäthylamin und 3 ml Chlorameisensäurebenzylester (50% in Toluol). Das Reaktionsgemisch wird über Nacht bei 50 °C gehalten. Nach Aufarbeitung und Chromatographie an Kieselgel mit Aether/Petroläther 2:1 können 500 mg kristalliner N-Benzyl-N-[p-[2 -(p-cyanobenzamido)-äthyl]phenyl]glycinmethylester isoliert werden. Schmelzpunkt 123-124 °C.

Beispiel 80

Aus 200 mg [p-[2-(p-Guanidinobenzamido)äthyl]phenoxy]essigsäuremethylester-acetat (Beispiel 76) erhält man durch basische Hydrolyse, wie in Beispiel 1 beschrieben, 106 mg [p-[2-(p-Guanidinobenzamido)-äthyl]phenoxy]essigsäure. Schmelzpunkt >200 °C ($H_2O$).

Beispiel 81

a) Aus 3,14 g p-[(R)-2-(p-Cyanobenzamido)-3-hydroxypropyl]phenoxyessigsäure-t-butylester konnten durch aufeinanderfolgende Umsetzung mit $H_2S$/Pyridin, $CH_3I$/Aceton und $NH_4OAc$/Methanol, wie in Beispiel 1 beschrieben, nach Chromatographie an Kieselgel mit Wasser/Methanol (100:0→1:1) 1,2 g p-[-(R)-2-(p-Amidinobenzamido)-3-hydroxypropyl]phenoxyessigsäure-t-butylester in Form eines weissen Schaumes erhalten werden. MS: 428 (M + 1).

b) Aus 427 mg des Produkts von Beispiel 81a) konnten nach Hydrolyse mit 2N HCl/THF (1:1) bei 50 °C und anschliessender Chromatographie an Kieselgel mit Wasser/Methanol (100:0→0:100) 136 mg p-[(R)-2-(p-Amidinobenzamido)-3-hydroxypropyl]phenoxyessigsäure mit Smp. 238-240 °C erhalten werden.

Herstellung des Ausgangsmaterials:

Durch Reduktion von 7,63 g 3-[p-[(tert-Butoxycarbonyl)methoxy]phenyl]-N-(p-cyanobenzoyl)-D-alanin-methylester (Beispiel 70) mit Lithiumborhydrid in Methanol erhält man nach Aufarbeitung und Chromatographie an Kieselgel mit $CH_2Cl_2$/MeOH (100:0→9:1) 3,14 g p-[(R)-2-(p-Cyanobenzamido)-3-hydroxypropyl]-phenoxyessigsäure-t-butylester in Form eines weissen Schaumes, IR = 3389, 2231, 1752, 1645 $cm^{-1}$.

Beispiel 82

Nach Umsetzung von 1,4 g [4-[p-Cyano-N-methylbenzamido)acetyl-o-phenylen]dioxy]-diessigsäuredimethylester, wie in Beispiel 1 beschrieben, isoliert man nach Chromatographie an Kieselgel mit Wasser/Methanol (100:0→4:1) 205 mg [[4-[p-Amidino-N-methylbenzamido)acetyl]-o-phenylen]dioxy]-diessigsäuredimethylester mit Smp. 177-178 °C.

Herstellung des Ausgangsmaterials:

1) Zu einer Lösung von 2,17 g Adrenalon-hydrochlorid in 26 ml DMF/Pyridin (10:3) gab man 1,65 g 4-Cyanobenzoesäurechlorid. Nach 1 Stunde wurde das Reaktionsgemisch auf Eiswasser gegossen; man stellte mit 2N Salzsäure sauer, rührte 35 Minuten und filtrierte die ausgefallenen Kristalle ab. Nach Chromatographie an Kieselgel mit $CH_2Cl_2/CH_3OH$ (100:0→98:2) erhielt man 1,06 g p-Cyano-N-methyl-benzamido-3,4-dihydroxy-acetophenon mit Smp. 223-225 °C (Zers.).

2) Eine Lösung von 3,9 g p-Cyano-N-methylbenzamido-3,4-dihydroxy-acetophenon in 50 ml Aceton wurde in Gegenwart von 3,47 g Kaliumcarbonat 1 Stunde auf 65 °C erhitzt. Danach gab man bei Raumtemperatur 4,8 g Bromessigsäure-methylester tropfenweise zu. Das Reaktionsgemisch wurde 12 Stunden bei Raumtemperatur und 6 Stunden bei 50 °C gehalten. Anschliessend wurden die Lösungsmittel entfernt, der Rückstand mit Eiswasser verrührt und der ausgefallene Niederschlag abfiltriert. Der klebrige Festkörper wurde aus Aether/Essigester (2:1) umkristallisiert, wobei 4,4 g farbloser [4-[p-Cyano-N-methylbenzamido)acetyl-o-phenylen]dioxy]diessigsäuredimethylester mit Smp. 115-117 °C anfielen.

Beispiel 83

Eine Lösung von 600 mg [[4-(p-Amidino-N-methylbenzamido)acetyl]-o-phenylen]dioxy]-diessigsäuredimethylester (Beispiel 82) in 20 ml 10 proz. wässriger Essigsäure wurde über Nacht bei Siedetemperatur gehalten. Anschliessend wurde eingeengt und der Rückstand mit Aethanol zur Kristallisation gebracht. Nach Umkristallisation aus WasserAethanol erhielt man 110 mg [4-(p-Amidino-N-methylbenzamido)acetyl-o-phenylen]dioxy]diessigsäure mit Smp. 230 ° C (Sinterpunkt 190 ° C).

Beispiel 84

Gemäss Beispiel 1 wurden aus 5,40 g p-[N-(p-Cyanobenzoyl)-(RS)-alanyl]phenoxyessigsäure-methylester

a) 1.75 g p-[N-(p-Amidinobenzoyl)-(RS)-alanyl]phenoxyessigsäure-methylester-hydrochlorid erhalten, IR-Banden (KBr) bei 3377, 3049, 1755, 1681, 1600, 1540, 1509, 1483, 1215, 971 $cm^{-1}$ und

b) 700 mg [p-[(all-RS)-2-(p-Amidinobenzamido)-1-(methyldithio)-propyl]phenoxy]essigsäure-methylester-hydrochlorid, IR-Banden (KBr) bei 3249, 3036, 1744, 1680, 1639, 1542, 1484, 1210, 1177, 1079, 708 $cm^{-1}$.

Das Ausgangsmaterial wurde durch Reaktion von p-[(p-Cyanobenzamido)acetyl]phenoxyessigsäure-methylester (Smp. 200-203 °; siehe Beispiel 25) mit Butyllithium/Methyljodid in 1,2-Dimethoxyäthan bei Raumtemperatur erhalten. Smp. 190-192 °.

Beispiel 85

500 mg p-[(p-Amidinobenzoyl)alanyl]phenoxyessigsäure-methylester-hydrochlorid (Beispiel 84) wurden in 10 ml 2N Salzsäure 1 Stunde bei 0-5 ° gerührt. Die ausgefallenen Kristalle wurden abgenutscht und im Vakuum getrocknet. Man erhielt 250 mg reines kristallines [p-[N-(p-Amidinobenzoyl)-(R,S)-alanyl]phenoxy]-essigsäure-hydrochlorid, Smp. 146-151 °.

Beispiel 86

Gemäss Beispiel 11 wurden aus 502 mg p-[2-[p-(Cyano-N-(p-carbomethoxybenzyl)benzamido]-1-hydroxyäthyl]phenoxy-essigsäure-methylester 390 mg amorphes p-[2-[p-Amidino-N-(p-carbomethoxybenzyl)-benzamido]-1-hydroxyäthyl]phenoxy-essigsäure-methylester-hydrochlorid erhalten. IR-Banden (KBr) bei 2922, 2853, 1744, 1718, 1680, 1608, 1511, 1461, 1281, 1110, 1016, 857 $cm^{-1}$.

Das Ausgangsmaterial wurde durch Reaktion von p-[2-[N-(p-Carbomethoxybenzyl)amino]-1-hydroxy-äthyl]phenoxyessig säure-methylester (Smp. 97-100 °, erhalten durch Reaktion von 4-Formylbenzoesäuremethylester mit 4-(2-Amino-1-hydroxyäthyl)phenoxyessigsäure-methylester und Natriumcyanoborhydrid) mit p-Cyanobenzoylchlorid in Pyridin erhalten. Amorph, IR-Banden (KBr) bei 3429, 2953, 2230, 1759, 1720, 1612, 1510, 1436, 1285, 1110, 1017, 850, 758 $cm^{-1}$.

Beispiel 87

Gemäss Beispiel 12 wurden aus 190 mg p-[2-[p-(Amidino-N-(p-carbomethoxybenzyl)benzamido]-1-hydroxyäthyl]phenoxy-essigsäure-methylester-hydrochlorid (Beispiel 86) 110 mg farblose, amorphe p-[2-[p-(Amidino-N-(p-carboxybenzyl)benzamido]-1-hydroxyäthyl]phenoxyessigsäure erhalten. IR-Banden (KBr) bei 3387, 2933, 1685, 1610, 1510, 1412, 1222, 1177, 1064, 1016 $cm^{-1}$.

Beispiel 88

Gemäss Beispiel 11 wurden aus 800 mg p-[[p-Cyano-N-(p-carbomethoxybenzyl)benzamido]acetyl]-phenoxyessigsäure-methylester-hydrochlorid 540 mg reines, amorphes p-[[p-Amidino-N-(p-carbomethoxybenzyl)benzamido]acetyl]phenoxyessigsäure-methylester-hydrochlorid erhalten. IR-Banden (KBr) bei 3377, 2952, 1757, 1722, 1681, 1636, 1599, 1536, 1438, 1285, 1171, 835 $cm^{-1}$.

Das Ausgangsmaterial wurde durch Reaktion von p-[2-[p-(Cyano-N-(p-carbomethoxybenzyl)benzamido]-1-hydroxyäthyl]phenoxyessigsäure-methylester (siehe Beispiel 86) mit Mangandioxid in Chloroform erhalten. Amorph, IR-Banden (KBr)bei 2954, 2230, 1760, 1720, 1689, 1642, 1601, 1509, 1436, 1356, 1110, 844, 757 $cm^{-1}$.

Beispiel 89

Gemäss Beispiel 12 wurden aus 310 mg p-[[p-Amidino-N-(p-carbomethoxybenzyl)benzamido]acetyl]-phenoxyessigsäure-methylester-hydrochlorid (Beispiel 88) 238 mg reine, amorphe p-[[p-Amidino-N-(p-carboxybenzyl)benzamido]acetyl]phenoxyessigsäure erhalten. IR-Banden (KBr) bei 2924, 2853, 1685, 1606, 1464, 1378, 1230, 1174 $cm^{-1}$.

Beispiel 90

Gemäss Beispiel 11 wurden aus 500 mg [p-[(RS)-2-(p-Cyanobenzamido)-1-methoxyäthyl]phenoxy]-essigsäure-methylester 330 mg amorphes [p-[(RS)-2-(p-Amidinobenzamido)-1-methoxyäthyl]phenoxy]-essigsäure-hydrochlorid erhalten. IR-Banden (KBr) bei 3252, 3052, 1756, 1681, 1643, 1545, 1485, 1211, 1078, 714 $cm^{-1}$.

Das Ausgangsmaterial wurde durch Reaktion von p-[2-(p-Cyanobenzamido)-1-hydroxyäthyl]-phenoxyessigsäure-methylester (Beispiel 21) mit Butyllithium/Methyljodid in 1,2-Dimethoxyäthan erhalten, IR-Banden (KBr) bei 3350, 2933, 2230, 1759, 1655, 1610, 1511, 1176, 1081, 834 $cm^{-1}$.

Beispiel 91

Gemäss Beispiel 12 wurden aus 63 mg [p-[(RS)-2-(p-Amidinobenzamido)-1-methoxyäthyl]phenoxy]-essigsäure-methylester-hydrochlorid (Beispiel 90) 33 mg [p-[(RS)-2-(p-Amidinobenzamido)-1-methoxyäthyl]-phenoxy]essigsäure erhalten, IR-Banden (KBr) bei 3270, 2932, 1674, 1609, 1511, 1482, 1424, 1336, 1235, 1105, 721 $cm^{-1}$.

Beispiel 92

Gemäss Beispiel 85 wurden aus 100 mg [p-[(all-RS)-2-(p-Amidinobenzamido)-1-(methyldithio)propyl]-phenoxy]essigsäuremethylester-hydrochlorid (Beispiel 84b) 90 mg [p-[(all-RS)-2-(p-Amidinobenzamido)-1-(methyldithio)propyl]phenoxy]essigsäure-hydrochlorid erhalten, IR-Banden (KBr) bei 3042, 2926, 1679, 1638, 1541, 1508, 1482, 1210, 1177, 1072, 858 $cm^{-1}$.

Beispiel 93

In Analogie zu Beispiel 11 wurde aus [p-[(RS)-2-(p-Cyanobenzamido)-1-(2′-dimethylaminoäthoxy)äthyl]-phenoxy]essigsäure-methylester [p-[(RS)-2-(p-Amidinobenzamido)-1-(2′-dimethylaminoäthoxy)äthyl]-phenoxy]essigsäure-methylester-hydrochlorid erhalten.

Das Ausgangsmaterial wird durch Reaktion von p-[2-(p-Cyanobenzamido)-1-hydroxyäthyl]-phenoxyessigsäure-methylester (Beispiel 21) mit Butyllithium/Methyljodid in 1,2-Dimethoxyäthan hergestellt.

Beispiel 94

In zu den vorangehenden Beispielen analoger Weise wurden folgende Verbindungen hergestellt:

a) [p-[(S)-2-(p-Amidinobenzamido)propyl]phenoxy]essigsäure,
b) p-(p-Amidino-N-[(RS)-p-(carboxymethoxy)-β-hydroxyphenäthyl]benzamido]-p-toluylsäure,
c) rac-p-(2-(p-Amidinobenzamido)-1-oxopropyl]phenoxyessigsäure-methylester,
d) rac-p-(2-(p-Amidinobenzamido)-1-oxopropyl]phenoxyessigsäure,
e) [p-[(p-Amidino-N-methylbenzamido)acetyl]phenoxy]essigsäure,
f) [p-[(E)-2-[(p-Amidinophenyl)carbamoyl]vinyl]phenoxy]essigsäure-methylester.

Beispiel 95

In Analogie zu Beispiel 11 wurden aus 114 mg p-[2-(p-Cyanophenyl)-2-oxazolidin-5-yl]-phenoxyessigsäure-methylester 38 mg p-[(E)-2-[p-Amidinobenzamido]vinyl]phenoxyessigsäure-methylester-hydrochlorid erhalten. IR-Banden (KBr) bei 3424, 1747, 1609, 1508, 1484, 1437, 1216, 1177, 1080 $cm^{-1}$.

Das Ausgangsmaterial wurde durch Behandeln von p-[(RS)-1-Hydroxy-2-(p-cyanobenzamido)äthyl]-phenoxyessigsäure-methylester (Beispiel 21) mit saurem Aluminiumoxid in Xylol bei Siedetemperatur erhalten.

Beispiel 96

In Analogie zu Beispiel 11 wurden aus 1,59 g p-[(RS)-1-Acetoxy-2-(p-cyanobenzamido)äthyl]-phenoxyessigsäure-methylester 880 mg p-[(RS)-1-Acetoxy-2-(p-amidinobenzamido)äthyl]-phenoxyessigsäure-methylester-hydrochlorid erhalten. IR-Banden (KBr) bei 3374, 3084, 1739, 1680, 1646, 1545, 1512, 1485, 1233, 1078, 711 $cm^{-1}$.

Das Ausgangsmaterial wurde durch Reaktion von p-[(RS)-1-Hydroxy-2-(p-cyanobenzamido)äthyl]-phenoxyessigsäure-methylester (Beispiel 21) mit Acetanhydrid/Pyridin erhalten. IR-Banden (KBr) bei 3360, 2954, 2230, 1740, 1661, 1612, 1540, 1513, 1373, 1081, 1032, 833 $cm^{-1}$.

Beispiel 97

In Analogie zu Beispiel 11 wurden aus 1,30 g p-[2-[(p-Cyanobenzoyl)methylamino]äthyl]-phenoxyessigsäure-methylester 860 mg p-[2-[(p-Amidinobenzoyl)methylamino]äthyl]phenoxy-essigsäure-methylester-hydrochlorid erhalten, IR-Banden (KBr) bei 3031, 1756, 1680, 1612, 1511, 1408, 1208, 1077, 858 $cm^{-1}$.

Das Ausgangsmaterial wurde durch Reaktion von p-[2-[(p-Cyanobenzoyl)methylamino]äthyl]phenol mit Bromessigsäure-methylester in Aceton in Gegenwart von Kaliumcarbonat erhalten. Smp. 113-115°.

p-[2-[(p-Cyanobenzoyl)methylamino]äthyl]phenol wurde durch Reaktion von N-Methyltyramin mit p-Cyanobenzoesäurechlorid in Pyridin erhalten, Smp. 151-152°.

Beispiel 98

In Analogie zu Beispiel 12 wurden aus 640 mg p-[2-[(p-Amidinobenzoyl)methylamino]äthyl]-phenoxyessigsäure-methylester-hydrochlorid 580 mg p-[2-[(p-Amidinobenzoyl)methylamino]äthyl]-phenoxyessigsäure-hydrochlorid erhalten. IR-Banden (KBr) bei 3382, 3042, 1680, 1609, 1511, 1405, 1210, 1074 $cm^{-1}$.

Beispiel 99

In Analogie zu Beispiel 11 wurde aus 480 mg p-[(6-Cyanonicotinamido)acetyl]phenoxyessigsäure-methylester 166 mg p-[(6-Amidinonicotinamido)acetyl]phenoxyessigsäure-methylester-hydrochlorid erhalten.

Das Ausgangsmaterial wurde durch Reaktion von p-[(RS)-2-(6-Cyanonicotinamido)-1-hydroxyäthyl]-phenoxyessigsäure-methylester mit Mangandioxid in Chloroform erhalten, welches seinerseits durch Reaktion von 4-(2-Amino-1-hydroxyäthyl)phenoxyessigsäure-methylester-hydrochlorid (Smp. 123-125°C) mit 6-Cyanonicotinsäure und Chlorameisensäureester/4-Aethylmorpholin entstand (Smp. 131-132°C).

Beispiel 100

Analog Beispiel 85 wurden aus 125 mg p-[(6-Amidinonicotinamido)acetyl]phenoxyessigsäure-methylester-hydrochlorid 115 mg p-[(6-Amidinonicotinamido)acetyl]phenoxyessigsäure-hydrochlorid erhalten. IR-Banden (KBr) bei 3337, 3249, 3069, 1692, 1637, 1599, 1535, 1422, 1178, 1059, 832 $cm^{-1}$.

Beispiel 101

Analog Beispiel 100 werden hergestellt:

a) p-[(5-Amidinopicolinamido)acetyl]phenoxyessigsäure-methylester-hydrochlorid, IR-Banden (KBr) bei 3380, 3265, 2954, 1760, 1657, 1598, 1519, 1221, 1074, 987 $cm^{-1}$

b) p-[(5-Amidinopicolinamido)acetyl]phenoxyessigsäure-hydrochlorid. IR-Banden (KBr) bei 3598, 3351, 3073, 2908, 1683, 1660, 1598, 1524, 1360, 1178, 1072, 992 $cm^{-1}$.

Beispiel 102

Analog zu Beispiel 11 wurden aus 550 mg [[α-[(p-Cyanobenzamido)oxy]-p-tolyl]oxy]essigsäure-methylester 200 mg [[α-[(p-Amidinobenzamido)oxy]-p-tolyl]oxy]essigsäure-methylester-hydrochlorid erhalten. IR-Banden (KBr) bei 3128, 1738, 1668, 1511, 1477, 1431, 1231, 1077, 714 $cm^{-1}$.

Das Ausgangsmaterial wurde durch Reaktion von 4-Aminoxymethyl-phenoxyessigsäure-methylester mit p-Cyanobenzoylchlorid in Pyridin erhalten. Smp. 173-175°C.

Beispiel 103

Analog zu Beispiel 11 wurden aus 550 mg Methyl-rac-p-[2-(p-Cyano-N-methyl-benzamido)-1-oxopropyl]-phenoxyacetat 220 mg Methyl-rac-p-[2-(p-amidino-N-Methyl-benzamido)-1-oxo-propyl]pheneoxyacetat hydrochlorid erhalten, amorph, IR-Banden (Kbr) bei 2952, 1767, 1686, 1603, 1214, 1174, 1081, 847 $cm^{1-}$.
Das Ausgangsmaterial wurde durch Reaktion von rac-4-Hydroxy-2-methylamino-propiophenon mit p-Cyanobenzosäurechlorid in Methylenchlorid/Triäthylamin zu rac-p-[2-(p-Cyano-N-methyl-benzamido)-1-oxopropyl]-phenol (Smp. 176-178°) und weitere Umsetzung mit Bromessigsäuremethylester/Kaliumcarbonatzu rac-p-[2-(p-Cyano-N-methylbenzamido)-1-oxopropyl]phenoxyessigsäure-methylester vom Smp. 162-163° hergestellt.

Beispiel A

Eine Verbindung der Formel I kann man in an sich bekannter Weise als Wirkstoff zur Herstellung von Tabletten folgender Zusammensetzung verwenden:

| | pro Tablette |
|---|---|
| Wirkstoff | 200 mg |
| mikrokristalline Cellulose | 155 mg |
| Maisstärke | 25 mg |
| Talk | 25 mg |
| Hydroxypropylmethylcellulose | 20 mg |
| | 425 mg |

Beispiel B

Eine Verbindung der Formel I kann man in an sich bekannter Weise als Wirkstoff zur Herstellung von Kapseln folgender Zusammensetzung verwenden:

| | pro Kapsel |
|---|---|
| Wirkstoff | 100,0 mg |
| Maisstärke | 20,0 mg |
| Milchzucker | 95,0 mg |
| Talk | 4,5 mg |
| Magnesiumstearat | 0,5 mg |
| | 220,0 mg |

**Patentansprüche**
**Patentansprüche für folgende Vertragsstaaten : AT, BE, CH, DE, DK, FR, GB, IT, LI, LU, NL, SE**

**1.** Verbindungen der allgemeinen Formel

$R^1$-A-$(W)_a$-X-$(CH_2)_b$-$(Y)_c$-B-Z-COOR (I)

worin
A einen Rest

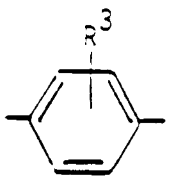 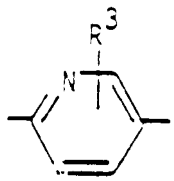 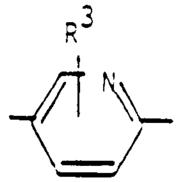 oder 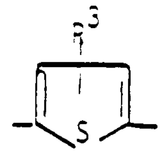 ;

B einen Rest

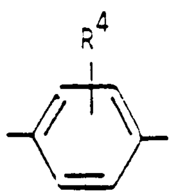 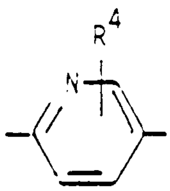 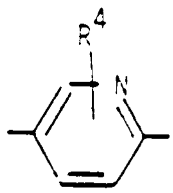 oder 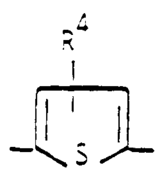 ;

W $-CH_2-$, $-CH_2CH_2-$, $-CH=CH-$, $-CH=CH-CH_2-$, $-(CH_2)_3-$, $-CH_2CH(CH_3)-$, $-COCH_2-$, $-CH(OH)CH_2-$ oder $-CH_2COCH_2-$;

X $-CONR^2-$, $-NR^2CO-$, $-SO_2NR^2-$ oder $-NR^2SO_2-$;

Y $-CH_2CH_2-$, $-CH_2CH_2O-$, $-OCH_2-$, $CH(CH_3)CH_2-$, $-CH=CH-$, $-CH_2-CH=CH-$, $-C(Q^1,Q^2)-CO(CH_2)_d-$, $-CH_2-$, $-CH_2CH_2CH_2-$, $-CH(CH_3)CH_2CH_2-$, $-CH_2COCH_2-$, $-C(Q^1,Q^2)-CH(OH)-$, $-C(Q^1,Q^2)-CH(SSCH_3)-$, $-CH(CH_2OH)CH_2-$ oder $-CH(COOR)CH_2-$ bedeutet, wobei Carbonylgruppen auch als Oxim, Oximäther, Ketal oder Thioketal oder Enoläther und Hydroxygruppen als nieder-Alkyläther, Di(niederalkyl)amino-niederalkyläther oder als Ester von niederen Alkancarbonsäuren vorliegen können

Z $-OCH_2-$, $-NR^6CH_2-$, $-CH_2CH_2-$, $-CH(CH_3)CH_2-$, $-CH_2-$, $-CH=CH-$ oder $-C(CH_3)=CH-$;

R Wasserstoff, nieder-Alkyl, Phenyl oder Phenyl-nieder-Alkyl;

$Q^1$ und $Q^2$ Wasserstoff oder nieder-Alkyl sind oder zusammen mit dem C-Atom, an dem sie gebunden sind, einen 3- bis 6-gliedrigen gesättigten Ring bilden;

$R^1$ Amidino oder Guanidino;

$R^2$ Wasserstoff, nieder-Alkyl, Phenyl-nieder-alkyl, im Phenylteil durch Amino, Amidino oder -COOR substituiertes Phenyl-nieder-Alkyl, oder einen Rest $-CH_2COOR$ oder -Y-B-Z-COOR;

$R^3$ Wasserstoff, nieder-Alkyl, nieder-Alkoxy, Halogen, nieder-Carbalkoxy, Amino, nieder-Alkylamino, di-nieder-Alkylamino oder Amidino;

$R^4$ Wasserstoff, nieder-Alkyl, nieder-Alkoxy, Halogen, nieder-Carbalkoxy, Amino, nieder-Alkylamino, di-nieder-Alkyl-amino, oder einen Rest -Z-COOR oder $-CH=CH-(CH_2)-_nCOOR$;

$R^6$ Wasserstoff, nieder-Alkyl oder Benzyl;

n eine ganze Zahl von 0-4;

a, c und d unabhängig voneinander 0 oder 1; und

b eine ganze Zahl von 0-2 bedeuten, wobei jedoch a und b 0 sein sollen, wenn c 1 ist. und c 0 sein soll, wenn a oder b von 0 verschieden ist;

und physiologisch verträgliche Salze davon.

**2.** Verbindungen nach Anspruch 1, worin W und X die in Anspruch 1 angegebene Bedeutung haben und Y $-CH_2CH_2-$, $-CH_2CH_2O-$, $-CH(CH_3)CH_2-$, $-CH=CH-$, $-CH_2-CH=CH-$, $-CH_2CO-$, $-CH_2-$, $-CH_2CH_2CH_2-$, $-CH(CH_3)CH_2CH_2-$, $-CH_2COCH_2-$, $-CH_2CHOH-$, $-CH(CH_2OH)CH_2-$ oder $-CH(COOR)CH_2-$ bedeutet, wobei Carbonylgruppen auch als Oxim, Ketal oder Thioketal oder Enoläther und Hydroxygruppen als nieder-Alkyläther oder als Ester von niederen Alkancarbonsäuren vorliegen können.

**3.** Verbindungen gemäss Anspruch 1 oder 2 der Formel

$R^1$-A-X-Y-B-Z-COOR (Ia)

**4.** Verbindungen gemäss Anspruch 1 oder 2 der Formel

$R^1$-A-W-X-$(CH_2)_b$-B-Z-COOR (Ib)

**5.** Verbindungen gemäss den Ansprüchen 1-4, in denen die Summe der in $(W)_a$, $(CH_2)_b$, $(Y)_c$, X und Z in gerader Kette vorliegenden C-, O-, N- und S-Atome 6 ist.

**6.** Verbindungen gemäss den Ansprüchen 1-5, in denen $R^1$ Amidino ist.

**7.** Verbindungen gemäss den Ansprüchen 1-6, in denen Y $-CH(CH_3)CO-$, $-CH_2CH_2$ oder $-CH_2CO-$; Z $-OCH_2-$ oder $-CH_2CH_2-$ und X -NHCO- oder -CONH- ist.

**8.** Verbindungen gemäss den Ansprüchen 1-7, in denen R Wasserstoff ist.

**9.** Verbindungen nach Anspruch 1, 2 oder 3 aus der Gruppe der folgenden:
p-[2-(p-Amidinobenzamido)äthyl]phenoxyessigsäuremethylester,
[p-[2-(p-Amidinobenzamido)äthyl]phenoxy]essigsäure,
Methyl [4-[2-(p-amidinobenzamido)äthyl]-2-jodphenoxy]acetat,
[4-[2-(p-Amidinobenzamido)äthyl]-2-jodphenoxy]essigsäure,
(E)-5-[5-[2-(p-Amidinobenzamido)äthyl]-2-(carbomethoxymethoxy)phenyl]-4-pentensäurebenzylester,
(E)-5-[5-[2-(p-Amidinobenzamido)äthyl]-2-(carboxymethoxy)phenyl]-4-pentensäure,
p-[2-(p-Amidinobenzamido)äthoxyphenyl]essigsäuremethylester,
p-[2-(p-Amidinobenzamido)äthoxy]phenylessigsäure,
p-[2-(p-Amidinophenylsulfonamido)äthoxy]phenylessigsäuremethylester,
p-[2-(p-Amidinophenylsulfonamido)äthoxy]phenylessigsäure,
p-[2-(p-Amidinobenzolsulfonamido)äthyl]phenoxyessigsäuremethylester,
p-[2-(p-Amidinobenzolsulfonamido)äthyl]phenoxyessigsäure,
p-[(S)-2-(p-Amidinobenzolsulfonamido)propyl]phenoxyessigsäure-methylester-acetat,
p-[(S)-2-(p-Amidinobenzolsulfonamido)propyl]phenoxyessigsäure,
p-[2-(p-Amidino-N-methylphenylsulfonamido)äthyl]phenoxyessigsäure-methylester,
p-[2-(p-Amidino-N-methylphenylsulfonamido)äthyl]phenoxyessigsäure,
p-[2-(p-Amidino-N-benzylphenylsulfonamido)äthyl]phenoxyessigsäure-methylester,
p-[2-(p-Amidino-N-benzylphenylsulfonamido)äthyl]phenoxyessigsäure,
p-[2-(p-Amidinobenzolsulfonamido)-1-hydroxyäthyl]phenoxyessigsäure-methylester,
p-[2-(p-Amidinobenzolsulfonamido)-1-hydroxyäthyl]phenoxyessigsäure,
p-[2-(p-Amidinobenzamido)-1-hydroxyäthyl]phenoxyessigsäure-methylester,
p-[2-(p-Amidinobenzamido)-1-hydroxyäthyl]phenoxyessigsäure,
p-[(p-Amidinophenylsulfonamido)acetyl]phenoxyessigsäuremethylester,
p-[(p-Amidinophenylsulfonamido)acetyl]phenoxyessigsäure,
p-[(p-Amidinobenzamido)acetyl]phenoxyessigsäure-methylester,
p-[(p-Amidinobenzamido)acetyl]phenoxyessigsäure,
p-[2-[p-Amidino-N-(p-carbomethoxybenzyl)phenylsulfonamido]äthyl]phenoxyessigsäure-methylester,
p-[2-[p-Amidino-N-(p-carboxybenzyl)phenylsulfonamido]äthyl]phenoxyessigsäure,
[[4-[2-(p-Amidinophenylsulfonamido)äthyl]-o-phenylen]dioxy]diessigsäure-dimethylester,
[[4-[2-(p-Amidinophenylsulfonamido)äthyl]-o-phenylen]dioxy]diessigsäure,
[4-[2-(p-Amidinophenylsulfonamido)äthyl]-2-methoxyphenoxy]essigsäure-methylester,
[4-[2-(p-Amidinophenylsulfonamido)äthyl]-2-methoxyphenoxy]essigsäure,
(E)-p-[2-(p-Amidinophenylsulfonamido)äthyl]-$\beta$-methylzimtsäure-methylester,
(E)-p-[2-(p-Amidinophenylsulfonamido)äthyl]-$\beta$-methylzimtsäure,
p-[2-(p-Amidinophenylsulfonamido)äthyl]-$\beta$-methyl-hydrozimtsäure-methylester,
p-[2-(p-Amidinophenylsulfonamido)äthyl]-$\beta$-methyl-hydrozimtsäure,
(E)-5-[(RS)-2-(p-Amidinobenzamido)propyl]-$\beta$-methyl-2-thiophen-acrylsäure-äthylester,
(E)-5-[(RS)-2-(p-Amidinobenzamido)propyl]-$\beta$-methyl-2-thiophen-acrylsäure,
(RS)-5-[(RS)-2-(p-Amidinobenzamido)propyl]-$\beta$-methyl-2-thiophen-propionsäure-äthylester,
(RS)-5-[(RS)-2-(p-Amidinobenzamido)propyl]-$\beta$-methyl-2-thiophen-propionsäure,
p-[2-(p-Amidinobenzamido)äthyl]-hydrozimtsäure-methylester,

p-[2-(p-Amidinobenzamido)äthyl]-hydrozimtsäure,
p-[3-(p-Amidinobenzamido)propyl]phenylessigsäure-methylester,
p-[3-(p-Amidinobenzamido)propyl]phenylessigsäure,
p-[3-(p-Amidinophenylsulfonamido)propyl]phenylessigsäure-methylester,
p-[3-(p-Amidinophenylsulfonamido)propyl]phenylessigsäure,
p-[2-(4-Amidino-3-pyridylamido)äthyl]phenoxyessigsäure-methylester,
p-[2-(4-Amidino-3-pyridylamido)äthyl]phenoxyessigsäure,
p,p′-[[(p-Amidinobenzoyl)imino]diäthylen]-dihydrozimtsäure-dimethylester,
p,p′-[[(p-Amidinobenzoyl)imino]diäthylen]-dihydrozimtsäure,
p-[2-[(p-Amidinophenyl)carbamoyl]äthyl]phenoxyessigsäure-methylester,
p-[2-[(p-Amidinophenyl)carbamoyl]äthyl]phenoxyessigsäure,
p-[2-(p-Amidinobenzamido)-1-hydroxy-äthyl]hydrozimtsäure-methylester,
p-[2-(p-Amidinobenzamido)-1-hydroxyäthyl]hydrozimtsäure,
p-[(p-Amidinobenzamido)acetyl]hydrozimtsäure-methylester,
p-[(p-Amidinobenzamido)acetyl]hydrozimtsäure,
p-[2-(p-Guanidinobenzamido)äthyl]hydrozimtsäure-methylester,
p-[2-(p-Guanidinobenzamido)äthyl]hydrozimtsäure,
[α-(p-Guanidinobenzamido)-p-tolyl]oxy-essigsäure-methylester,
[α-(p-Guanidinobenzamido)-p-tolyl]oxy-essigsäure,
p-[(p-Guanidinobenzamido)methyl]hydrozimtsäure-methylester,
p-[(p-Guanidinobenzamido)methyl]hydrozimtsäure,
N-(p-Amidinobenzoyl)-3-[p-(tert-butoxycarbonyl)methoxy)phenyl]-D-alanin-methylester,
[p-[(R)-2-(p-Amidinobenzamido)-2-(methoxycarbonyl)äthyl]phenoxy]essigsäure,
N-(p-Amidinobenzoyl)-3-[p-(carboxymethoxy)phenyl]-D-alanin,
(E)-p-[2-(p-Amidinobenzamido)äthyl]-β-methyl-zimtsäuremethylester,
rac-p-[2-(p-Amidinobenzamido)äthyl]-β-methylhydrozimtsäuremethylester,
p-[2-(p-Guanidinobenzamido)äthyl]phenoxyessigsäuremethylester,
N-Benzyl-N-[p-[2-(p-amidinobenzamido)äthyl]phenyl]glycinmethylester,
[p-[2-(p-Guanidinobenzamido)äthyl]phenoxy]essigsäure,
p-[(R)-2-(p-Amidinobenzamido)-3-hydroxypropyl]phenoxyessigsäure-t-butylester,
p-[(R)-2-(p-Amidinobenzamido)-3-hydroxypropyl]phenoxyessigsäure,
[[4-[p-Amidino-N-methylbenzamido)acetyl]-o-phenylen]dioxy]diessigsäuredimethylester,
[4-(p-Amidino-N-methylbenzamido)acetyl-o-phenylen]dioxy]diessigsäure,
p-[N-(p-Amidinobenzoyl)-(RS)-alanyl]phenoxyessigsäure-methylester,
[p-[N-(p-Amidinobenzoyl)-(R,S)-alanyl]phenoxy]essigsäure,
p-[2-[p-Amidino-N-(p-carbomethoxybenzyl)benzamido]-1-hydroxyäthyl]phenoxyessigsäure-methylester,
p-[2-[p-(Amidino-N-(p-carboxybenzyl)benzamido]-1-hydroxyäthyl]phenoxyessigsäure,
p-[[p-Amidino-N-(p-carbomethoxybenzyl)benzamido]acetyl]phenoxyessigsäure-methylester und
p-[[p-Amidino-N-(p-carboxybenzyl)benzamido]acetyl]phenoxyessigsäure.

**10.** Verbindungen nach Anspruch 1, 2 oder 4 aus der Gruppe der folgenden:
α-[(p-Amidinobenzyl)carbamoyl]-p-tolyloxyessigsäure-methylester,
α-[(p-Amidinobenzyl)carbamoyl]-p-tolyloxyessigsäure,
p-[(p-Amidinophenäthyl)carbamoyl]phenoxyessigsäure-methylester,
p-[(p-Amidinophenäthyl)carbamoyl]phenoxyessigsäure,
[[α-(p-Amidinohydrocinnamamido)-p-tolyl]oxy]essigsäure,
[p-(p-Amidinohydrocinnamamido)phenoxy]essigsäure,
[p-[(E)-p-Amidinocinnamamido]phenoxy]essigsäure,
[[α-[2-(p-Amidinophenyl)acetamido]-p-tolyl]oxy]essigsäuremethylester und
[[p-[2-(p-Amidinophenyl)acetamido]-p-tolyl]oxy]essigsäure.

**11.** Die Verbindungen gemäss den Ansprüchen 1-10 zur Verwendung als Heilmittel.

**12.** Die Verbindungen gemäss den Ansprüchen 1-10 zur Verwendung als Wirkstoffe bei der Herstellung von Heilmitteln zur Behandlung von Thrombosen, Apoplexie, Herzinfarkt, Entzündungen, Arteriosklerose sowie von Tumoren.

**13.** Pharmazeutische Präparate, enthaltend eine Verbindung gemäss den Ansprüchen 1-10 und übliche pharmazeutische Trägerstoffe.

**14.** Verfahren zur Herstellung von Verbindungen der allgemeinen Formel I von Anspruch 1 und deren Salzen, dadurch gekennzeichnet, dass man

a) in einer Verbindung der allgemeinen Formel

$NC\text{-}A\text{-}(W)_a\text{-}X\text{-}(CH_2)_b\text{-}(Y)_c\text{-}B\text{-}Z\text{-}COOR^5$ (II)

worin $R^5$ nieder-Alkyl oder Benzyl ist und A, B, W, X, Y, Z, a, b und c oben angegebene Bedeutung haben und worin im Molekül gegebenenfalls vorhandene Carboxygruppen als Ester vorliegen,

die Nitrilgruppe in die Amidinogruppe überführt, oder

b) in einer Verbindung der allgemeinen Formel

$R^{11}\text{-}A\text{-}(W)_a\text{-}X\text{-}(CH_2)_b\text{-}(Y)_c\text{-}B\text{-}Z\text{-}COOR^5$ (III)

worin A, B, W, X, Y, Z, $R^5$, a, b und c die oben angegebene Bedeutung haben und $R^{11}$ eine geschützte Amidino- oder Guanidinogruppe darstellt,

die Schutzgruppen der Amidino- oder Guanidinogruppe abspaltet, oder

c) in einer Verbindung der allgemeinen Formel

$H_2N\text{-}A\text{-}(W)_a\text{-}X\text{-}(CH_2)_b\text{-}(Y)_c\text{-}B\text{-}Z\text{-}COOR^5$ (IV)

worin A, B, W, X, Y, Z, $R^5$, a, b und c die oben angegebene Bedeutung haben, und worin im Molekül gegebenenfalls vorhandene Carboxygruppen als Ester vorliegen,

die Aminogruppe in die Guanidinogruppe überführt, oder

d) eine Verbindung der allgemeinen Formel

$R^1\text{-}A\text{-}(W)_a\text{-}COE$ (V)

worin E eine aktivierte Estergruppe oder Chlor oder Brom darstellt und $R^1$, A, W und a die oben angegebene Bedeutung haben,

mit einer Verbindung der allgemeinen Formel

$HNR^2\text{-}(CH_2)_b\text{-}(Y)_c\text{-}B\text{-}Z\text{-}COOR^5$ (VI)

worin Y, B, Z, $R^5$, $R^2$, b und c die oben angegebene Bedeutung haben,

umsetzt, eine gegebenenfalls anwesende Benzylgruppe $R^5$ abspaltet

und gewünschtenfalls in einer so erhaltenen Verbindung der allgemeinen Formel I anwesende, funktionelle Gruppen abwandelt.

**Patentansprüche für folgende Vertragsstaaten : ES, GR**

**1.** Verfahren zur Herstellung von Verbindungen der allgemeinen Formel

$R^1\text{-}A\text{-}(W)_a\text{-}X\text{-}(CH_2)_b\text{-}(Y)_c\text{-}B\text{-}Z\text{-}COOR$ (I)

worin

A einen Rest

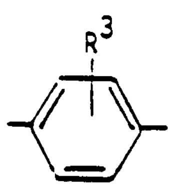

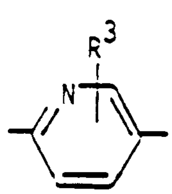

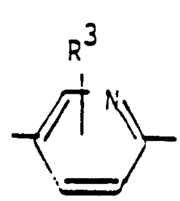

oder

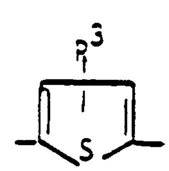

;

B einen Rest

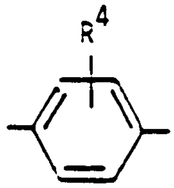 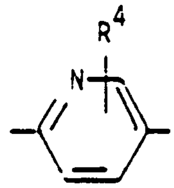 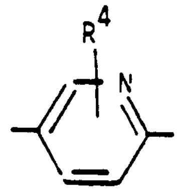 oder 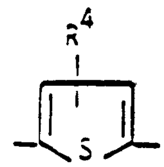 ;

W $-CH_2-$, $-CH_2CH_2-$, $-CH=CH-$, $-CH=CH-CH_2-$, $-(CH_2)_3-$, $-CH_2CH(CH_3)-$, $-COCH_2-$, $-CH(OH)CH_2-$ oder $-CH_2COCH_2-$;

X $-CONR^2-$, $-NR^2CO-$, $-SO_2NR^2-$ oder $-NR^2SO_2-$;

Y $-CH_2CH_2-$, $-CH_2CH_2O-$, $-OCH_2-$, $CH(CH_3)CH_2-$, $-CH=CH-$, $-CH_2-CH=CH-$, $C(Q^1,Q^2)-CO(CH_2)_d-$, $-CH_2-$, $-CH_2CH_2CH_2-$, $-CH(CH_3)CH_2CH_2-$, $-CH_2COCH_2-$, $-C(Q^1,Q^2)-CH(OH)-$, $-C(Q^1,Q^2)-CH(SSCH_3)-$, $-CH(CH_2OH)CH_2-$ oder $-CH(COOR)CH_2-$ bedeutet, wobei Carbonylgruppen auch als Oxim, Oximäther, Ketal oder Thioketal oder Enoläther und Hydroxygruppen als nieder-Alkyläther, Di(niederalkyl)amino-niederalkyläther oder als Ester von niederen Alkancarbonsäuren vorliegen können

Z $-OCH_2-$, $-NR^6CH_2-$, $-CH_2CH_2-$, $-CH(CH_3)CH_2-$, $-CH_2-$, $-CH=CH-$ oder $-C(CH_3)=CH-$;

R Wasserstoff, nieder-Alkyl, Phenyl oder Phenyl-nieder-Alkyl;

$Q^1$ und $Q^2$ Wasserstoff oder nieder-Alkyl sind oder zusammen mit dem C-Atom, an dem sie gebunden sind, einen 3- bis 6-gliedrigen gesättigten Ring bilden;

$R^1$ Amidino oder Guanidino;

$R^2$ Wasserstoff, nieder-Alkyl, Phenyl-nieder-alkyl, im Phenylteil durch Amino, Amidino oder -COOR substituiertes Phenyl-nieder-Alkyl, oder einen Rest $-CH_2COOR$ oder -Y-B-Z-COOR;

$R^3$ Wasserstoff, nieder-Alkyl, nieder-Alkoxy, Halogen, nieder-Carbalkoxy, Amino, nieder-Alkylamino, di-nieder-Alkylamino oder Amidino;

$R^4$ Wasserstoff, nieder-Alkyl, nieder-Alkoxy, Halogen, nieder-Carbalkoxy, Amino, nieder-Alkylamino, di-nieder-Alkyl-amino, oder einen Rest -Z-COOR oder $-CH=CH-(CH_2)_nCOOR$;

$R^6$ Wasserstoff, nieder-Alkyl oder Benzyl;

n eine ganze Zahl von 0-4;

a, c und d unabhängig voneinander 0 oder 1; und

b eine ganze Zahl von 0-2 bedeuten, wobei jedoch a und b 0 sein soll, wenn c 1 ist, und c 0 sein soll, wenn a oder b von 0 verschieden ist;

und von physiologisch verträglichen Salze davon, dadurch gekennzeichnet, dass man

a) in einer Verbindung der allgemeinen Formel

$NC-A-(W)_a-X-(CH_2)_b-(Y)_c-B-Z-COOR^5$ (II)

worin $R^5$ nieder-Alkyl oder Benzyl ist und A, B, W, X, Y, Z, a, b und c oben angegebene Bedeutung haben und worin im Molekül gegebenenfalls vorhandene Carboxygruppen als Ester vorliegen,

die Nitrilgruppe in die Amidinogruppe überführt, oder

b) in einer Verbindung der allgemeinen Formel

$R^{11}-A-(W)_a-X-(CH_2)_b-(Y)_c-B-Z-COOR^5$ (III)

worin A, B, W, X, Y, Z, $R^5$, a, b und c die oben angegebene Bedeutung haben und $R^{11}$ eine geschützte Amidino- oder Guanidinogruppe darstellt,

die Schutsgruppen der Amidino- oder Guanidinogruppe abspaltet, oder

c) in einer Verbindung der allgemeinen Formel

$H_2N-A-(W)_a-X-(CH_2)_b-(Y)_c-B-Z-COOR^5$ (IV)

worin A, B, W, X, Y, Z, $R^5$, a, b und c die oben angegebene Bedeutung haben, und worin im

Molekül gegebenenfalls vorhandene Carboxygruppen als Ester vorliegen, die Aminogruppe in die Guanidinogruppe überführt, oder
d) eine Verbindung der allgemeinen Formel

$R^1$-A-$(W)_a$-COE (V)

worin E eine aktivierte Estergruppe oder Chlor oder Brom darstellt und $R^1$, A, W und a die oben angegebene Bedeutung haben,
mit einer Verbindung der allgemeinen Formel

$HNR^2$-$(CH_2)_b$-$(Y)_c$-B-Z-$COOR^5$ (VI)

worin Y, B, Z, $R^5$, $R^2$, b und c die oben angegebene Bedeutung haben,
umsetzt, eine gegebenenfalls anwesende Benzylgruppe $R^5$ abspaltet
und gewünschtenfalls in einer so erhaltenen Verbindung der allgemeinen Formel I anwesende, funktionelle Gruppen abwandelt.

**2.** Verfahren nach Anspruch 1, worin W und X die in Anspruch 1 angegebene Bedeutung haben und Y $-CH_2CH_2-$, $-CH_2CH_2O-$, $-CH(CH_3)CH_2-$, $-CH=CH-$, $-CH_2-CH=CH-$, $-CH_2CO-$, $-CH_2-$, $-CH_2CH_2CH_2-$, $-CH(CH_3)CH_2CH_2-$, $-CH_2COCH_2-$, $-CH_2CHOH-$, $-CH(CH_2OH)CH_2-$ oder $-CH(COOR)CH_2-$ bedeutet, wobei Carbonylgruppen auch als Oxim, Ketal oder Thioketal oder Enoläther und Hydroxygruppen als nieder-Alkyläther oder als Ester von niederen Alkancarbonsäuren vorliegen können.

**3.** Verfahren gemäss Anspruch 1 oder 2, dadurch gekennzeichnet, dass Verbindungen der Formel

$R^1$-A-X-Y-B-Z-COOR (Ia)

hergestellt werden.

**4.** Verfahren gemäss Anspruch 1 oder 2, dadurch gekennzeichnet, dass Verbindungen der Formel

$R^1$-A-W-X-$(CH_2)_b$-B-Z-COOR (Ib)

hergestellt werden.

**5.** Verfahren gemäss den Ansprüchen 1-4, in dem die Summe der in $(W)_a$, $(CH_2)_b$, $(Y)_c$, X und Z in gerader Kette vorliegenden C-, O-, N- und S-Atome 6 ist.

**6.** Verfahren gemäss den Ansprüchen 1-5, in dem $R^1$ Amidino ist.

**7.** Verfahren gemäss den Ansprüchen 1-6. in dem Y $-CH(CH_3)CO-$, $-CH_2CH_2$ oder $-CH_2CO-$; Z $-OCH_2-$ oder $-CH_2CH_2-$ und X -NHCO- oder -CONH- ist.

**8.** Verfahren gemäss den Ansprüchen 1-7, in dem R Wasserstoff ist.

**9.** Verfahren nach Anspruch 1, 2 oder 3 zur Herstellung der Verbindungen aus der Gruppe der folgenden:
p-[2-(p-Amidinobenzamido)äthyl]phenoxyessigsäuremethylester,
[p-[2-(p-Amidinobenzamido)äthyl]phenoxy]essigsäure,
Methyl [4-[2-(P-amidinobenzamido)äthyl]-2-jodphenoxy]acetat,
[4-[2-(p-Amidinobenzamido)äthyl]-2-jodphenoxy]essigsäure,
(E)-5-[5-[2-(p-Amidinobenzamido)äthyl]-2-(carbomethoxymethoxy)phenyl]-4-pentensäurebenzylester,
(E)-5-[5-[2-(p-Amidinobenzamido)äthyl]-2-(carboxymethoxy)phenyl]-4-pentensäure,
p-[2-(p-Amidinobenzamido)äthoxyphenyl]essigsäuremethylester,
p-[2-(p-Amidinobenzamido)äthoxy]phenylessigsäure,
p-[2-(p-Amidinophenylsulfonamido)äthoxy]phenylessigsäuremethylester,
p-[2-(p-Amidinophenylsulfonamido)äthoxy]phenylessigsäure,
p-[2-(p-Amidinobenzolsulfonamido)äthyl]phenoxyessigsäuremethylester,

p-[2-(p-Amidinobenzolsulfonamido)äthyl]phenoxyessigsäure,
p-[(S)-2-(p-Amidinobenzolsulfonamido)propyl]phenoxyessigsäure-methylester-acetat,
p-[(S)-2-(p-Amidinobenzolsulfonamido)propyl]phenoxyessigsäure,
p-[2-(p-Amidino-N-methylphenylsulfonamido)äthyl]phenoxyessigsäure-methylester,
p-[2-(p-Amidino-N-methylphenylsulfonamido)äthyl]phenoxyessigsäure,
p-[2-(p-Amidino-N-benzylphenylsulfonamido)äthyl]phenoxyessigsäure-methylester,
p-[2-(p-Amidino-N-benzylphenylsulfonamido)äthyl]phenoxyessigsäure,
p-[2-(P-Amidinobenzolsulfonamido)-1-hydroxyäthyl]phenoxyessigsäure-methylester,
p-[2-(p-Amidinobenzolsulfonamido)-1-hydroxyäthyl]phenoxyessigsäure,
p-[2-(p-Amidinobenzamido)-1-hydroxyäthyl]phenoxyessigsäure-methylester,
p-[2-(p-Amidinobenzamido)-1-hydroxyäthyl]phenoxyessigsäure,
p-[(p-Amidinophenylsulfonamido)acetyl]phenoxyessigsäuremethylester,
p-[(p-Amidinophenylsulfonamido)acetyl]phenoxyessigsäure,
p-[(p-Amidinobenzamido)acetyl]phenoxyessigsäure-methylester,
p-[(p-Amidinobenzamido)acetyl]phenoxyessigsäure,
p-[2-[p-Amidino-N-(p-carbomethoxybenzyl)phenylsulfonamido]äthyl]phenoxyessigsäure-methylester,
p-[2-[p-Amidino-N-(p-carboxybenzyl)phenylsulfonamido]äthyl]phenoxyessigsäure,
[[4-[2-(p-Amidinophenylsulfonamido)äthyl]-o-henylen]dioxy]diessigsäure-dimethylester,
[[4-[2-(p-Amidinophenylsulfonamido)äthyl]-o-phenylen]dioxy]diessigsäure,
[4-[2-(p-Amidinophenylsulfonamido)äthyl]-2-methoxyphenoxy]essigsäure-methylester,
[4-[2-(p-Amidinophenylsulfonamido)äthyl]-2-methoxyphenoxy]essigsäure,
(E)-p-[2-(p-Amidinophenylsulfonamido)äthyl]-β-methylzimtsäure-methylester,
(E)-p-[2-(p-Amidinophenylsulfonamido)äthyl]-β-methylzimtsäure,
p-[2-(p-Amidinophenylsulfonamido)äthyl]-β-methyl-hydrozimtsäure-methylester,
p-[2-(p-Amidinophenylsulfonamido)äthyl]-β-methyl-hydrozimtsäure,
(E)-5-[(RS)-2-(p-Amidinobenzamido)propyl]-β-methyl-2-thiophen-acrylsäurea-äthylester,
(E)-5-[(RS)-2-(p-Amidinobenzamido)propyl]-β-methyl-2-thiophen-acrylsäure,
(RS)-5-[(RS)-2-(p-Amidinobenzamido)propyl]-β-methyl-2-thiophen-propionsäure-äthylester,
(RS)-5-[(RS)-2-(p-Amidinobenzamido)propyl]-β-methyl-2-thiophen-propionsäure,
p-[2-(p-Amidinobenzamido)äthyl]-hydrozimtsäure-methylester,
p-[2-(p-Amidinobenzamido)äthyl]-hydrozimtsäure,
p-[3-(p-Amidinobenzamido)propyl]phenylessigsäure-methylester,
p-[3-(p-Amidinobenzamido)propyl]phenylessigsäure,
p-[3-(p-Amidinophenylsulfonamido)propyl]phenylessigsäuremethylester,
p-[3-(p-Amidinophenylsulfonamido)propyl]phenylessigsäure,
p-[2-(4-Amidino-3-pyridylamido)äthyl]phenoxyessigsäure-methylester,
p-[2-(4-Amidino-3-pyridylamido)äthyl]phenoxyessigsaure,
p,p'-[[(p-Amidinobenzoyl)imino]diäthylen]-dihydrozimtsäure-dimethylester,
P,P'-[[(P-Amidinobenzoyl)imino]diäthylen]-dihydrozimtsäure,
p-[2-[(p-Amidinophenyl)carbamoyl]äthyl]phenoxyessigsäure-methylester,
p-[2-[(p-Amidinophenyl)carbamoyl]äthyl]phenoxyessigsäure,
p-[2-(p-Amidinobenzamido)-1-hydroxy-äthyl]hydrozimtsäure-methylester,
p-[2-(p-Amidinobenzamido)-1-hydroxyäthyl]hydrozimtsäure,
p-[(p-Amidinobenzamido)acetyl]hydrozimtsäure-methylester,
p-[(p-Amidinobenzamido)acetyl]hydrozimtsäure,
p-[2-(p-Guanidinobenzamido)äthyl]hydrozimtsäure-methylester,
p-[2-(p-Guanidinobenzamido)äthyl]hydrozimtsäure,
[α-(p-Guanidinobenzamido)-p-tolyl]oxy-essigsäure-methylester,
[α-(p-Guanidinobenzamido)-p-tolyl]oxy-essigsäure,
p-[(p-Guanidinobenzamido)methyl]hydrozimtsäure-methylester,
p-[(p-Guanidinobenzamido)methyl]hydrozimtsäure,
N-(p-Amidinobenzoyl)-3-[p-(tert-butoxycarbonyl)methoxy)phenyl]-D-alanin-methylester,
[p-[(R)-2-(p-Amidinobenzamido)-2-(methoxycarbonyl)äthyl]phenoxy]essigsäure,
N-(p-Amidinobenzoyl)-3-[p-(carboxymethoxy)phenyl]-D-alanin,
(E)-p-[2-(p-Amidinobenzamido)äthyl]-β-methyl-zimtsäuremethylester,
rac-p-[2-(p-Amidinobenzamido)äthyl]-β-methylhydrozimtsäuremethylester,
p-[2-(p-Guanidinobenzamido)äthyl]phenoxyessigsäuremethylester,

N-Benzyl-N-[p-[2-(p-amidinobenzaffiido)äthyl]phenyl]glycinmethylester,
[p-[2-(p-Guanidinobenzamido)äthyl]phenoxy]essigsäure,
p-[(R)-2-(p-Amidinobenzamido)-3-hydroxypropyl]phenoxyessigsäure-t-butylester,
p-[(R)-2-(p-Amidinobenzamido)-3-hydroxypropyl]phenoxyessigsäure,
[[4-[p-Amidino-N-methylbenzamido)acetyl]-o-phenylen]dioxy]diessigsäuredimethylester,
[4-(p-Amidino-N-methylbenzamido)acetyl-o-phenylen]dioxy]diessigsäure,
p-[N-(p-Amidinobenzoyl)-(RS)-alanyl]phenoxyessigsäuremethylester,
[p-[N-(p-Amidinobenzoyl)-(R,S)-alanyl]phenoxy]essigsäure,
p-[2-[p-Amidino-N-(p-carbomethoxybenzyl)benzamido]-1-hydroxyäthyl]phenoxyessigsäure-methylester,
p-[2-[p-(Amidino-N-(p-carboxybenzyl)benzamido]-1-hydroxyäthyl]phenoxyessigsäure,
p-[[p-Amidino-N-(p-carbomethoxybenzyl)benzamido]acetyl]phenoxyessigsäure-methylester und
p-[[p-Amidino-N-(p-carboxybenzyl)benzamido]acetyl]phenoxyessigsäure.

**10.** Verfahren nach Anspruch 1, 2 oder 4 zur Herstellung der Verbindungen aus der Gruppe der folgenden:
α-[p-Amidinobenzyl)carbamoyl]-p-tolyloxyessigsäure-methylester,
α-[(p-Amidinobenzyl)carbamoyl]-p-tolyoxyessigsäure,
p-[(p-Amidinophenäthyl)carbamoyl]phenoxyessigsäure-methylester,
p-[(p-Amidinophenäthyl)carbamoyl]phenoxyessigsäure,
[[α-(p-Amidinohydrocinnamamido)-p-tolyl]oxy]essigsäure,
[p-(p-Amidinohydrocinnamamido)phenoxy]essigsäure,
[p-[(E)-p-Amidinocinnamamido]phenoxy]essigsäure,
[[α-[2-(p-Amidinophenyl)acetamido]-p-tolyl]oxy]essigsäuremethylester und
[[p-[2-(p-Amidinophenyl)acetamido]-p-tolyl]oxy]essigsäure.

**11.** Verfahren nach Anspruch 1, dadurch gekennzeichnet, dass man
a) das Nitril der Formel II mit Schwefelwasserstoff und einer Base in einem Lösungsmittel umsetzt, das erhaltene Thioamid methyliert und die erhaltene Verbindung der Formel I, in der an Stelle von $R^1$ die Gruppe $-C(NH)SCH_3$ steht, mit Ammoniumacetat umsetzt,
b) die Schutzgruppen durch katalytische Hydrierung oder durch Behandlung mit einer Säure abspaltet,
c) die Aminogruppe mittels 2-S-Isothioharnstoff-äthansulfonat in Gegenwart einer Base in die Guanidingruppe überführt,
d) die Reaktion der Verbindungen der Formeln V und VI in Gegenwart einer Base bewerkstelligt und die Benzylgruppe $R^5$ hydrogenolytisch abpaltet
und gewünschtenfalls Estergruppen mit einer Base verseift, Aether-, Enoläther-, Ketal- oder Thioketalgruppen mit Säuren spaltet; eine olefinische Doppelbindung katalytisch hydriert, oder eine Carbonsäuregruppe in ein Salz überführt.

**12.** Verfahren zur Herstellung von Arzneimitteln, dadurch gekennzeichnet, dass man eine Verbindung der Formel I oder ein Salz davon in eine galenische Darreichungsform bringt.

**13.** Die Produkte gemäss den Ansprüchen 1-8 zur Verwendung als Wirkstoffe bei der Herstellung von Heilmitteln zur Behandlung von Thrombosen, Apoplexie, Herzinfarkt, Entzündungen, Arteriosklerose sowie von Tumoren.

**Claims**
**Claims for the following Contracting States : AT, BE, CH, DE, DK, FR, GB, IT, LI, LU, NL, SE**

**1.** Compounds of the general formula

$$R^1\text{-}A\text{-}(W)_a\text{-}X\text{-}(CH_2)_b\text{-}(Y)_c\text{-}B\text{-}Z\text{-}COOR \qquad (I)$$

wherein
A signifies a residue

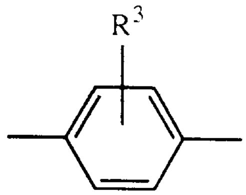 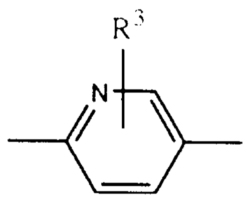 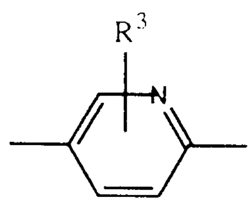 or 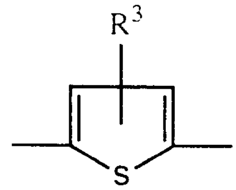 ;

B signifies a residue

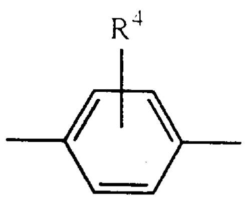 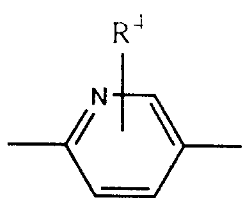 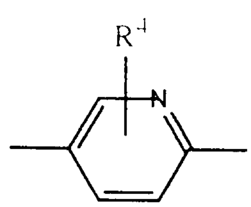 or 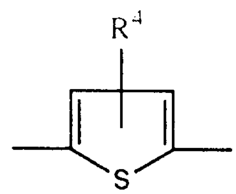 ;

- W signifies $-CH_2-$, $-CH_2CH_2-$, $-CH=CH-$, $-CH=CH-CH_2-$, $-(CH_2)_3-$, $-CH_2CH(CH_3)-$, $-COCH_2-$, $-CH(OH)CH_2-$ or $-CH_2COCH_2-$;
- X signifies $-CONR^2-$, $-NR^2CO-$, $-SO_2NR^2-$ or $-NR^2SO_2-$;
- Y signifies $-CH_2CH_2-$, $-CH_2CH_2O-$, $-OCH_2-$, $CH(CH_3)CH_2-$, $-CH=CH-$, $-CH_2-CH=CH-$, $-C(Q^1,Q^2)-CO(CH_2)_d-$, $-CH_2-$, $-CH_2CH_2CH_2-$, $-CH(CH_3)CH_2CH_2-$, $-CH_2COCH_2-$, $-C(Q^1,Q^2)-CH(OH)-$, $-C(Q^1,Q^2)-CH(SSCH_3)-$, $-CH(CH_2OH)CH_2-$ or $-CH(COOR)CH_2-$, whereby carbonyl groups can also be present as oximes, oxime ethers, ketals or thioketals or enol ethers and hydroxy groups can be present as lower-alkyl ethers, di-(lower alkyl)amino-lower alkyl ethers or as esters of lower alkanecarboxylic acids;
- Z signifies $-OCH_2-$, $-NR^6CH_2-$, $-CH_2CH_2-$, $-CH(CH_3)CH_2-$, $-CH_2-$, $-CH=CH-$ or $-C(CH_3)=CH-$;
- R signifies hydrogen, lower-alkyl, phenyl or phenyl-lower alkyl;
- $Q^1$ and $Q^2$ are hydrogen or lower-alkyl or together with the C atom to which they are attached form a 3- to 6-membered saturated ring;
- $R^1$ signifies amidino or guanidino;
- $R^2$ signifies hydrogen, lower-alkyl, phenyl-lower-alkyl, phenyl-lower-alkyl which is substituted in the phenyl part by amino, amidino or -COOR, or a residue $-CH_2COOR$ or -Y-B-Z-COOR;
- $R^3$ signifies hydrogen, lower-alkyl, lower-alkoxy, halogen, lower-carbalkoxy, amino, lower-alkylamino, di-lower-alkylamino or amidino;
- $R^4$ signifies hydrogen, lower-alkyl, lower-alkoxy, halogen, lower-carbalkoxy, amino, lower-alkylamino, di-lower-alkyl-amino or a residue -Z-COOR or $-CH=CH-(CH_2)_nCOOR$;
- $R^6$ signifies hydrogen, lower-alkyl or benzyl;
- n signifies a whole number of 0-4;
- a, c and d each independently signify 0 or 1;
- b signifies a whole number of 0-2, whereby, however, a and b must be 0 when c is 1 and c must be 0 when a or b is different from 0;

and physiologically compatible salts thereof.

**2.** Compounds according to claim 1, wherein W and X have the significance given in claim 1 and Y signifies $-CH_2CH_2-$,$-CH_2CH_2O-$, $-CH(CH_3)CH_2-$, $-CH=CH-$, $-CH_2-CH=CH-$, $-CH_2CO-$, $-CH_2-$, $-CH_2CH_2CH_2-$, $-CH(CH_3)CH_2CH_2-$, $-CH_2COCH_2-$, $-CH_2CHOH-$, $-CH(CH_2OH)CH_2-$ or $-CH(COOR)CH_2-$, whereby carbonyl groups can also be present as oximes, ketals or thioketals or enol ethers and hydroxy groups can be present as lower-alkyl ethers or as esters of lower alkanecarboxylic acids.

**3.** Compounds in accordance with claim 1 or 2 of the formula

$R^1$-A-X-Y-B-Z-COOR (Ia)

4. Compounds in accordance with claim 1 or 2 of the formula

$R^1$-A-W-X-$(CH_2)_b$-B-Z-COOR (Ib)

5. Compounds in accordance with claims 1-4, in which the sum of the C, O, N and S atoms in $(W)_a$, $(CH_2)_b$, $(Y)_c$, X and Y in a straight chain is 6.

6. Compounds in accordance with claims 1-5, in which $R^1$ is amidino.

7. Compounds in accordance with claims 1-6, in which Y is $-CH(CH_3)CO-$, $-CH_2CH_2$ or $-CH_2CO-$; Z is $-OCH_2-$ or $-CH_2CH_2-$ and X is -NHCO- or -CONH-.

8. Compounds in accordance with claims 1-7, in which R is hydrogen.

9. Compounds according to claim 1, 2 or 3 from the following group:
Methyl p-[2-(p-amidinobenzamido)ethyl]phenoxyacetate,
[p-[2-(p-amidinobenzamido)ethyl]phenoxy]acetic acid,
methyl [4-[2-(p-amidinobenzamido)ethyl]-2-iodophenoxy]acetate,
[4-[2-(p-amidinobenzamido)ethyl]-2-iodophenoxy]acetic acid,
benzyl (E)-5-[5-[2-(p-amidinobenzamido)ethyl]-2-(carbomethoxymethoxy)phenyl]-4-pentenoate,
(E)-5-[5-[2-(p-amidinobenzamido)ethyl]-2-(carboxymethoxy)phenyl]-4-pentenoic acid,
methyl p-[2-(p-amidinobenzamido)ethoxyphenyl]acetate,
p-[2-(p-amidinobenzamido)ethoxy]phenylacetic acid,
methyl p-[2-(p-amidinophenylsulphonamido)ethoxy]phenylacetate,
p-[2-(p-amidinophenylsulphonamido)ethoxy]phenylacetic acid,
methyl p-[2-(p-amidinobensolsulphonamido)ethyl]phenoxyacetate,
p-[2-(p-amidinobenzenesulphonamido)ethyl]phenoxyacetic acid,
methyl p-[(S)-2-(p-amidinobenzenesulphonamido)propyl]phenoxyacetate acetate,
p-[(S)-2-(p-amidinobenzenesulphonamido)propyl]phenoxyacetic acid,
methyl p-[2-(p-amidino-N-methylphenylsulphonamido)ethyl]phenoxyacetate,
p-[2-(p-amidino-N-methylphenylsulphonamido)ethyl]phenoxyacetic acid,
methyl p-[2-(p-(amidino-N-benzylphenylsulphonamido)ethyl]phenoxyacetate,
p-[2-(p-amidino-N-benzylphenylsulphonamido)ethyl]phenoxyacetic acid,
methyl p-[2-(p-amidinobenzenesulphonamido)-1-hydroxyethyl]phenoxyacetate,
p-[2-(p-amidinobenzenesulphonamido)-1-hydroxyethyl]phenoxyacetic acid,
methyl p-[2-(p-amidinobenzamido)-1-hydroxyethyl]phenoxyacetate,
p-[2-(p-amidinobenzamido)-1-hydroxyethyl]phenoxyacetic acid,
methyl p-[(p-amidinophenylsulphonamido)acetyl]phenoxyacetate,
p-[(p-amidinophenylsulphonamido)acetyl]phenoxyacetic acid,
methyl p-[(p-amidinobenzamido)acetyl]phenoxyacetate,
p-[(p-amidinobensamido)acetyl]phenoxyacetic acid,
methyl p-[2-[p-amidino-N-(p-carbomethoxybenzyl)phenylsulphonamido]ethyl]phenoxyacetate,
p-[2-[p-amidino-N-(p-carboxybenzyl)phenylsulphonamido]ethyl]phenoxyacetic acid,
dimethyl [[4-[2-(p-amidinopheny(sulphonamido)ethyl]-o-phenylene]dioxy]diacetate,
[[4-[2-(p-amidinophenylsulphonamido)ethyl]-o-phenylene]dioxy]diacetic acid,
methyl [4-[2-(p-amidinophenylsulphonamido)ethyl]-2-methoxyphenoxy]acetate,
[4-[2-(p-amidinophenylsulphonamido)ethyl]-2-methoxy-phenoxy]acetic acid,
methyl (E)-p-[2-(p-amidinophenylsulphonamido)ethyl]-$\beta$-methylcinnamate,
(E)-p-[2-(p-amidinophenylsulphonamido)ethyl]-$\beta$-methylcinnamic acid,
methyl p-[2-(p-amidinophenylsulphonamido)ethyl]-$\beta$-methyl-hydrocinnamate,
p-[2-(p-amidinophenylsulphonamido)ethyl]-$\beta$-methylhydrocinnamic acid,
ethyl (E)-5-[(RS)-2-(p-amidinobenzamido)propyl]-$\beta$-methyl-2-thiopheneacrylate,
(E)-5-[(RS)-2-(p-amidinobenzamido)propyl]-$\beta$-methyl-2-thiopheneacrylic acid,
ethyl (RS)-5-[(RS)-2-(p-amidinobenzamido)propyl]-$\beta$-methyl-2-thiophenepropionate,
(RS)-5-[(RS)-2-(p-amidinobenzamido)propyl]-$\beta$-methyl-2-thiophenepropionic acid,
methyl p-[2-(p-amidinobenzamido)ethyl]-hydrocinnamate,
p-[2-(p-amidinobenzamido)ethyl]-hydrocinnamic acid,
methyl p-[3-(p-amidinobenzamido)propyl]phenylacetate,

p-[3-(p-amidinobenzamido)propyl]pnenylacetic acid,
methyl p-[3-(p-amidinophenylsulphonamido)propyl]phenylacetate,
p-[3-(p-amidinophenylsulphonamido)propyl]phenylacetic acid,
methyl p-[2-(4-amidino-3-pyridylamido)ethyl]phenoxyacetate,
p-[2-(4-amidino-3-pyridylamido)ethyl]phenoxyacetic acid,
dimethyl p,p'-[[(p-amidinobenzoyl)imino]diethylene]dihydrocinnamate,
p,p'-[[(p-amidinobenzoyl)imino]diethylene]-dihydrocinnamic acid,
methyl p-[2-[(p-amidinophenyl)carbamoyl]ethyl]phenoxyacetate,
p-[2-[(p-amidinophenyl)carbamoyl]ethyl]phenoxyacetic acid,
methyl p-[2-(p-amidinobenzamido)-1-hydroxyethyl]hydrocinnamate,
p-[2-(p-amidinobenzamido)-1-hydroxyethyl]hydrocinnamic acid,
methyl p-[(p-amidinobenzamido)acetyl]hydrocinnamate,
p-[(p-amidinobenzamido)acetyl]hydrocinnamic acid,
methyl p-[2-(p-guanidinobenzamido)ethyl]hydrocinnamate,
p-[2-(p-guanidinobenzamido)ethyl]hydrocinnamic acid,
methyl [α-(p-guanidinobenzamido)-p-tolyl]oxy-acetate,
[α-(p-guanidinobenzamido)-p-tolyl]oxy-acetic acid,
methyl p-[(p-guanidinobenzamido)methyl]hydrocinnamate,
p-[(p-guanidinobenzamido)methyl]hydrocinnamic acid,
N-(p-amidinobenzoyl)-3-[p-(tert-butoxycarbonyl)methoxy)phenyl]-D-alanine methyl ester,
[p-[(R)-2-(p-amidinobenzamido)-2-(methoxycarbonyl)ethyl]phenoxy]acetic acid,
N-(p-amidinobenzoyl)-3-[p-(carboxymethoxy)phenyl]-D-alanine,
methyl (E)-p-[2-(p-amidinobenzamido)ethyl]-β-methylcinnamate,
methyl rac-p-[2-(p-amidinobenzamido)ethyl]-β-methylhydrocinnamate,
methyl p-[2-(p-guanidinobenzamido)ethyl]phenoxyacetate,
N-benzyl-N-[p-[2-(p-amidinobenzamido)ethyl]phenyl]glycinemethyl ester,
[p-[2-(p-guanidinobenzamido)ethyl]phenoxy]acetic acid,
t-butyl p-[(R)-2-(p-amidinobenzamido)-3-hydroxypropyl]phenoxyacetate,
p-[(R)-2-(p-amidinobenzamido)-3-hydroxypropyl]phenoxyacetic acid,
dimethyl [[4-[p-amidino-N-methylbenzamido)acetyl]-o-phenylene]dioxy]diacetate,
[4-(p-amidino-N-methylbenzamido)acetyl-o-phenylene]dioxy]acetic acid,
methyl p-[N-(p-amidinobenzoyl)-(RS)-alanyl]phenoxyacetate,
[p-[N-(p-amidinobenzoyl)-(R,S)-alanyl]phenoxy]acetic acid,
methyl p-[2-[p-amidino-N-(p-carbomethoxybenzyl)benzamido]-1-hydroxyethyl]phenoxyacetate,
p-[2-[p-(amidino-N-(p-carboxybenzyl)benzamido]-1-hydroxy-ethyl]phenoxyacetic acid,
methyl p-[[p-amidino-N-(p-carbomethoxybenzyl)benzamido]acetyl]phenoxyacetate and
p-[[p-amidino-N-(p-carboxybenzyl)benzamido]acetyl]phenoxyacetic acid.

**10.** Compounds according to claim 1, 2 or 4 from the following group:
Methyl α-[(p-amidinobenzyl)carbamoyl]-p-tolyloxyacetate,
α-[(p-amidinobenzyl)carbamoyl]-p-tolyloxyacetic acid,
methyl p-[2-[(p-amidinophenethyl)carbamoyl]phenoxyacetate,
p-[2-[(p-amidinophenethyl)carbamoyl]phenoxy]acetic acid,
[[α-(p-amidinohydrocinnamamido)-p-tolyl]oxy]acetic acid,
[p-(p-amidinohydrocinnamamido)phenoxy]acetic acid,
[p-[(E)-p-amidinocinnamamido]phenoxy]acetic acid,
methyl [[α-[2-(p-amidinophenyl)acetamido]-p-tolyl]oxy]acetate and
[[p-[2-(p-amidinophenyl)acetamido]-p-tolyl]oxy]acetic acid.

**11.** The compounds in accordance with claims 1-10 for use as medicaments.

**12.** The compounds in accordance with claims 1-10 for use as active ingredients in the manufacture of medicaments for the treatment of thromboses, stroke, cardiac infarct, inflammations, arteriosclerosis and of tumours.

**13.** Pharmaceutical preparations containing a compound in accordance with claims 1-10 and usual pharmaceutical carrier materials.

**14.** A process for the manufacture of compounds of general formula I of claim 1 and their salts, characterized by

a) converting the nitrile group in a compound of the general formula

$NC\text{-}A\text{-}(W)_a\text{-}X\text{-}(CH_2)_b\text{-}(Y)_c\text{-}B\text{-}Z\text{-}COOR^5$ (II)

wherein $R^5$ is lower-alkyl or benzyl and A, B, W, X, Y, Z, a, b and c have the significance given above and wherein carboxy groups which may exist in the molecule are present as esters, into the amidino group, or

b) cleaving off the protecting groups on the amidino or guanidino group in a compound of the general formula

$R^{11}\text{-}A\text{-}(W)_a\text{-}X\text{-}(CH_2)_b\text{-}(Y)_c\text{-}B\text{-}Z\text{-}COOR^5$ (III)

wherein A, B, W, X, Y, Z, $R^5$, a, b and c have the significance given above and $R^{11}$ represents a protected amidno or guanidino group,
or

c) converting the amino group in a compound of the general formula

$H_2N\text{-}A\text{-}(W)_a\text{-}X\text{-}(CH_2)_b\text{-}(Y)_c\text{-}B\text{-}Z\text{-}COOR^5$ (IV)

wherein A, B, W, X, Y, Z, $R^5$, a, b and c have the significance given above and wherein carboxy groups which may exist in the molecule are present as esters, into the guanidino group, or

d) reacting a compound of the general formula

$R^1\text{-}A\text{-}(W)_a\text{-}COE$ (V)

wherein E represents an activated ester group or chlorine or bromine and $R^1$, A, W and a have the significance given above,
with a compound of the general formula

$HNR^2\text{-}(CH_2)_b\text{-}(Y)_c\text{-}B\text{-}Z\text{-}COOR^5$ (VI)

wherein Y, B, Z, $R^5$, $R^2$, b and c have the significance given above,
cleaving off a benzyl group $R^5$ which may be present
and, if desired, modifying functional groups present in a thus-obtained compound of general formula I.

**Claims for the following Contracting States : ES, GR**

**1.** A process for the manufacture of compounds of the general formula

$R^1\text{-}A\text{-}(W)_a\text{-}X\text{-}(CH_2)_b\text{-}(Y)_c\text{-}B\text{-}Z\text{-}COOR$ (I)

wherein

A signifies a residue

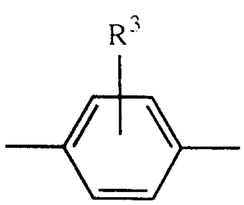 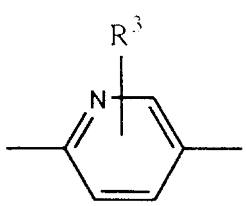 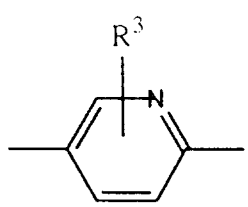 or 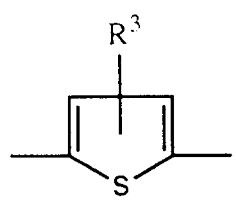 ;

B signifies a residue

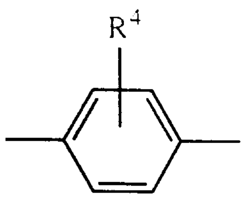 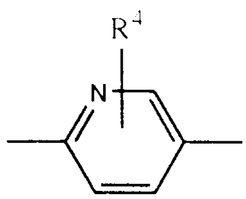 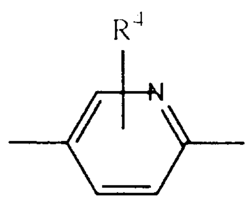 or 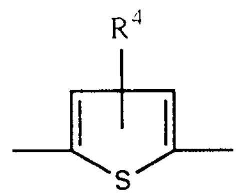 ;

| | |
|---|---|
| W | signifies $-CH_2-$, $-CH_2CH_2-$, $-CH=CH-$, $-CH=CH-CH_2-$, $-(CH_2)_3-$, $-CH_2CH(CH_3)-$, $-COCH_2-$, $-CH(OH)CH_2-$ or $-CH_2COCH_2-$; |
| X | signifies $-CONR^2-$, $-NR^2CO-$, $-SO_2NR^2-$ or $-NR^2SO_2-$; |
| Y | signifies $-CH_2CH_2-$, $-CH_2CH_2O-$, $-OCH_2-$, $CH(CH_3)CH_2-$, $-CH=CH-$, $-CH_2-CH=CH-$, $-C(Q^1,Q^2)-CO(CH_{2})_d-$, $-CH_2-$, $-CH_2CH_2CH_2-$, $-CH(CH_3)CH_2CH_2-$, $-CH_2COCH_2-$, $-C(Q^1,Q^2)-CH(OH)-$, $-C(Q^1,Q^2)-CH(SSCH_3)-$, $-CH(CH_2OH)CH_2-$ or $-CH(COOR)CH_2-$, whereby carbonyl groups can also be present as oximes, oxime ethers, ketals or thioketals or enol ethers and hydroxy groups can be present as lower-alkyl ethers, di-(lower alkyl)amino-lower alkyl ethers or as esters of lower alkanecarboxylic acids |
| Z | signifies $-OCH_2-$, $-NR^6CH_2-$, $-CH_2CH_2-$, $-CH(CH_3)CH_2-$, $-CH_2-$, $-CH=CH-$ or $-C(CH_3)=CH-$; |
| R | signifies hydrogen, lower-alkyl, phenyl or phenyl-lower-alkyl; |
| $Q^1$ and $Q^2$ | are hydrogen or lower-alkyl or together with the C atom to which they are attached form a 3- to 6-membered saturated ring; |
| $R^1$ | signifies amidino or guanidino; |
| $R^2$ | signifies hydrogen, lower-alkyl, phenyl-lower-alkyl, phenyl-lower-alkyl which is substituted in the phenyl part by amino, amidino or -COOR, or a residue $-CH_2COOR$ or -Y-B-Z-COOR; |
| $R^3$ | signifies hydrogen, lower-alkyl, lower-alkoxy, halogen, lower-carbalkoxy, amino, lower-alkylamino, di-lower-alkylamino or amidino; |
| $R^4$ | signifies hydrogen, lower-alkyl, lower-alkoxy, halogen, lower-carbalkoxy, amino, lower-alkylamino, di-lower-alkyl-amino or a residue -Z-COOR or $-CH=CH-(CH_2)_nCOOR$; |
| $R^6$ | signifies hydrogen, lower-alkyl or benzyl; |
| n | signifies a whole number of 0-4; |
| a, c and d | each independently signify 0 or 1; |
| b | signifies a whole number of 0-2, whereby, however, a and b must be 0 when c is 1 and c must be 0 when a or b is different from 0; |

and of physiologically caompatible salts thereof, characterized by

a) converting the nitrile group in a compound of the general formula

$NC-A-(W)_a-X-(CH_2)_b-(Y)_c-B-Z-COOR^5$ (II)

wherein $R^5$ is lower-alkyl or benzyl and A, B, W, X, Y, Z, a, b and c have the significance given above and wherein carboxy groups which may exist in the molecule are present as esters, into the amidino group, or

b) cleaving off the protecting groups on the amidino or guanidino group in a compound of the general formula

$R^{11}-A-(W)_a-X-(CH_2)_b-(Y)_c-B-Z-COOR^5$ (III)

wherein A, B, W, X, Y, Z, $R^5$, a, b and c have the significance given above and $R^{11}$ represents a protected amidino or guanidino group,

or

c) converting the amino group in a compound of the general formula

$H_2N-A-(W)_a-X-(CH_2)_b-(Y)_c-B-Z-COOR^5$ (IV)

wherein A, B, W, X, Y, Z, $R^5$, a, b and c have the significance given above and wherein carboxy groups which may exist in the molecule are present as esters,

into the guanidino group, or
d) reacting a compound of the general formula

$R^1$-A-$(W)_a$-COE (V)

wherein E represents an activated ester group or chlorine or bromine and $R^1$, A, W and a have the significance given above,
with a compound of the general formula

$HNR^2$-$(CH_2)_b$-$(Y)_c$-B-Z-$COOR^5$ (VI)

wherein Y, B, Z, $R^5$, $R^2$, b and c have the significance given above,
cleaving off a benzyl group $R^5$ which may be present
and, if desired, modifying functional groups present in a thus-obtained compound of general formula I.

**2.** A process according to claim 1, wherein W and X have the significance given in claim 1 and Y signifies $-CH_2CH_2-$,$-CH_2CH_2O-$, $-CH(CH_3)CH_2-$, $-CH=CH-$, $-CH_2-CH=CH-$, $-CH_2CO-$, $-CH_2-$, $-CH_2CH_2CH_2-$, $-CH(CH_3)CH_2CH_2-$, $-CH_2COCH_2-$, $-CH_2CHOH-$, $-CH(CH_2OH)CH_2-$ or $-CH(COOR)CH_2-$, whereby carbonyl groups can also be present as oximes, ketals or thioketals or enol ethers and hydroxy groups can be present as lower-alkyl ethers or as esters of lower alkanecarboxylic acids.

**3.** A process in accordance with claim 1 or 2, characterized in that compounds of the formula

$R^1$-A-X-Y-B-Z-COOR (Ia)

are manufactured.

**4.** A process in accordance with claim 1 or 2, characterized in that compounds of the formula

$R^1$-A-W-X-$(CH_2)_b$-B-Z-COOR (Ib)

are manufactured.

**5.** A process in accordance with claims 1-4, in which the sum of the C, O, N and S atoms in $(W)_a$, $(CH_2)_b$, $(Y)_c$, X and Y in a straight chain is 6.

**6.** A process in accordance with claims 1-5, in which $R^1$ is amidino.

**7.** A process in accordance with claims 1-6, in which Y is $-CH(CH_3)CO-$, $-CH_2CH_2$ or $-CH_2CO-$; Z is $-OCH_2-$ or $-CH_2CH_2-$ and X is -NHCO- or -CONH-.

**8.** A process in accordance with claims 1-7, in which R is hydrogen.

**9.** A process according to claim 1, 2 or 3 for the manufacture of the compounds from the following group:
Methyl p-[2-(p-amidinobenzamido)ethyl]phenoxyacetate,
[p-(2-(p-amidinobenzamido)ethyl]phenoxy]acetic acid,
methyl [4-[2-(p-amidinobenzamido)ethyl]-2-iodophenoxy]acetate,
[4-[2-(p-amidinobenzamido)ethyl]-2-iodophenoxy]acetic acid,
benzyl (E)-5-[5-[2-(p-amidinobenzamido)ethyl]-2-(carbomethoxymethoxy)phenyl]-4-pentenoate,
(E)-5-[5-[2-(p-amidinobenzamido)ethyl]-2-(carboxymethoxy)phenyl]-4-pentenoic acid,
methyl p-[2-(p-amidinobenzamido)ethoxyphenyl]acetate,
p-[2-(P-amidinobenzamido)ethoxy]phenylacetic acid,
methyl p-[2-(p-amidinophenylsulphonamido)ethoxy]phenylacetate,
p-[2-(p-amidinophenylsulphonamido)ethoxy]phenylacetic acid,
methyl p-[2-(p-amidinobenzolsulphonamido)ethyl]phenoxyacetate,
p-[2-(p-amidinobenzenesulphonamido)ethyl]phenoxyacetic acid,
methyl p-[(S)-2-(p-amidinobenzenesulphonamido)propyl]phenoxyacetate acetate,

p-[(S)-2-(p-amidinobenzenesulphonamido)propyl]phenoxyacetic acid,
methyl p-[2-(p-amidino-N-methylphenylsulphonamido)ethyl]phenoxyacetate,
p-[2-(p-amidino-N-methylphenylsulphonamido)ethyl]phenoxyacetic acid,
methyl p-[2-(p-(amidino-N-benzylphenylsulphonamido)ethyl]phenoxyacetate,
p-[2-(p-amidino-N-benzylphenylsulphonamido)ethyl]phenoxyacetic acid,
methyl p-[2-(p-amidinobenzenesulphonamido)-1-hydroxyethyl]phenoxyacetate,
p-[2-(p-amidinobenzenesulphonamido)-1-hydroxyethyl]phenoxyacetic acid,
methyl p-[2-(p-amidinobenzamido)-1-hydroxyethyl]phenoxyacetate,
p-[2-(p-amidinobenzamido)-1-hydroxyethyl]phenoxyacetic acid,
methyl p-[(p-amidinophenylsulphonamido)acetyl]phenoxyacetate,
p-[(p-amidinophenylsulphonamido)acetyl]phenoxyacetic acid,
methyl p-[(p-amidinobenzamido)acetyl]phenoxyacetate,
p-[(p-amidinobenzamido)acetyl]phenoxyacetic acid,
methyl p-[2-[p-amidino-N-(p-carbomethoxybenzyl)phenylsulphonamido]ethyl]phenoxyacetate,
p-[2-[p-amidino-N-(p-carboxybenzyl)phenylsulphonamido]ethyl]phenoxyacetic acid,
dimethyl [[4-[2-(p-amidinophenylsulphonamido)ethyl]-o-phenylene]dioxy]diacetate,
[[4-[2-(p-amidinophenylsulphonamido)ethyl]-o-phenylene]dioxy]diacetic acid,
methyl [4-[2-(p-amidinophenylsulphonamido)ethyl]-2-methoxyphenoxy]acetate,
[4-[2-(p-amidinophenylsulphonamido)ethyl]-2-methoxyphenoxy]acetic acid,
methyl (E)-p-[2-(p-amidinophenylsulphonamido)ethyl]-β-methylcinnamate,
(E)-p-[2-(p-amidinophenylsulphonamido)ethyl]-β-methylcinnamic acid,
methyl p-[2-(p-amidinophenylsulphonamido)ethyl]-β-methyl-hydrocinnamate,
p-[2-(p-amidinophenylsulphonamido)ethyl]-β-methylhydrocinnamic acid,
ethyl (E)-5-[(RS)-2-(p-amidinobenzamido)propyl]-β-methyl-2-thiopheneacrylate,
(E)-5-[(RS)-2-(p-amidinobenzamido)propyl]-β-methyl-2-thiopheneacrylic acid,
ethyl (RS)-5-[(RS)-2-(p-amidinobenzamido)propyl]-β-methyl-2-thiophenepropionate,
(RS)-5-[(RS)-2-(p-amidinobenzamido)propyl]-β-methyl-2-thiophenepropionic acid,
methyl p-[2-(p-amidinobenzamido)ethyl]-hydrocinnamate,
p-[2-(p-amidinobenzamido)ethyl]-hydrocinnamic acid,
methyl p-[3-(p-amidinobenzamido)propyl]phenylacetate,
p-[3-(p-amidinobenzamido)propyl]phenylacetic acid,
methyl p-[3-(p-amidinophenylsulphonamido)propyl]phenylacetate,
p-[3-(p-amidinophenylsulphonamido)propyl]phenylacetic acid,
methyl p-[2-(4-amidino-3-pyridylamido)ethyl]phenoxyacetate,
p-[2-(4-amidino-3-pyridylamido)ethyl]phenoxyacetic acid,
dimethyl p,p'-[[(p-amidinobenzoyl)imino]diethylene]dihydrocinnamate,
p,p'-[[(p-amidinobenzoyl)imino]diethylene]-dihydrocinnamic acid,
methyl p-[2-[(p-amidinophenyl)carbamoyl]ethyl]phenoxyacetate,
p-[2-[(p-amidinophenyl)carbamoyl]ethyl]phenoxyacetic acid,
methyl p-[2-(p-amidinobenzamido)-1-hydroxyethyl]hydrocinnamate,
p-[2-(p-amidinobenzamido)-1-hydroxyethyl]hydrocinnamic acid,
methyl p-[(p-amidinobenzamido)acetyl]hydrocinnamate,
p-[(p-amidinobenzamido)acetyl]hydrocinnamic acid,
methyl p-[2-(p-guanidinobenzamido)ethyl]hydrocinnamate,
p-[2-(p-guanidinobenzamido)ethyl]hydrocinnamic acid,
methyl [α-(p-guanidinobenzamido)-p-tolyl]oxy-acetate,
[α-(p-guanidinobenzamido)-p-tolyl]oxy-acetic acid,
methyl p-[(p-guanidinobenzamido)methyl]hydrocinnamate,
p-[(p-guanidinobenzamido)methyl]hydrocinnamic acid,
N-(p-amidinobenzoyl)-3-[p-(tert-butoxycarbonyl)methoxy)phenyl]-D-alanine methyl ester,
[p-[(R)-2-(p-amidinobenzamido)-2-(methoxycarbonyl)ethyl]phenoxy]acetic acid,
N-(p-amidinobenzoyl)-3-[p-(carboxymethoxy)phenyl]-D-alanine,
methyl (E)-p-[2-(p-amidinobenzamido)ethyl]-β-methylcinnamate,
methyl rac-p-[2-(p-amidinobenzamido)ethyl]-β-methylhydrocinnamate,
methyl p-[2-(p-guanidinobenzamido)ethyl]phenoxyacetate,
N-benzyl-N-[p-[2-(p-amidinobenzamido)ethyl]phenyl]glycinemethyl ester,
[p-[2-(p-guanidinobenzamido)ethyl]phenoxy]acetic acid,
t-butyl p-[(R)-2-(p-amidinobenzamido)-3-hydroxypropyl]phenoxyacetate,

p-[(R)-2-(p-amidinobenzamido)-3-hydroxypropyl]phenoxyacetic acid,
dimethyl [[4-[p-amidino-N-methylbenzamido)acetyl]-o-phenylene]dioxy]diacetate,
[4-(p-amidino-N-methylbenzamido)acetyl-o-phenylene]dioxy]acetic acid,
methyl p-[N-(p-amidinobenzoyl)-(RS)-alanyl]phenoxyacetate,
[p-[N-(p-amidinobenzoyl)-(R,S)-alanyl]phenoxy]acetic acid,
methyl p-[2-[p-amidino-N-(p-carbomethoxybenzyl)benzamido]-1-hydroxyethyl]phenoxyacetate,
p-[2-[p-(amidino-N-(p-carboxybenzyl)benzamido]-1-hydroxy-ethyl]phenoxyacetic acid,
methyl p-[[p-amidino-N-(p-carbomethoxybenzyl)benzamido]acetyl]phenoxyacetate and
p-[[p-amidino-N-(p-carboxybenzyl)benzamido]acetyl]phenoxyacetic acid.

**10.** A process according to claim 1, 2 or 4 for the manufacture of the compoundsfrom the following group:
Methyl α-[(p-amidinobenzyl)carbamoyl]-p-tolyloxyacetate,
α-[(p-amidinobenzyl)carbamoyl]-p-tolyloxyacetic acid,
methyl p-[2-[(p-amidinophenethyl)carbamoyl]phenoxyacetate,
p-[2-[(p-amidinophenethyl)carbamoyl]phenoxy]acetic acid,
[[α-(p-amidinohydrocinnamamido)-p-tolyl]oxy]acetic acid,
[p-(p-amidinohydrocinnamamido)phenoxy]acetic acid,
[p-[(E)-p-amidinocinnamamido]phenoxy]acetic acid,
methyl [[α-[2-(p-amidinophenyl)acetamido]-p-tolyl]oxy]acetate and
[[p-[2-(p-amidinophenyl)acetamido]-p-tolyl]oxy]acetic acid.

**11.** A process according to claim 1 characterized by
a) reacting the nitrile of formula II with hydrogen sulphide and a base in a solvent, methylating the resulting thioamide and reacting the resulting compound of formula I in which the group $-C(NH)SCH_3$ is present in place of $R^1$ with ammonium acetate,
b) cleaving off the protecting groups by catalytic hydrogenation or by treatment with an acid,
c) converting the amino group into the guanidino group using 2-S-isothioureamethane sulphonate in the presence of a base,
d) carrying out the reaction of the compounds of formulae V and VI in the presence of a base and hydrogenolytically cleaving off the benzyl group $R^5$
and, if desired, saponifying ester groups with a base, cleaving ether, enol ether, ketal or thio ketal groups with acids; catalytically hydrogenating an olefinic double bond, or converting a carboxylic acid group into a salt.

**12.** A process of the manufacture of medicaments, characterized by bringing a compound of formula I or a salt thereof into a galenical administration form.

**13.** The products in accordance with claims 1-8 for use as active substances in the manufacture of medicaments for the treatment of throboses, stroke, cardiac infarct, inflammations, arteriosclerosis and of tumours.

**Revendications**
**Revendications pour les Etats contractants suivants : AT, BE, CH, DE, DK, FR, GB, IT, LI, LU, NL, SE**

**1.** Composés de formule générale

$R^1$-A-$(W)_a$-X-$(CH_2)_b$-$(Y)_c$-B-Z-COOR (I)

où
A désigne un resta

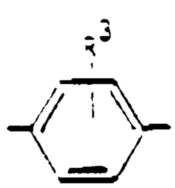 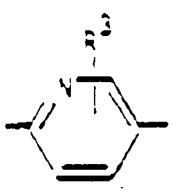 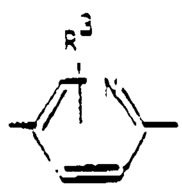 ou 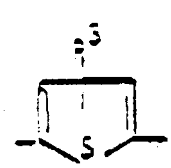 ;

B désigne un reste

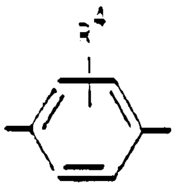 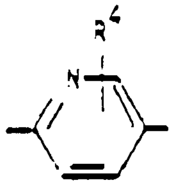 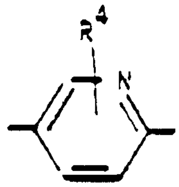 ou 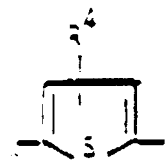 ;

W représente $-CH_2-$, $-CH_2CH_2-$, $-CH=CH-$, $-CH=CH-CH_2-$, $-(CH_2)_3-$, $-CH_2CH(CH_3)-$, $-COCH_2-$, $-CH(OH)CH_2-$ ou $-CH_2COCH_2-$;

X représente $-CONR^2-$, $-NR^2CO-$, $SO_2NR^2-$ ou $-NR^2SO_2-$;

Y représente $-CH_2CH_2-$, $-CH_2CH_2O-$, $-OCH_2-$, $-CH(CH_3)CH_2-$,$-CH=CH-$, $-CH_2-CH=CH-$, $-C(Q^1,Q^2)-CO(CH_2)_d-$, $-CH_2-$, $-CH_2CH_2CH_2-$, $-CH(CH_3)CH_2CH_2-$, $-CH_2COCH_2-$, $-C-(Q^1,Q^2)-CH(OH)-$, $-C((Q^1,Q^2)-CH(SSCH_3)-$, $-CH(CH_2OH)CH_2-$ ou $-CH(COOR)CH_2-$, tandis que les groupes carbonyle peuvent également être présents sous forme de groupes oxime, éther d'oxime, cétal ou thiocétal ou éther d'énol et les groupes hydroxy sous forme de groupes éther d'alkyle inférieur, éther de di(alkyl inférieur)amino-alkyle iniérieur ou sous forme de groupes ester d'acides alcanecarboxyliques inférieurs,

Z désigne $-OCH_2-$, $-NR^6CH_2$, $-CH_2CH_2-$, $-CH(CH_3)CH_2-$, $-CH_2-$, $-CH=CH-$ ou $-C(CH_3)=CH-$;

R représente l'hydrogène ou un groupe alkyle inférieur, phényle ou phényl-alkyle inférieur;

$Q^1$ et $Q^2$ sont l'hydrogène ou un groupe alkyle inférieur ou forment avec l'atome de carbone auquel ils sont liés un cycle saturé à 3 à 6 chaînons;

$R^1$ désigne un groupe amidino ou guanidino;

$R^2$ désigne l'hydrogène ou un groupe alkyle inférieur, phényl-alkyle inférieur, phényl-alkyle inférieur substitué dans la partie phényle par des radicaux amino, amidino ou -COOR, ou un reste $-CH_2COOR$ ou Y-B-Z-COOR;

$R^3$ représente l'hydrogène ou un groupe alkyle inférieur, alcoxy inférieur, halogène, carbalcoxy inférieur, amino, alkylamino inférieur, dialkyl(inférieur)amino ou amidino;

$R^4$ représente l'hydrogène ou un groupe alkyle inférieur, alcoxy inférieur, halogène, carbalcoxy inférieur, amino, alkylamino inférieur, dialkyl(inférieur)amino ou un reste -Z-COOR ou $-CH=CH-(CH_2)_nCOOR$;

$R^6$ désigne l'hydrogène ou un groupe alkyle inférieur ou benzyle;

n représente un nomore entier de 0 à 4;

a c et d sont, indépendamment l'un de l'autre, 0 ou 1; et

b désigne un nombre entier de 0 à 2, tandis que cependant a et b doivent valoir 0 lorsque c est 1, et c doit valoir 0 lorsque a ou b est différent de 0;

et leurs sels physiologiquement acceptables.

2. Composés selon la revendication 1, dans lesquels W et X ont la signification indiquée dans la revendication 1 et Y désigne $-CH_2CH_2-$, $-CH_2CH_2O-$, $-CH(CH_3)CH_2$, $-CH=CH-$, $-CH_2-CH=CH-$, $-CH_2CO-$, $-CH_2-$, $-CH_2CH_2CH_2-$, $-CH(CH_3)CH_2CH_2-$, $-CH_2COCH_2-$, $-CH_2CHOH-$, $-CH(CH_2OH)CH_2-$ ou $-CH(COOR)CH_2-$, tandis que les groupes carbonyle peuvent également être présents sous forme de groupes oxime, éther d'oxime, cétal ou thiocétal ou étber d'énol et les groupes hydroxy sous forme de groupes éther d'alkyle inférieur, ou sous forme de groupes ester d'acides alcanecarboxyliques inférieurs.

3. Composés selon l'une des revendications 1 ou 2, de formule

$R^1$-A-X-Y-B-Z-COOR (Ia)

4. Composés selon l'une des revendîcations 1 ou 2, de formule

$R^1-A-W-X-(CH_2)_b-B-Z-COOR$ (Ib)

5. Composés selon l'une quelconque des revendications 1-4, dans lesquels la somme des atomes de C, O, N et S présents en chaîne droite dans $(W)_a$, $(CH_2)_b$, $(Y)_c$, X et Z est de 6.

6. Composés selon l'une quelconque des revendications 1-5, dans lesquels $R^1$ est un groupe amidino.

7. Composés selon l'une quelconque des revendications 1-6, dans lesquels Y est $-CH(CH_3)CO-$, $-CH_2CH_2-$ ou $-CH_2CO-$; Z est $-OCH_2-$ ou $-CH_2CH_2-$ et X est -NHCO- ou -CONH-.

8. Composés selon l'une quelconque des revendications 1-7, dans lesquels R est de l'hydrogène.

9. Composés selon l'une quelconque des revendications 1, 2 ou 3 du groupe de ceux qui suivent :
ester méthylique de l'acide p-[2-(p-amidinobenzamido)éthyl]phénoxyacétique,
acide [p-[2-(p-amidinobenzamido)éthyl]phénoxy]acétique,
[4-[2-(p-amidinobenzamido)éthyl]-2-iodophénoxy]acétate de méthyle,
acide [4-[2-(p-amidinobenzamido)éthyl]-2-iodophénoxy]acétique,
ester benzylique de l'acide (E)-5-[5-[2-(p-amidino-benzamido)éthyl]-2-(carboxyméthoxy)phényl]-4-penténoïque,
acide (E)-5-[5-[2-(p-amidinobenzamido)éthyl]-2-(carboxyméthoxy)phényl]-4-penténoïque,
ester méthylique de l'acide p-[2-(p-amidinobenzamido)éthoxy]phénylacétique,
acide p-[2-(p-amidinobenzamido)éthoxy]phénylacétique,
ester méthylique de l'acide p-[2-(p-amidinophénylsulfonamido)éthoxy]phénylacétique,
acide p-[2-(p-amidinophénylsulfonamido)éthoxy]phénylacétique,
ester méthylique de l'acide p-[2-(p-amidinobenzènesulfonamido)éthyl]phénoxyacétique,
acide p-[2-(p-amidinobenzènesulfonamido)éthyl]phénoxyacétique,
ester méthylique de l'acide p-[(S)-2-(p-amidinobenzènesulfonamido)propyl]phénoxyacétique,
acide p-[(S)-2-(p-amidinobenzène-sulfonamido)propyl]phénoxyacétique,
ester méthylique de l'acide p-[2-(p-amidino-N-méthylphénylsulfonamido)éthyl]phénoxyacétique,
acide p-[2-(p-amidino-N-méthylphénylsulfonamido)éthyl]phénoxyacétique,
ester méthylique de l'acide p-[2-(p-amidino-N-benzylphénylsulfonamido)éthyl]-phénoxyacétique,
acide p-[2-(p-amidino-N-benzylphénylsulfonamido)éthyl]phénoxyacétique,
ester méthylique de l'acide p-[2-(p-amidinobenzènesulfonamido)-1-hydroxyéthyl]phénoxyacétique,
acide p-[2-(p-amidinobenzènesulfon-amido)-1-hydroxyéthyl]phénoxyacétique,
ester méthylique de l'acide p-[2-(p-amidinobenzamido)-1-hydroxyéthyl]-phénoxyacétique,
acide p-[2-(p-amidinobenzamido)-1-hydroxyéthyl]phénoxyacétique,
ester méthylique de l'acide p-[(p-amidinophénylsulfonamido)acétyl]phénoxyacétique,
acide p-[(p-amidinophénylsulfonamido)acétyl]phénoxyacétique,
ester méthylique de l'acide p-[(p-amidinobenzamido)acétyl]phénoxyacétique,
acide p-[(p-amidinobenzamido)acétyl]phénoxyacétique,
ester méthylique de l'acide p-[2-[p-amidino-N-(p-carbométhoxybenzyl)phénylsulfonamido]éthyl]-phénoxyacétique,
acide p-[2-[p-amidino-N-(p-carboxybenzyl)phénylsulfonamido]éthyl]phénoxyacétique,
ester diméthylique de l'acide [[4-[2-(p-amidinophénylsulfonamido)éthyl]-o-phénylène]dioxy]diacétique,
acide [[4-[2-(p-amidinophénylsulfonamido)éthyl]-o-phénylène]dioxy]diacétique,
ester méthylique de l'acide [4-[2-(p-amidinophénylsulfonamido)éthyl]-2-méthoxyhénoxy] acétique,
acide [4-[2-(p-amidinophénylsulfon-amido)éthyl]-2-méthoxyphénoxy]acétique,
ester méthylique de l'acide (E)-p-[2-(p-amidinophénylsulfonamido)étnyl]-$\beta$-méthylcinnamique,
acide (E)-p-[2-(p-amidinopbénylsulfonamido)éthyl]-$\beta$-méthylcinnamique,
ester méthylique de l'acide p-[2-(p-amidinophénylsulfonamido)éthyl]-$\beta$-méthyl-hydrocinnamique,
acide p-[2-(p-amidinophénylsulffonamido)éthyl]-$\beta$-méthylhydrocinnamique,
ester éthylique de l'acide (E)-5-[(RS)-2-(p-amidinobenzamido)propyl]-$\beta$-méthyl-2-thiophène-acrylique,
acide (E)-5-[(RS)-2-(p-amidinobenzamido)propyl]-$\beta$-méthyl2-thiophène-acrylique,
ester éthylique de l'acide (RS)-5-[(RS)-2-(p-amidinobenzamido)propyl]-$\beta$-méthyl-2-thiophène-propioni-que,
acide (RS)-5-[(RS)-2-(p-amidinobenzamido)propyl]-$\beta$-méthyl2-thiophène-propionique,
ester méthylique de l'acide p-[2-(p-amidinobenzamido)éthyl]hydrocinnamique,
acide p-[2-(p-amidinobenzamido)éthyl]hydrocinnnamiqueester méthylique de l'acide p-[3-(p-amidino-benzamido)propyl]phénylacétique,
acide p-[3-(p-amidinobenzamido)propyl]phénylacétique,

ester méthylique de l'acide p-[3-(p-amidinophénylsulfonamido)propyl]phénylacétique,
acide p-[3-(p-amidinophénylsulfonamido)propyl]phénylacétique,
ester méthylique de l'acide p-[2-(4-amidino-3-pyridylamido)éthyl]phénoxyacétique,
acide p-[2-(4-amidino-3-pyridylamido)éthyl]phénoxyacétique,
ester diméthylique de l'acide p,p'-[[p-amidinobenzoyl)imino]diéthylène]dihydrocinnamique,
acide p,p'-[[p-amidinobenzoyl)imino]diéthylène]dihydrocinnamique,
ester méthylique de l'acide p-[2-[(p-amidinophényl)carbamoyl]éthyl]phénoxyacétique,
acide p-[2-[(p-amidinophényl)carbemoyl]éthyl]phénoxyacétique,
ester méthylique de l'acide p-[2-(p-amidinobenzamido)-1-hydroxyéthyl]hydrocinnamique,
acide p-[2-(p-amidinobenzamido)-1-hydroxyéthyl]hyrocinnamique,
ester méthylique de l'acide p-[(p-amidinobenzamido)acétyl]hydrocinnamique,
acide p-[(p-amidinobenzamido)acétyl]hydrocinnamique,
ester méthylique de l'acide p-[2-(p-guanidinobenzamido)éthyl]hyrocinnamique,
acide p-[2-(p-guanidinobenzamido)éthyl]hydrocinnamique,
ester méthylique de l'acide [α(p-guanidinobenzamido)-p-tolyl]oxy-acétique,
acide [α-(p-guanidinobenzamido)-p-tolyl]oxy-acétique,
ester méthylique de l'acide p-[(p-guanidinobenzamido)méthyl]hydrocinnamique,
acide p-[(p-guanidintobenzamido)méthyl]hydrocinnamique,
ester méthylique de la N-(p-amidinobenzoyl)-3-[p-(tert-butoxycarbonyl)méthoxy)phényl]-D-alanine,
acide [p-(R)-2-(p-amidonobenzamido)-2-(méthoxycarbonyl)éthyl)phénoxy]acétique,
N-(p-amidinobenzoyl)-3-[p-(carboxyméthoxy)phényl]-D-alanine,
ester méthylique de l'acide (E)-p-[2-(p-amidinobenzamido)éthyl]-β-méthyl-cinnamique,
ester méthylique de l'acide rac-p-[2-(p-amidinobenzamido)éthyl]-β-méthylhydrocinnamique,
ester méthylique de l'acide p-[2-(p-guanidinobenzamido)éthyl]phénoxyacétique,
ester méthylique de la N-benzyl-N-[p-[2-(p-amidinobenzamido)éthyl]phényl]glycine,
acide [p-[2-(p-guanidinobenzamido)éthyl]phénoxy]acétique,
ester t-butylioue de l'acide p-[(R)-2-(p-amidinobenzamido)-3-hydroxypropyl]phénoxyacétique,
acide p-[(R)-2-(p-amidinobenzamido)-3-hydroxypropyl]phénoxyacétique,
ester diméthylique de l'acide [[4-[p-amidino-N-méthylbenzamido)acétyl]-o-phénylène]dioxy]diacétique,
acide [[4-[p-amidino-N-méthyl-benzamido)acétyl]-o-phénylène]dioxy]diacétique,
ester méthylique de l'acide p-[N-(p-amidinobenzoyl)-(RS)-alanyl]phénoxyacétique,
acide p-[N-(p-amidinobenzoyl)-(RS)-alanyl]phénorxyacétique,
ester méthylique de l'acide p-[2-[p-amidino-N-(p-carbométhoxybenzyl)benzamido]-1-hydroxyéthyl]-phénoxyacétique,
acide p-[2-[p-amidino-N-(p-carboxybenzyl)benzamido]-1-hydroxyéthyl]phénoxyacétique, ester méthylique de l'acide p-[[p-amidino-N-(p-carbométhoxybenzyl)benzamido]acétyl]phénoxyacétique et
acide p-[[p-amidino-N-(p-carboxybenzyl)benzamido]acétyl]phénoxyacétique.

10. Composés selon l'une quelconque des revendications 1, 2 ou 4 du groupe de ceux qui suivent :
ester méthylique de l'acide α[(p-amidinobenzyl)carbamoyl]-p-tolyloxyacétique,
acide α-[(p-amidinobenzyl)carbamoyl]-p-tolyloxyacétique,
ester méthylique de l'acide p-[(p-amidinophénéthyl)carbamoyl]phénoxyacétique,
acide p-[(p-amidinophénéthyl)carbamoyl]phénoxyacétique,
acide [[α-(p-amidinohydrocinnamamnido-p-tolyl]oxy]acétique,
acide [p-(p-amidinohydrocinnamamido)phénoxy]acéytique,
acide [p-[(E)-p-amidinocinnamamido]phénoxy]acétique,
ester méthylique de l'acide [[α[2-(p-amidinophényl)acétamido]-p-tolyl]oxy]acétique et
acide [[p-[2-(p-amidinophényl)-acétamdo]-p-tolyl]oxy]acétique.

11. Composés selon les revendications 1-10 pour une utilisation en tant que médicament.

12. Composés selon les revendications 1-10 pour utilisation comme principes actifs dans la préparation de médicaments pour le traitement de thromboses, d'apoplexie, d'infarctus du myocarde, d'inflammations, d'artériosclérose, ainsi que de tumeurs.

13. Préparation pharmaceutique, contenant un composé selon les revendications 1-10 et un support pharmaceutiquement acceptable.

**14.** Procédé pour la préparation de composés de formule I selon la revendication 1, et de leurs sels caractérisé par le fait que

a) dans un composé de formule générale

$NC\text{-}A\text{-}(W)_a\text{-}X\text{-}(CH_2)_b\text{-}(Y)_c\text{-}B\text{-}Z\text{-}COOR^5$ (II)

où $R^5$ est un groupe alkyle inférieur ou benzyleet A, B, W, X, Y, Z, a, b et c ont les significations indiquées plus haut et où les groupes carboxy éventuellement présents dans la molécule se présentent soug forme d'ester,
on convertit le groupe nitrile en le groupe amidino, ou

b) dans un composé de formule générale

$R^{11}\text{-}A\text{-}(W)_a\text{-}X\text{-}(CH_2)_b\text{-}(Y)_c\text{-}B\text{-}Z\text{-}COOR^5$ (III)

où A, B, W, X, Y, Z, $R^5$, a, b et c ont la signification indiquée plus haut, et $R^{11}$ représente un groupe amidino ou guanidino protégé,
on élimine les groupes protecteurs du groupe amidino ou guanidino, ou

c) dans un composé de formule générale

$H_2N\text{-}A\text{-}(W)_a\text{-}X\text{-}(CH_2)_b\text{-}(Y)_c\text{-}B\text{-}Z\text{-}COOR^5$ (IV)

où A, B, W, X, Y, Z, $R^5$, a, b et c ont le signifi cation indiquée plus haut, et où des groupes carboxy éventuellement présents dans la molécule sont nous forme ester,
on transforme le groupe amino en groupe guanidino, ou

d) on fait réagir un composé de formule générale

$R^1\text{-}A\text{-}(W)_a\text{-}COE$ (V)

où E est un groupe ester activé ou du chlore ou du brome et $R^1$, A, W et a ont la signification indiquée plus haut,
avec un composé de formule générale

$HNR^2\text{-}(CH_2)_b\text{-}(Y)_c\text{-}B\text{-}Z\text{-}COOR^5$ (VI)

où Y, B, Z, $R^5$, $R^2$, b et c ont la signification indiquée plus haut,
on élimine un groupe benzyle $R^5$ éventuellement présent et en option on modifie des groupes fonctionnels présents dans un composé ainsi obtenu de formule générale I.

**Revendications pour les Etats contractants suivants : ES, GR**

**1.** Procédé pour la préparation de composés de formule générale

$R^1\text{-}A\text{-}(W)_a\text{-}X\text{-}(CH_2)_b\text{-}(Y)_c\text{-}B\text{-}Z\text{-}COOR$ (I)

où

A désigne un reste

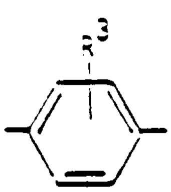 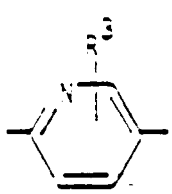 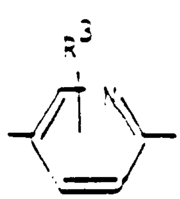 ou 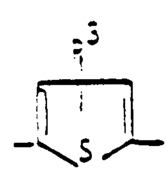 ;

B désigne un reste

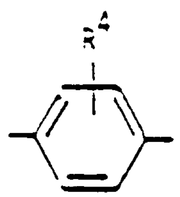 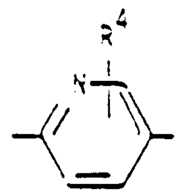 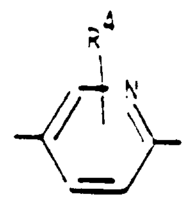 ou 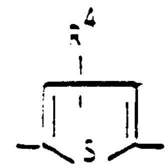 ;

W représente $-CH_2-$, $-CH_2CH_2-$, $-CH=CH-$, $-CH=CH-CH_2-$, $-(CH_2)_3-$, $-CH_2CH(CH_3)-$, $-COCH_2-$, $-CH(OH)CH_2-$ ou $-CH_2COCH_2-$;

X représente $-CONR^2-$, $-NR^2CO-$, $-SO_2NR^2-$ ou $-NR^2SO_2-$;

Y représente $-CH_2CH_2-$, $-CH_2CH_2O-$, $-OCH_2-$, $-CH(CH_3)CH_2-$,$-CH=CH-$, $-CH_2-CH=CH-$, $-C(Q^1,Q^2)-CO(CH_2)_d-$, $-CH_2-$, $-CH_2CH_2CH_2-$, $-CH(CH_3)CH_2CH_2-$, $-CH_2COCH_2-$, $-C-(Q^1,Q^2)-CH(OH)-$, $-C((Q^1,Q^2)-CH(SSCH_3)-$, $-CH(CH_2OH)CH_2-$ ou $-CH(COOR)CH_2-$, tandis que les groupes carbonyle peuvent également être présents sous forme de groupes oxime, éther d'oxime, cétal ou thiocétal ou éther d'énol et les groupes hydroxy sous forme de groupes éther d'alkyle inférieur, éther de di(alkyl inférieur)amino-alkyle inférieur ou sous forme de groupes ester d'acides alcanecarboxylioues inférieurs,

Z désigne $-OCH_2-$, $-NR^6CH_2-$, $-CH_2CH_2-$, $-CH(CH_3)CH_2-$, $-CH_2-$, $-CH=CH-$ ou $-C(CH_3)=CH-$;

R représente l'hydrogène ou un groupe alkyle inférieur, phényle ou phényl-alkyle inférieur;

$Q^1$ et $Q^2$ sont l'hydrogène ou un groupe alkyle inférieur ou forment avec l'atome de carbone auouel ils sont liés un cycle saturé à 3 à 6 chaînons;

$R^1$ désigne un groupe amidino ou guanidino;

$R^2$ désigne l'hydrogène ou un groupe alkyle inférieur, phényl-alkyle inférieur, phényl-alkyle inférieur substitué dans la partie phényle par aes radicaux amino, amidino ou -COOR, ou un reste $-CH_2COOR$ ou Y-B-Z-COOR;

$R^3$ représente l'hydrogène ou un groupe alkyle inférieur, alcoxy inférieur, halogène, carbalcoxy inférieur, amino, alkylamino inférieur, dialkyl(inférieur)amino ou amidino;

$R^4$ représente l'hydrogène ou un groupe alkyle inférieur, alcoxy inférieur, halogène, carbalcoxy inférieur, amino, alkylamino inférieur, dialkyl(inférieur)amino ou un reste -Z-COOR ou $-CH=CH-(CH_2)_nCOOR$;

$R^6$ désigne l'hydrogène ou un groupe alkyle inférieur ou benzyle;

n représente un nombre entier de 0 à 4;

a c et d sont, indépendamment l'un de l'autre, 0 ou 1; et

b désigne un nombre entier de 0 à 2, tandis que cependant a et b doivent valoir 0 lorsque c est 1, et c doit valoir 0 lorsque a ou b est différent de 0;

et de leurs sels physiologiquement acceptables, caractérisé en ce que

a) dans un composé de formule générale

$$NC-A-(W)_a-X-(CH_2)_b-(Y)_c-B-Z-COOR^5 \qquad (II)$$

où $R^5$ est un groupe alkyle inférieur ou benzyle et A, B, W, X, Y, Z, a, b et c ont les significations indiquées plus haut et où les groupes carboxy éventuellement présents dans la molécule se présentent sous forme d'ester,

on convertit le groupe nitrile en le groupe amidino, ou

b) dans un composé de formule générale

$$R^{11}-A-(W)_a-X-(CH_2)_b-(Y)_c-B-Z-COOR^5 \qquad (III)$$

où A, B, W, X, Y, Z, $R^5$, a, b et c ont la signification indiquée plus haut, et $R^{11}$ représente un groupe amidino ou guanidino protégé,

on élimine les groupes protecteurs du groupe amidino ou guanidino, ou

c) dans un composé de formule générale

$$H_2N-A-(W)_a-X-(CH_2)_b-(Y)_c-B-Z-COOR^5 \qquad (IV)$$

où A, B, W, X, Y, Z, $R^5$, a, b et c ont la signifi cation indiquée plus haut, et où des groupes

carboxy éventuellement présents dans la molécule sont sous forme ester,
on transforme le groupe amino en groupe guanidino, ou
d) on fait réagir un composé de formule générale

$R^1$-A-$(W)_a$-COE (V)

où E est un groupe ester activé ou du chlore ou du brome et $R^1$, A, W et a ont la signification indiquée plus haut,
avec un composé de formule générale

$HNR^2$-$(CH_2)_b$-$(Y)_c$-B-Z-$COOR^5$ (VI)

où Y, B, Z, $R^5$, $R^2$, b et c ont la signification indiquée plus haut,
on élimine un groupe benzyle $R^5$ éventuellement présent et en option on modifie des groupes fonctionnels présents dans un composé ainsi obtenu de formule générale I.

**2.** Procédé selon la revendication 1, dans lequel W et X ont la signification indiquée dans la revendication 1 et Y désigne $-CH_2CH_2-$, $-CH_2CH_2O-$, $-CH(CH_3)CH_2$, $-CH=CH-$, $-CH_2-CH=CH-$, $-CH_2CO-$, $-CH_2-$, $-CH_2CH_2CH_2-$, $-CH(CH_3)CH_2CH_2-$, $-CH_2COCH_2-$, $-CH_2CHOH-$, $-CH(CH_2OH)CH_2-$ ou $-CH(COOR)CH_2-$, tandis que les groupes carbonyle peuvent également être présents sous forme de groupes oxime, éther d'oxime, cétal ou thiocétal ou éther d'énol et les groupes hydroxy sous forme de groupes éther d'alkyle inférieur, ou sous forme de groupes ester d'acides alcanecarboxyliques inférieurs.

**3.** Procédé selon l'une des revendications 1 ou 2, caractérisé en ce qu'on prépare des composés de formule

$R^1$-A-X-Y-B-Z-COOR (Ia)

**4.** Procédé selon l'une des revendications 1 ou 2, caractérisé en ce qu'on prépare des composés de formule

$R^1$-A-W-X-$(CH_2)_b$-B-Z-COOR (Ib)

**5.** Procédé selon l'une quelconque des revendications 1-4, dans lequel la somme des atomes de C, O, N et S présents en chaîne droite dans $(W)_a$, $(CH_2)_b$, $(Y)_c$, X et Z est de 6.

**6.** Procédé selon l'une quelconque des revendications 1-5, dans lequel $R^1$ est un groupe amidino.

**7.** Procédé selon l'une quelconque des revendications 1-6, dans lequel Y est $-CH(CH_3)CO-$, $-CH_2CH_2-$ ou $-CH_2CO-$; Z est $-OCH_2-$ ou $-CH_2CH_2-$ et X est -NHCO- ou -CONH-.

**8.** Procédé selon l'une quelconque des revendications 1-7, dans lequel R est de l'hydrogène.

**9.** Procédé selon l'une quelconque des revendications 1, 2 ou 3 pour la préparation des composés du groupe de ceux qui suivent:
ester méthylique de l'acide p-[2-(p-amidinobenzamido)éthyl]phénoxyacétique,
acide [p-[2-(p-amidinobenzamido)éthyl]phénoxy]acétique,
[4-[2-(p-amidinobenzamido)éthyl]-2-iodophénoxy]acétate de méthyle,
acide [4-[2-(p-amidinobenzamido)éthyl]-2-iodophénoxy]acétique,
ester benzylique de l'acide (E)-5-[5-[2-(p-amidinobenzamido)éthyl]-2-(carboxyméthoxy)phényl]-4-penténoïque,
acide (E)-5-[5-[2-(p-amidinobenzamido)éthyl]-2-(carboxyméthoxy)phényl]-4-penténoïque,
ester méthylique de l'acide p-[2-(p-amidinobenzamido)éthoxy]phénylacétique,
acide p-[2-(p-amidinobenzamido)éthoxy]phénylacétique,
ester méthylique de l'acide p-[2-(p-amidinophénylsulfonamido)éthoxy]phénylacétique,
acide p-[2-(p-amidinophénylsulfonamido)éthoxy]phényl-acétique,
ester méthylique de l'acide p-[2-(p-amidinobenzènesulfonamido)éthyl]phénoxyacétique,
acide p-[2-(p-amidinobenzènesulfonamido)éthyl]phénoxyacétique,

ester méthylique de l'acide p-[(S)-2-(p-amidinobenzènesulfonamido)propyl]phénoxyacétique,
acide p-[(S)-2-(p-amidinobenzène-sulfonamido)propyl]phénoxyacétique,
ester méthylique de l'acide p-[2-(p-amidino-N-méthylphénylsulfonamido)éthyl]phénoxyacétique,
acide p-[2-(p-amidino-N-méthylphénylsulfonamido)éthyl]phénoxyacétique,
ester méthylique de l'acide p-[2-(p-amidino-N-benzylphénylsulfonamido)éthyl]-phénoxyacétique,
acide p-[2-(p-amidino-N-benzylphénylsulfonamido)éthyl]phénoxyacétique,
ester méthylique de l'acide p-[2-(p-amidinobenzènesulfonamido)-1-hydroryéthyl]phénoxyacétique,
acide p-[2-(p-amidinobenzènesulfon-amido)-1-hydroxyéthyl]phénoxyacétique,
ester méthylique de l'acide p-[2-(p-amidinobenzamido)-1-hydroxyéthyl]-phénoxyacétique,
acide p-[2-(p-amidinobenzamido)-1-hydroxyéthyl]phénoxyacétique,
ester méthylique de l'acide p-[(p-amidinophénylsulfonamido)acétyl]phénoxyacétique,
acide p-[(p-amidinophénylsulfonamido)acétyl]phénoxyacétique,
ester méthylique de l'acide p-[(p-amidinobenzamido)acétyl]phénoxyacétique,
acide p-[(p-amidinobenzamido)acétyl]phénoxyacétique,
ester méthylique de l'acide p-[2-[p-amidino-N-(p-carbométhoxybenzyl)phénylsulfonamido]éthyl]-phénoxyacétique,
acide p-[2-[p-amidino-N-(p-carboxybenzyl)phénylsulfonamido]éthyl]phénoxyacétique,
ester diméthylique de l'acide [[4-[2-(p-amidinophénylsulfonamido)éthyl]-o-phénylène]dioxy]diacétique,
acide [[4-[2-(p-amidinophénylsulfonamido)éthyl]-o-phénylène]dioxy]diacétique,
ester méthylique de l'acide [4-[2-(p-amidinophénylsulfonamido)éthyl]-2-méthoxyphénoxy]acétique,
acide [4-[2-(p-amidinophénylsulfon-amido)éthyl]-2-méthoxyphénoxy]acétique,
ester méthylique de l'acide (E)-p-[2-(p-amidinophénylsulfonamido)éthyl]-$\beta$-méthylcinnamique,
acide (E)-p-[2-(p-amidinophénylsulfonamido)éthyl]-$\beta$-méthylcinnamique,
ester méthylique de l'acide p-[2-(p-amidinophénylsulfonamido)éthyl]-$\beta$-méthyl-hydrocinnamique,
acide p-[2-(p-amidinophénylsulfonamido)éthyl]-$\beta$-méthylhydrocinnamique,
ester éthylique de l'acide (E)-5-[(RS)-2-(p-amidinobenzamido)propyl]-$\beta$-méthyl-2-thiophène-acrylique,
acide (E)-5-[(RS)-2-(p-amidinobenzamido)propyl]-$\beta$-méthyl-2-thiophène-acrylique,
ester éthylique de l'acide (RS)-5-[(RS)-2-(p-amidinobenzamido)propyl]-$\beta$-méthyl-2-thiophène-propionique,
acide (RS)-5-[(RS)-2-(p-amidinobenzamido)propyl]-$\beta$-méthyl-2-thiophène-propionique,
ester méthylique de l'acide p-[2-(p-amidinobensamido)éthyl]hydrocinnamique,
acide p-[2-(p-amidinobenzamido)éthyl]hydrocinnamique,
ester méthylique de l'acide p-[3-(p-amidinobenzamido)propyl]phénylacétique,
acide p-[3-(p-amidinobenzamido)propyl]phénylacétique,
ester méthylique de l'acide p-[3-(p-amidinophénylsulfon-amido)propyl]phénylacétique,
acide p-[3-(p-amidinophénylsulfonamido)propyl]phénylacétique,
ester méthylique de l'acide p-[2-(4-amidino-3-pyridylamido)éthyl]phénoryacétique,
acide p-[2-(4-amidino-3-pyridylamido)éthyl]phénoxyacétique,
ester diméthylique de l'acide p,p'-[[p-amidinobenzoyl)imino]diéthylène]dihydrocinnamique,
acide p,p'-[[p-amidinobenzoyl)imino]diéthylène]dihydrocinnamique,
ester méthylique de l'acide p-[2-[(p-amidinophényl)carbamoyl]éthyl]phénoxyacétique,
acide p-[2-[(p-amidinophényl)carbamoyl]éthyl]phénoxyacétique,
ester méthylique de l'acide p-[2-(p-amidinobenzamido)-1-hydroxyéthyl]hydrocinnamique,
acide p-[2-(p-amidinobensamido)-1-hydroxyéthyl]hydrocinnamique,
ester méthylique de l'acide p-[(p-amidinobenzamido)acétyl]hydrocinnamique,
acide p-[(p-amidinobenzamido)acétyl]hydrocinnamique,
ester méthylique de l'acide p-[2-(p-guanidinobenzamido)éthyl]hydrocinnamique,
acide p-[2-(p-guanidinobenzamido)éthyl]hydrocinnamique,
ester méthylique de l'acide [$\alpha$(p-guanidinobenzamido)-p-tolyl]oxy-acétique,
acide [$\alpha$-(p-guanidinobenzamido)-p-tolyl]oxy-acétique,
ester méthylique de l'acide p-[(p-guanidinobenzamido)méthyl]hydrocinnamique,
acide p-[(p-guanidinobenzamido)méthyl]hydrocinnamique,
ester méthylique de la N-(p-amidinobenzoyl)-3-[p-(tert-butoxycarbonyl)méthoxy)phényl]-D-alanine,
acide [p-(R)-2-(p-amidonobenzamido)-2-(méthoxycarbonyl)éthyl)phénoxy]acétique,
N-(p-amidinobenzoyl)-3-[p-(carboxyméthoxy)phényl]-D-alanine,
ester méthylique de l'acide (E)-p-[2-(p-amidinobenzamido)éthyl]-$\beta$-méthyl-cinnamique,
ester méthylique de l'acide rac-p-[2-(p-amidinobenzamido)éthyl]-$\beta$-méthylhydrocinnamique,
ester méthylique de l'acide p-[2-(p-guanidinobenzamido)éthyl]phénoxyacétique,

ester méthylique de la N-benzyl-N-[p-[2-(p-amidinobenzamido)éthyl]phényl]glycine,
acide [p-[2-(p-guanidinobenzamido)éthyl]phénoxy]acétique,
ester t-butylique de l'acide p-[(R)-2-(p-amidinobenzamido)-3-hydroxyoropyl]phénoxyacétique,
acide p-[(R)-2-(p-amidinobenzamido)-3-hydroxypropyl]phénoxyacétique,
ester diméthylique de l'acide [[4-[p-amidino-N-méthylbenzamido)acétyl]-o-phénylène]dioxy]diacétique,
acide [[4-[p-amidino-N-méthyl-benzamido)acétyl]-o-phénylène]dioxy]diacétique,
ester méthylique de l'acide p-[N-(p-amidinobenzoyl)-(RS)-alanyl]phénoxyacétique,
acide p-[N-(p-amidinobenzoyl)-(RS)-alanyl]phénoxyacétique,
ester méthylique de l'acide p-[2-[p-amidino-N-(p-carbométhoxybenzyl)benzamido]-1-hydroxyéthyl]-phénoxyacétique,
acide p-[2-[p-amidino-N-(p-carboxybenzyl)benzamido]-1-hydroxyéthyl]phénoxyacétique,
ester méthylique de l'acide p-[[p-amidino-N-(p-carbométhoxybenzyl)benzamido]acétyl]phénoxyacétique et
acide p-[[p-amidino-N-(p-carboxybenzyl)benzamido]acétyl]phénoxyacétique.

**10.** Procédé selon l'une quelconque des revendications 1, 2 ou 4 pour la préparation des composés du groupe de ceux qui suivent:
ester méthylique de l'acide α[(p-amidinobenzyl)carbamoyl]-p-tolyloxyacétique,
acide α-[(p-amidinobenzyl)carbamoyl]-p-tolyloxyacétique,
ester méthylique de l'acide p-[(p-amidinophénéthyl)carbamoyl]phénoxyacétique,
acide p-[(p-amidinophénéthyl)carbamoyl]phénoxyacétique,
acide [[α-(p-amidinohydrocinnamamido)-p-tolyl]oxy]acétique,
acide [p-(p-amidinohydrocinnamamido)phénoxy]acétique,
acide [p-[(E)-p-amidinocinnamamido]phénoxy]acétique,
ester méthylique de l'acide [[α[2-(p-amidinophényl)acétamido]-p-tolyl]oxy]acétique et
acide [[p-[2-(p-amidinophényl)-acétamido]-p-tolyl]oxy]acétique.

**11.** Procédé selon la revendication 1, caractérisé en ce qu'on

a) fait réagir le nitrile de formule II avec de l'hydrogène sulfuré et une base dans un solvant, on méthyle le thioamide obtenu et on fait réagir le composé obtenu de formule I, dans lequel au lieu de $R^1$ se trouve le groupe $-C(NH)SCH_3$, avec de l'acétate d'ammonium,

b) élimine les groupes protecteurs par hydrogénation catalytique ou par traitement avec un acide,

c) convertit le groupe amino en le groupe guanidine au moyen de 2-S-isothiourée-éthanesulfonate en présence d'une base,

d) effectue la réaction des composés de formules V et VI en présence d'une base et élimine le groupe benzyle $R^5$ par hydrogénolyse

et éventuellement saponifie des groupes ester avec une base, élimine des groupes éther, éther d'énol, cétal ou thiocétal avec des acides, hydrogène catalytiquement une double liaison oléfinique, ou convertit un groupe acide carboxylique en un sel.

**12.** Procédé pour la préparation de médicaments, caractérisé en ce qu'on met sous une forme de présentation galénique un composé de formule I ou un de ses sels.

**13.** Produits selon l'une quelconque des revendications 1-8 pour utilisation comme principes actifs dans la préparation de médicaments pour le traitement de thromboses, d'apoplexie, d'infarctus du myocarde, d'inflammations, d'artériosclérose, ainsi que de tumeurs.